# EUROPEAN PATENT SPECIFICATION

(11) **EP 3 143 035 B1**
(45) Date of publication and mention of the grant of the patent: **07.08.2019**
(21) Application number: 15793647.7
(22) Date of filing: 15.05.2015
(51) Int. Cl.: C07K 14/775, C12N 15/82, A23L 33/18, A61K 38/10

(54) **METHOD TO CONCENTRATE APOA-I MIMETIC PEPTIDES TRANSGENICALLY EXPRESSED IN PLANTS**
VERFAHREN ZUM KONZENTRIEREN VON TRANSGEN IN PFLANZEN EXPRIMIERTEN MIMETISCHEN APOA-I-PEPTIDEN
PROCÉDÉ POUR CONCENTRER DES PEPTIDES APOA-I-MIMÉTIQUES EXPRIMÉS DE MANIÈRE TRANSGÉNIQUE DANS DES PLANTES

(30) Priority: 15.05.2014 US 201461994003 P
(43) Date of publication of application: 22.03.2017
(73) Proprietor: THE REGENTS OF THE UNIVERSITY OF CALIFORNIA, Oakland, CA 94607-5200 (US)
(72) Inventor: FOGELMAN, Alan M., Los Angeles, California 90067 (US); REDDY, Srinivasa T., Cerritos, California 90703 (US); NAVAB, Mohamad, Los Angeles, CA 90049 (US)
(74) Representative: Chapman, Desmond Mark
(86) International application number: PCT/US2015/031134
(87) International publication number: WO 2015/175968

(56) References cited:
- US-A1- 2005 136 137
- US-A1- 2013 344 173
- US-B1- 7 572 468
- GADKARI ET AL.: 'Catechin concentrates of garden tea leaves (Camellia sinensis L.): extraction/isolation and evaluation of chemical composition' JOURNAL OF THE SCIENCE OF FOOD AND AGRICULTURE vol. 94, no. 14, 26 March 2014, ISSN 0022-5142 pages 2921 - 2928, XP055237696
- CHATTOPADHYAY ET AL.: 'A novel approach to oral apoA-I mimetic therapy' JOURNAL OF LIPID RESEARCH vol. 54, no. 4, 2013, ISSN 0022-2275 pages 995 - 1010, XP055237697
- NAVAB ET AL.: 'Transgenic 6F tomatoes act on the small intestine to prevent systemic inflammation and dyslipidemia caused by western diet and intestinally derived lysophosphatidic acid' JOURNAL OF LIPID RESEARCH vol. 54, no. 12, 2013, pages 3403 - 3418, XP055237698

## Description

### BACKGROUND

Mimetics of apolipoprotein A-I (apoA-I) containing only 18 amino acids showed promise in animal models of disease (Getz and Reardon (2011) J. Inflamm. Res. 4: 83-92; Navab et al. (2012) Arterioscler. Thromb. Vasc. Biol. 32: 2553-2560), and improved HDL function in humans when given orally at high doses despite achieving low plasma peptide levels (Bloedon (2008) J. Lipid Res. 49: 1344-1352). However, when high plasma levels were achieved with low doses of peptide given intravenously or by subcutaneous (SQ) injection, no improvement in HDL function was seen (Watson et al. (2011) J. Lipid Res. 52: 361-373). Studies in mice indicated that the major site of action for these peptides is in the intestine and that a high dose of peptide is required for efficacy (Navab et al. (2011) J. Lipid Res. 52: 1200-1210; Navab et al. (2012) J. Lipid Res. 53: 437-445).

The high dose requirement provides a barrier to use in humans because of the cost of chemically synthesizing these peptides. To overcome this barrier an 18 amino acid peptide (6F peptide, DWLKAFYDKFFEKFKEFF (SEQ ID NO:1) was transgenically expressed in tomatoes (Chattopadhyay et al. (2013) J. Lipid Res. 54: 995-1010). Feeding LDL receptor-null (LDLR^{-/-}) mice a Western diet (WD) for 13 weeks containing 2.2% by weight of freeze dried tomato powder made from transgenic tomatoes expressing the apoA-I mimetic peptide 6F (Tg6F) reduced plasma serum amyloid A (SAA) levels, reduced plasma total cholesterol levels, reduced plasma triglyceride levels, reduced plasma unsaturated (but not saturated) lysophosphatidic acid (LPA) levels, increased plasma paraoxonase-1 activity, increased plasma HDL-cholesterol levels, and decreased the extent of aortic atherosclerosis by about 50% (Chattopadhyay et al. (2013) J. Lipid Res. 54: 995-1010; Getz and Reardon (2013) J. Lipid Res. 54: 878-880).

Two hours after LDLR^{-/-} mice finished eating WD containing Tg6F, intact 6F peptide was found in the small intestine but not in the plasma (Chattopadhyay et al. (2013) J. Lipid Res. 54: 995-1010). Plasma levels of unsaturated (but not saturated) LPA correlated with the extent of aortic atherosclerosis. The content of LPA in the tissue of the small intestine was found to decrease after feeding Tg6F and the level of LPA (but not cholesterol) in the tissue of the small intestine correlated with the extent of aortic atherosclerosis (*Id.*).

Without any purification steps, when the transgenic tomatoes expressing 6F peptide were freeze-dried, ground into powder and fed to a mouse model of dyslipidemia and atherosclerosis at only 2.2% of a high-fat high-cholesterol diet by weight, the transgenic tomatoes significantly reduced dyslipidemia, inflammation and atherosclerosis in the mice. This provided a daily dose of approximately 40 mg/kg/day. However, if the same dose would be effective in a human, it would require the human to eat approximately 150 grams of freeze-dried tomato powder three times daily. While this is clearly possible, the volume of tomato powder required would be approximately three cups three times daily. Thus, while expression of the apolipoprotein A-I peptides in plants overcomes the cost of production barriers, the significant volume of material that would be consumed daily makes patient compliance challenging.

US 2005/0136137 teaches a composition for preventing atherosclerosis.

US 2013/344173 teaches modulating disease through genetic engineering of plants.

US 7,572,468 teaches extraction of carotenoids from plant material.

### SUMMARY

The invention provides a method of concentrating transgenic apoA-I mimetic peptides expressed in a transgenic tomato plant, said method comprising: providing a tissue of said transgenic plant wherein said tissue contains a heterologous ApoA-I mimetic peptide expressed by said plant, and wherein said tissue is provided as a substantially dry powder; mixing said powder with a solution comprising ethyl acetate and acetic acid to form an extraction mixture; collecting the liquid phase of said mixture and drying said liquid phase to provide a concentrated dry powder extract that displays the biological activity of said apoA-I mimetic. Other features of the invention are set out in the claims.

### DEFINITIONS

The HDL inflammatory index refers to the ability of HDL to inhibit LDL-induced monocyte chemotactic activity. In certain embodiments the HDL-inflammatory index is calculated by comparing the monocyte chemotactic activity generated by a standard control LDL in the absence and presence of the test HDL. In the absence of the test HDL the monocyte chemotactic activity is normalized to 1.0. If the monocyte chemotactic activity increases upon addition of the test HDL the HDL-inflammatory index is >1.0 and the test HDL is classified as pro-inflammatory. If the monocyte chemotactic activity decreases upon addition of the test HDL the HDL-inflammatory index is <1.0 and the HDL is classified as anti-inflammatory. A reduction in HDL inflammatory index is considered an improvement in HDL inflammatory index.

The terms "polypeptide", "peptide" and "protein" are used interchangeably herein to refer to a polymer of amino acid residues. The terms apply to amino acid polymers in which one or more amino acid residues is an artificial chemical analogue of a corresponding naturally occurring amino acid, as well as to naturally occurring amino acid polymers, however a recombinantly expressed peptide typically consists of amino acids that are all found in the host organism (*e.g.*, naturally occurring amino acids).

The term "an amphipathic helical peptide" refers to a peptide comprising at least one amphipathic helix (amphipathic helical domain). Certain amphipathic helical peptides contemplated herein can comprise two or more (*e.g.,* 3, 4, 5, *etc*.) amphipathic helices.

The term " class A amphipathic helix" refers to a protein structure that forms an α-helix producing a segregation of a polar and nonpolar faces with the positively charged residues residing at the polar-nonpolar interface and the negatively charged residues residing at the center of the polar face (*see, e.g.,* Segrest et al. (1990) Proteins: Structure, Function, and Genetics 8: 103-117).

"Apolipoprotein J" (apo J) is known by a variety of names including clusterin, TRPM2, GP80, and SP 40 (*see, e.g.,* Fritz (1995) Pp 112 In: Clusterin: Role in Vertebrate Development, Function, and Adaptation (Harmony JAK Ed.), R.G. Landes, Georgetown, TX,). It was first described as a heterodimeric glycoprotein and a component of the secreted proteins of cultured rat Sertoli cells (*see, e.g.,* Kissinger et al. (1982) Biol. Reprod.; 27: 233240). The translated product is a single-chain precursor protein that undergoes intracellular cleavage into a disulfide-linked 34kDa α subunit and a 47 kDa β subunit (*see, e.g.,* Collard and Griswold (1987) Biochem., 26: 3297-3303). It has been associated with cellular injury, lipid transport, apoptosis and it may be involved in clearance of cellular debris caused by cell injury or death. Clusterin has been shown to bind to a variety of molecules with high affinity including lipids, peptides, and proteins and the hydrophobic probe 1-anilino-8-naphthalenesulfonate (Bailey et al. (2001) Biochem., 40: 11828-11840).

The class G amphipathic helix is found in globular proteins, and thus, the name class G. The feature of this class of amphipathic helix is that it possesses a random distribution of positively charged and negatively charged residues on the polar face with a narrow nonpolar face. Because of the narrow nonpolar face this class does not readily associate with phospholipid (*see, e.g.,* Segrest et al. (1990) Proteins: Structure, Function, and Genetics. 8: 103-117; Erratum (1991) Proteins: Structure, Function and Genetics, 9: 79). Several exchangeable apolipoproteins possess similar but not identical characteristics to the G amphipathic helix. Similar to the class G amphipathic helix, this other class possesses a random distribution of positively and negatively charged residues on the polar face. However, in contrast to the class G amphipathic helix which has a narrow nonpolar face, this class has a wide nonpolar face that allows this class to readily bind phospholipid and the class is termed G* to differentiate it from the G class of amphipathic helix (*see, e.g.,* Segrest et al. (1992) J. Lipid Res., 33: 141-166; Anantharamaiah et al. (1993) Pp. 109-142 In: The Amphipathic Helix, Epand, R.M. Ed CRC Press, Boca Raton, Florida). Computer programs to identify and classify amphipathic helical domains have been described by Jones et al. (1992) J. Lipid Res. 33: 287-296) and include, but are not limited to the helical wheel program (WHEEL or WHEEL/SNORKEL), helical net program (HELNET, HELNET/SNORKEL, HELNET/Angle), program for addition of helical wheels (COMBO or COMBO/SNORKEL), program for addition of helical nets (COMNET, COMNET/SNORKEL, COMBO/SELECT, COMBO/NET), consensus wheel program (CONSENSUS, CONSENSUS/SNORKEL), and the like.

The term "treat" when used with reference to treating, *e.g.* a pathology or disease refers to the mitigation and/or elimination of one or more symptoms of that pathology or disease, and/or a reduction in the rate of onset or severity of one or more symptoms of that pathology or disease, and/or the prevention of that pathology or disease.

The term "ameliorating" when used with respect to "ameliorating one or more symptoms of atherosclerosis" refers to a reduction, prevention, or elimination of one or more symptoms characteristic of atherosclerosis and/or associated pathologies. Such a reduction includes, but is not limited to a reduction or elimination of oxidized phospholipids, a reduction in atherosclerotic plaque formation and rupture, a reduction in clinical events such as heart attack, angina, or stroke, a decrease in hypertension, a decrease in inflammatory protein biosynthesis, reduction in plasma cholesterol, and the like.

A "transgenic plant" is a plant that expresses in at least some of the cells of the plant a heterologous peptide. In certain embodiments the heterologous peptide consists of, or comprises the amino acid sequence of one or more apolipoprotein(s) or apolipoprotein mimetics, *e.g.,* an apoA-I mimetic, and/or a G* peptide, and/or an apoE peptide, *e.g.,* as described herein. In certain embodiments the transgenic plant is a plant that at least a portion of which is edible by a human and/or by a non-human mammal.

The term "biological activity" when used with respect to an apolipoprotein peptide, an apolipoprotein peptide mimetic, a peptide/protein comprising one or more apolipoprotein and/or apolipoprotein mimetic domains indicates that the peptide, when fed to a mammal lowers plasma SAA levels, and/or increases paraoxonase activity, and/or reduces levels of lysophosphatidic acid, and/or reduces levels of metabolites of arachidonic and linoleic acids. A transgenic plant or portion thereof having biological activity indicates that the plant or portion thereof when fed to a mammal lowers plasma SAA levels, and/or increases paraoxonase activity, and/or reduces levels of lysophosphatidic acid, and/or reduces levels of metabolites of arachidonic and linoleic acids.

The term "recombinant nucleic acid" as used herein refers to nucleic acid, originally formed *in vitro,* in general, in a form not normally found in nature.

A "heterologous" DNA coding sequence is a structural coding sequence that is not native to the plant being transformed, or a coding sequence that has been engineered for improved characteristics of its protein product. Heterologous, with respect to the promoter, refers to a coding sequence that does not exist in nature in the same gene with the promoter to which it is currently attached.

A "heterologous promoter" is a promoter manipulated so that it controls the transcription of a nucleic acid that is not a nucleic acid typically under regulation of that promoter.

A "regulatable promoter" is any promoter whose activity is affected by a cis or trans acting factor (*e.g.,* an ethylene-inducible promoter such as the tomato E8 promoter).

A "constitutive promoter" is any promoter that directs RNA transcription in many or all tissues of a plant transformant at most times.

A "tissue-specific promoter" is any promoter that directs RNA transcription at higher levels in particular types of cells and tissues (*e.g*., a fruit specific promoter).

By "promoter" or "promoter segment" (*e.g.*, a tomato E8 promoter or E4 promoter or hybrid E4/E8 promoter) is meant a sequence of DNA that functions alone as a promoter or as a component of a promoter herein to direct transcription of a downstream gene, and can include promoter or promoter segments derived by means of ligation with operator regions, random or controlled mutagenesis, addition or duplication of enhancer sequences, addition or modification with synthetic linkers, and the like.

By an E8 or an E4 gene promoter is meant a promoter obtained from an E8 or E4 gene considered to share sequence identity with the tomato E8 or E4 gene sequences (*e.g.,* as described in U.S. Patent 6,118,049), or a particular region or regions thereof, or from a gene having at least about 70%, preferably at least about 80%, more preferably at least about 85%, even more preferably at least about 90% sequence identify, or at least about 95% sequence identity, or at least about 98% sequence identity over a length of polynucleotide sequence corresponding to the tomato E8 or tomato E4 gene sequences.

The term "conservative substitution" is used in reference to proteins or peptides to reflect amino acid substitutions that do not substantially alter the activity (*e.g.,* ability to reduce SAA, and/or ability to increase paroxonase in a mammal. Typically conservative amino acid substitutions involve substitution one amino acid for another amino acid with similar chemical properties (*e.g*. charge or hydrophobicity). The following six groups each contain amino acids that are typical conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); and 6) Phenylalanine (F), Tyrosine (Y), Tryptophan (W).

A "macro-lipid component of the diet" refers to a lipid component of a mammal's diet that is typically present in milligram amounts per gram of diet. In a Western diet such macro-lipid components typically include, but are not limited to phospholipids such as phosphatidylcholine and sterols such as cholesterol. Even lysophosphatidylcholine is likely to be present in milligram quantities after phosphatidylcholine is acted upon in the Duodenum by PLA₂ and hence, in various embodiments, can be regarded as a macro-lipid component.

A "micro-lipid component of the diet" refers to a lipid component of a mammal's diet that is typically present in microgram (or lower) amounts per gram of diet. Illustrative microlipid components typically include, but are not limited to lysophosphatidic acid, phosphatidic acid, and the like.

### BRIEF DESCRIPTION OF THE DRAWINGS

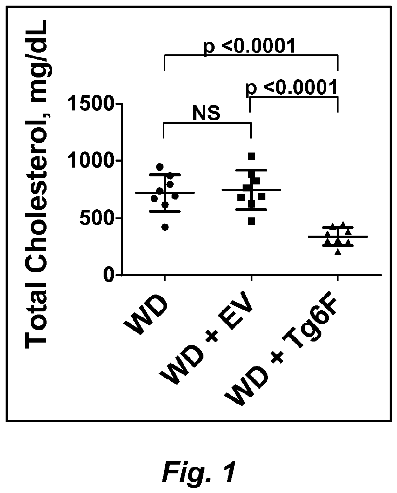

Figure 1 shows plasma total cholesterol levels.

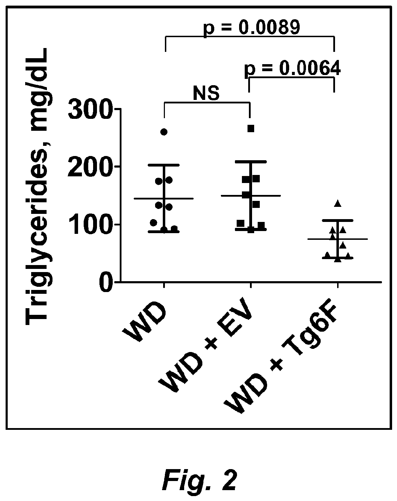

Figure 2 shows plasma triglyceride levels.

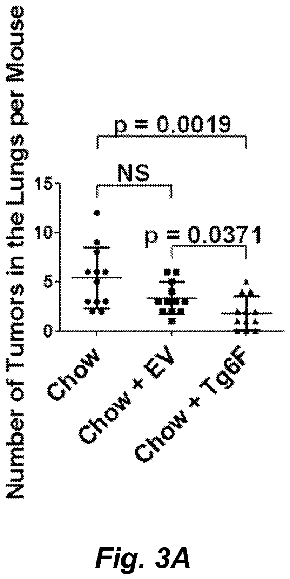

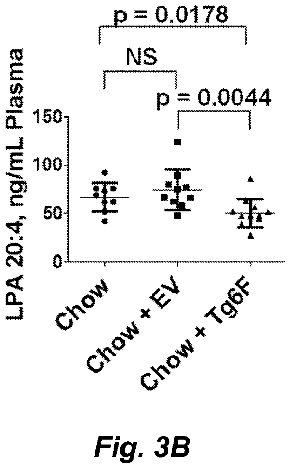

Figure 3A shows the number of metastatic tumors in the lungs of mice treated with Chow, Chow + EV, and Chow + T6gF extract produced as described herein. Fig. 3B shows plasma LPA 20:4 in mice treated with Chow, Chow + EV, and Chow + T6gF extract produced as described herein.

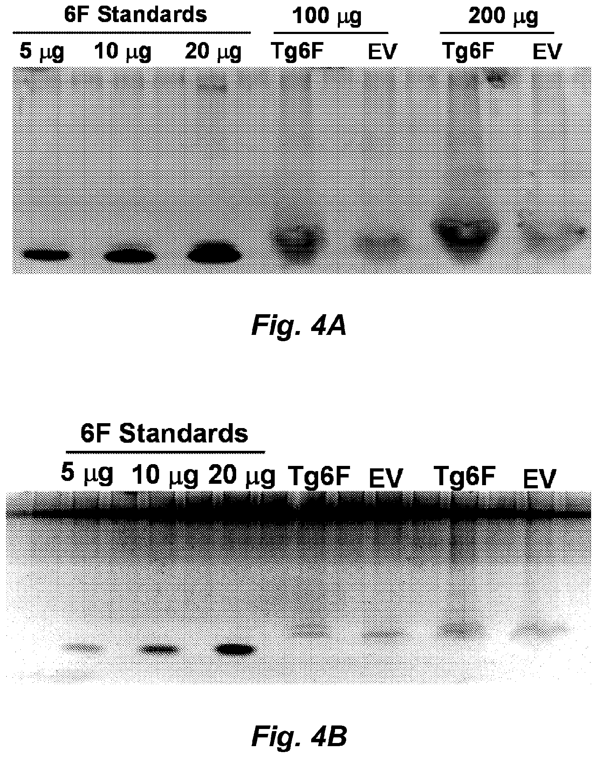

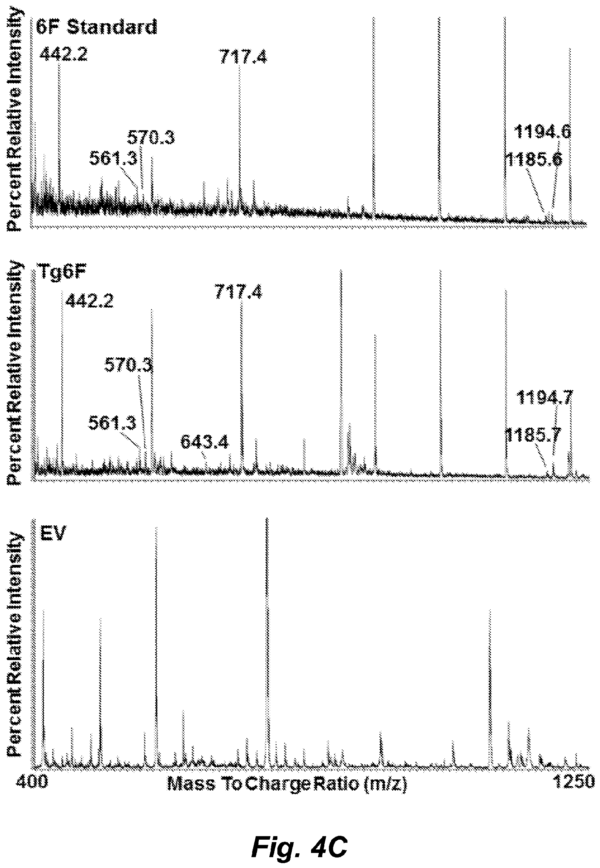

Figure 4, panels A-C. Ethyl acetate with 5% acetic acid (but not 60% ethanol) efficiently concentrates the 6F peptide produced in transgenic tomatoes. Freeze-dried transgenic tomatoes expressing β-glucuronidase (EV), or expressing the 6F peptide (Tg6F) were extracted overnight at room temperature in either ethyl acetate/acetic acid, or in aqueous ethanol as described in Example 3 Materials and Methods. The ethyl acetate/acetic acid extract was dried under argon; the aqueous ethanol extract was dried under vacuum as described in Materials and Methods. The remaining solids were suspended in water and analyzed on 18% SDS PAGE gels as described in Example 3 Materials and Methods. Panel A shows an image of the Sypro Ruby-stained gel of the tomato concentrates prepared with ethyl acetate/acetic acid (100 µg or 200 µg of protein per lane). Panel B shows an image of the Sypro Ruby-stained gel of tomato concentrates prepared with aqueous ethanol (100 µg protein per lane). Authentic chemically synthesized 6F peptide at 5, 10 or 20 µg per lane were included as standards in Panels A and B. Panel C shows a summary of the results of LC/MS analysis of the trypsin digest of the authentic 6F standard (6F Standard, top of Panel C, 5 µg), the trypsin digest of the corresponding band from the Tg6F lane (Tg6F, middle of Fig. 4C, 100 µg), and the trypsin digest of the corresponding band from the EV lane (EV, bottom of Panel C, 100 µg) performed as described in Example 3 Materials and Methods. To simplify data presentation, the spectra collected between 13 and 25 minutes from all three chromatograms were averaged. All the tryptic peptides eluted within this window. The arrows point to signals identified on the basis of molecular weight concordance as being derived from 6F peptide: EFF (residues 16-18), found m/z 442.2 (detected in both 6F Standard and Tg6F, but not in EV), calculated for MH⁺ 442.197 Da (monoisotopic, mi); DWLK (residues 1-4, SEQ ID NO:2), found m/z 561.3 (detected in both 6F Standard and Tg6F, but not in EV), calculated for MH⁺ 561.303 Da (mi); FFEK (residues 10-13, SEQ ID NO:3), found m/z 570.3 (detected in both 6F Standard and Tg6F, but not in EV), calculated for MH⁺ 570.292 Da (mi); AFYDK (residues 5-9, SEQ ID NO:4), found m/z 643.3 (detected in Tg6F, but not in 6F Standard or EV), calculated for MH⁺ 643.308 Da (mi); FKEFF (residues 14-18, SEQ ID NO:5), found m/z 717.4 (detected in both 6F Standard and Tg6F, but not in EV), calculated for MH⁺ 717.361 Da (mi); DWLKAFYDK (residues 1-9), found m/z 1185.6 and 1185.7 (detected in 6F Standard and Tg6F, respectively, but not in EV), calculated for MH⁺ 1185.594 Da (mi); AFYDKFFEK (residues 5-13, SEQ ID NO:6), found m/z 1194.6 and 1194.7 (detected in 6F Standard and Tg6F, respectively, but not in EV), calculated for MH⁺ 1194.583 Da (mi). These partially-overlapping tryptic peptides provided 100% sequence coverage for both the 6F standard and the Tg6F sample. These results are representative of two of two experiments.

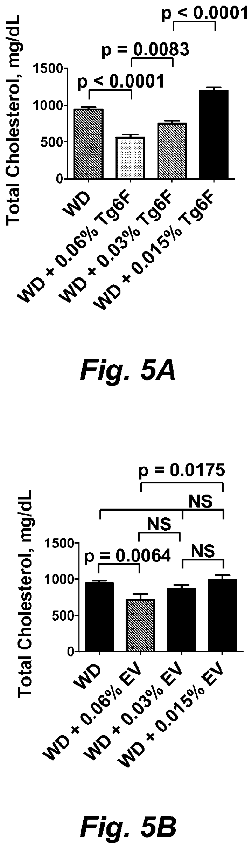

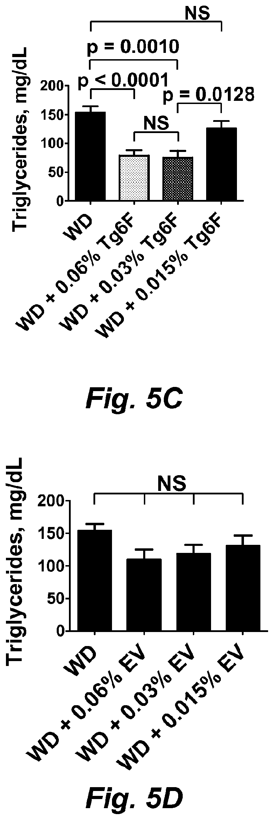

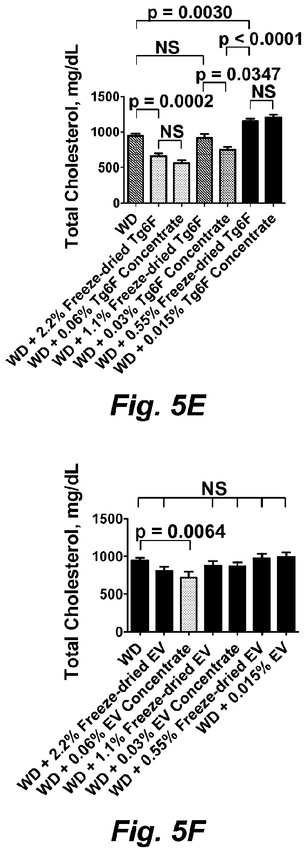

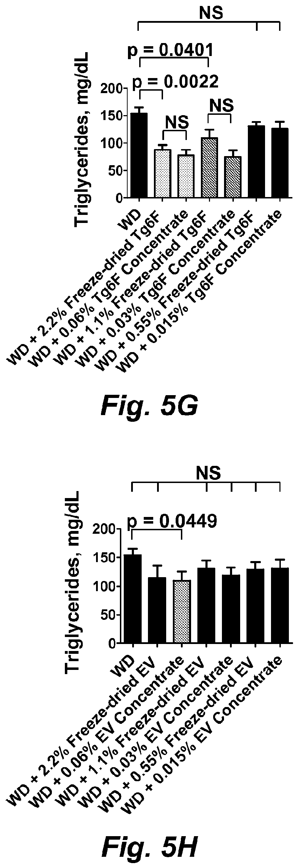

Figures 5A-5H. Dose response of tomato concentrates in a mouse model of dyslipidemia. Female LDLR null mice 4 - 5 months of age (n = 8 - 24 mice per group) were fed a Western diet (WD) or WD supplemented with 0.015%, 0.03% or 0.06% tomato concentrate by weight or the mice were fed the freeze-dried transgenic tomatoes from which the concentrates were made added to the WD at 0.55%, 1.1% or 2.2% by weight as described in Example 3 Materials and Methods. After two weeks the mice were fasted overnight and plasma total cholesterol and triglycerides were determined as described in Example 3 Materials and Methods. Figs. 5A and 5B show plasma total cholesterol levels for mice receiving Tg6F or EV tomato concentrates, respectively. Figs. 5C and 5D show plasma triglyceride levels for mice receiving Tg6F or EV tomato concentrates, respectively. Figs. 5E and 5F show plasma total cholesterol levels for the mice in Figs. 5A and 5B, respectively, compared to mice that received the freeze-dried transgenic tomatoes from which the concentrates were made. Figs. 5G and 5H show plasma triglyceride levels for the mice in Figs. 5C and. 5D, respectively, compared to mice that received the freeze-dried transgenic tomatoes from which the concentrates were made. The data shown are Mean ± SEM. NS = Not Significant; Tg6F = tomato concentrate from transgenic tomatoes expressing the 6F peptide; EV = tomato concentrate from transgenic tomatoes expressing the marker protein, β-glucuronidase. The experiment was done once.

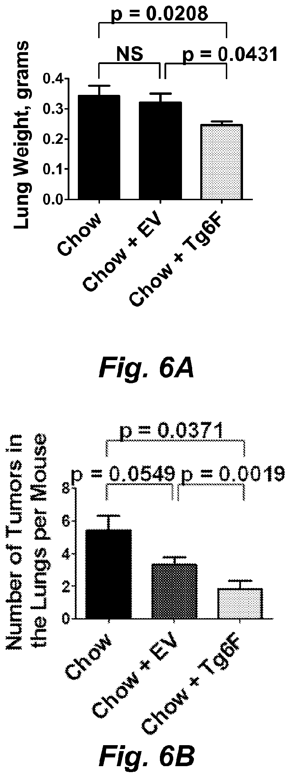

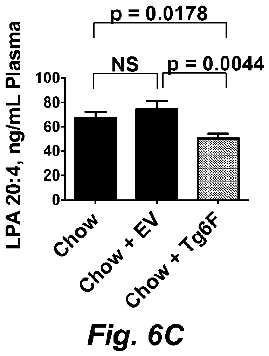

Figures 6A-6C. Tomato concentrate containing the 6F peptide (Tg6F), but not control tomato concentrate (EV) reduced metastasis of colon cancer cells to the lungs. Female BALB/c mice 6 weeks of age (n = 12 per group) were injected with 2 x 10⁴ CT26 cells in 100 µL of PBS via tail vein as described in Example 3 Materials and Methods. After injection the mice were maintained on either standard mouse chow (Chow), or standard mouse chow containing 0.06% by weight of the control transgenic tomato concentrate (EV), or standard mouse chow containing 0.06% by weight of the transgenic 6F tomato concentrate (Tg6F), which provided the mice with a dose of tomato concentrate of ∼120 mg/kg/day per mouse, which provided the mice with a dose of the 6F peptide of ∼7 mg/kg/day. After 4 weeks the mice were subjected to a terminal bleed, and after sacrifice the lungs were harvested, weighed and fixed with Bouin's solution, and the number of tumor nodules on the surface of the lungs was determined as described in Example 3 Materials and Methods. Fig. 6A shows the weight of the lungs in grams. Fig. 6B shows the number of tumor nodules on the surface of the lungs. Fig. 6C shows the plasma levels of lysophosphatidic acid 20:4 (LPA 20:4). The data shown are Mean ± SEM; NS = Not Significant. These results are representative of two of two experiments.

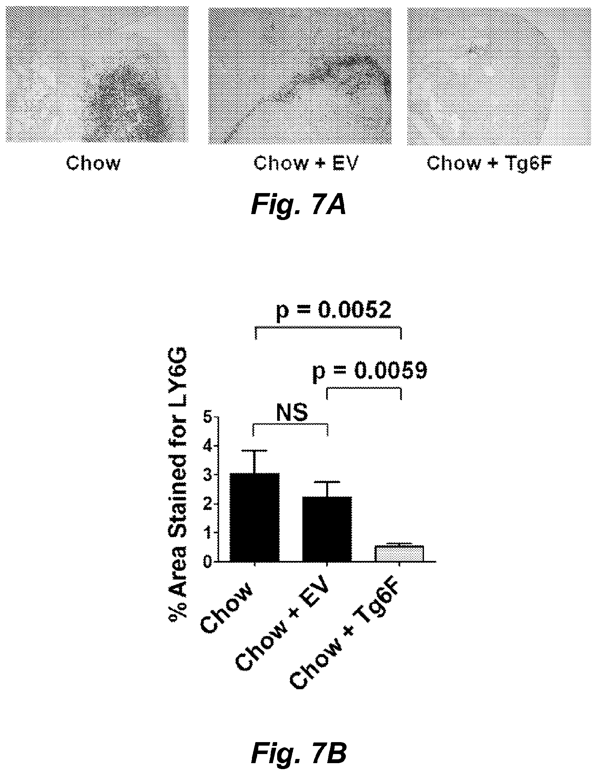

Figure 7A-7B. Tumor-associated neutrophils are dramatically reduced by tomato concentrate containing the 6F peptide (Tg6F), but not control tomato concentrate (EV) in a mouse model of colon cancer cells metastasizing to the lungs. Tumor-associated neutrophils in the lungs of the mice described in Figs. 6A-6C were determined as described in Example 3 Materials and Methods. Fig. 7A shows representative examples from each treatment group. Fig. 7B shows the % area stained for Ly6G (Mean ± SEM). NS = Not Significant. The experiment was done once.

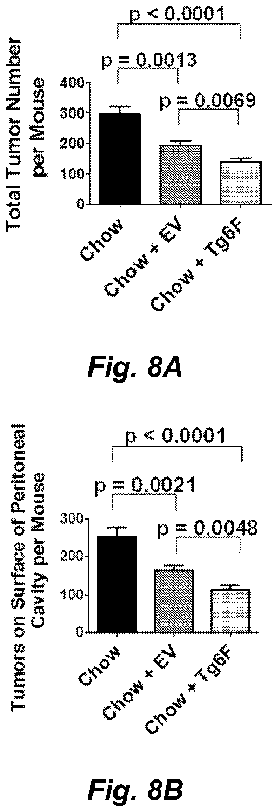

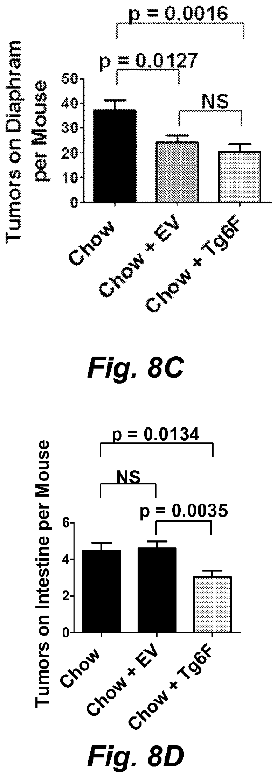

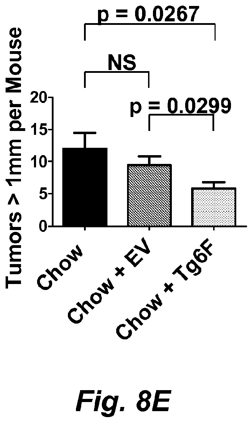

Figures 8A-8E. Both the control tomato concentrate (EV) and the tomato concentrate containing the 6F peptide (Tg6F) significantly reduced ovarian cancer cell tumor burden, but the tomato concentrate containing the 6F peptide was significantly more effective. Female C57BL/6J mice at age 9 weeks (n = 24 per group) were given an intraperitoneal injection containing 8 x 10⁶ ID8 cells in a total volume of 0.8 mL of DMEM (without supplements). Following injection of the ID8 cells the mice were maintained on either standard mouse chow (Chow), or standard mouse chow containing 0.06% by weight of the control transgenic tomato concentrate (EV), or containing 0.06% by weight of the transgenic 6F tomato concentrate (Tg6F), which provided the mice with a dose of the 6F peptide of ∼7 mg/kg/day. After 12 weeks the mice were subjected to a terminal bleed, and after sacrifice the number of tumor nodules on peritoneal surfaces and on the surface of abdominal organs was determined as described in Example 3 Materials and Methods. Fig. 8A shows the total number of tumor nodules in the abdomen. Fig. 8B shows the number of tumor nodules on the surface of the peritoneal cavity. Fig. 8C shows number of tumor nodules on the diaphragm. Fig. 8D shows the number of tumor nodules on the surface of the intestine. Fig. 8E shows the number of tumor nodules in the abdomen that were greater than 1 mm in size. The data shown are Mean ± SEM; NS = Not Significant. These results are representative of two of two experiments.

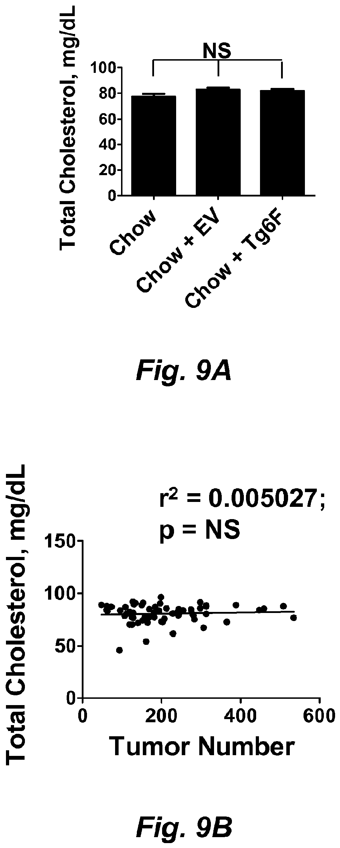

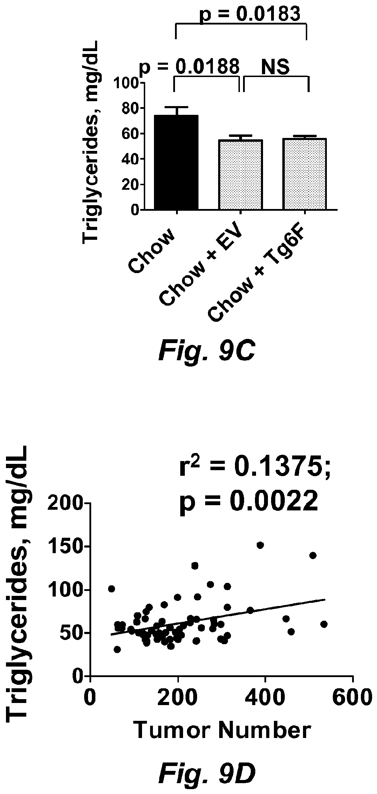

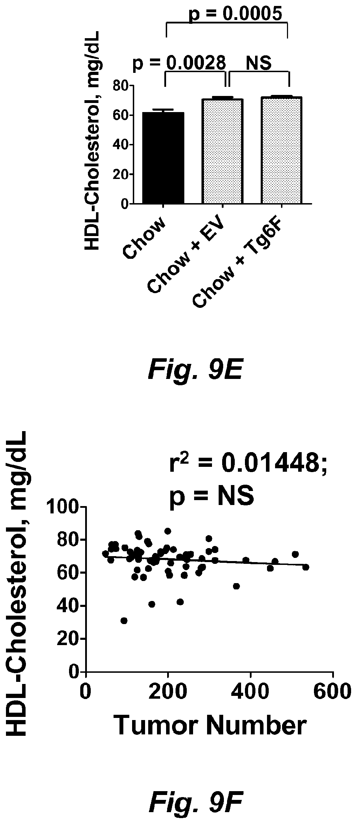

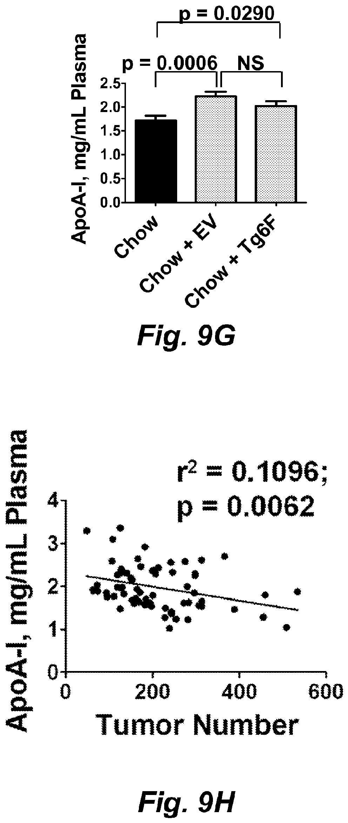

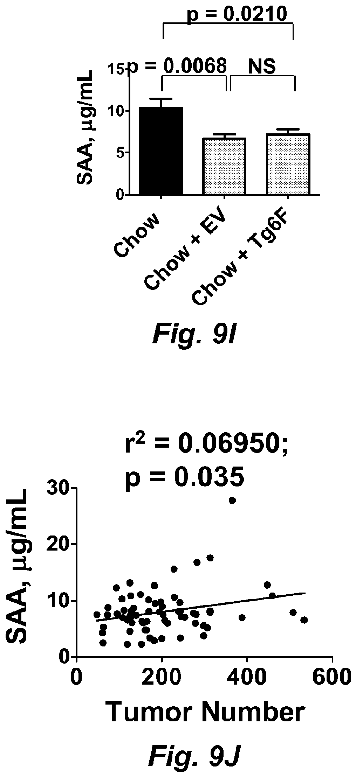

Figures 9A-9J. Plasma lipids and plasma serum amyloid A (SAA) levels in mice after intraperitoneal injection of ovarian cancer cells. Plasma lipid levels and SAA levels were measured in the mice described in Figure 8A-8E. Abbreviations are as in Figure 8. Fig. 9A shows plasma total cholesterol levels. Fig. 9B shows the linear correlation between plasma total cholesterol levels and the total number of tumors in the abdomen. Fig. 9C shows plasma triglyceride levels. Fig. 9D shows the linear correlation between plasma triglyceride levels and the total number of tumors in the abdomen. Fig. 9E shows plasma HDL-cholesterol levels. Fig. 9F shows the linear correlation between plasma HDL-cholesterol levels and the total number of tumors in the abdomen. Fig. 9G shows plasma apoA-I levels. Fig. 9H shows the linear correlation between plasma apoA-I levels and the total number of tumors in the abdomen. Fig. 9I shows plasma serum amyloid A (SAA) levels. Fig. 9J shows the linear correlation between plasma SAA levels and the total number of tumors in the abdomen. The data shown in the bar graphs are Mean ± SEM; NS = Not Significant. This experiment was done once.

### DETAILED DESCRIPTION

Methods of concentrating apolipoprotein A-I mimetic peptide activity in transgenic plant material expressing such peptides are provided, as defined in the claims. It was a surprising discovery that a simple, efficient, and inexpensive concentration protocol can reduce the amount of transgenic plant material required to produce a given bioactivity by as much as 97%.

We previously discovered a means to transgenically express apoA-I memetic peptides (*e.g.,* the 6F peptide) in various plants including tomatoes (*see, e.g.,* PCT Publication No: WO 2013/148214 A1). Without any purification steps, when the transgenic tomatoes were freeze-dried, ground into powder and fed to a mouse model of dyslipidemia and atherosclerosis at only 2.2% of a high-fat high-cholesterol diet by weight, the transgenic tomatoes significantly reduced dyslipidemia, inflammation and atherosclerosis in the mice.

This amout of plant matter provided a daily dose to mice of approximately 40 mg/kg/day. Delivery of an equivalent doage to a human would require the human to eat approximately 150 grams of freeze-dried tomato powder three times daily. While this is clearly possible, the volume of tomato powder required would be approximately 3 cups three times daily. The concentration methods described herein reduces the volume of plant matter (*e.g.,* tomato powder) required to only 3 - 4% of that volume. Such a dramatic decrease in volume will make compliance much more likely for patients.

### Methods of concentrating Apolipoprotein A-I activity of peptides expressed in transgenic plants.

In various embodiments, the methods of concentration ApoA-I activity comprises providing a tissue of transgenic plant that expresses one or more peptides that have ApoA-I activity where the tissue is provided as a substantially dry powder. The powder is mixed with a solution comprising ethyl acetate and acetic acid or with a solution comprising ethyl lactate and lactic acid, to form an extraction mixture. In various embodiments, the mixture is incubated (*e.g*., at room temperature, or at an elevated temperature (*e.g*., up to about 30°C, or up to about 35°C, or up to about 37°C or 38°C, or up to about 40°C) for at least 1 minute, or at least about 5 minutes, or at least about 10 minutes, or least about 15 minutes, or at least about 1/2 hour, or at least about 1 hour, or at least about 2 hours, or at least about 3 hours, or at least about 4 hours, or at least about 5 hours, or at least about 6 hours, or at least about 7 hours, or at least about 8 hours, or at least about 12 hours, or at least about 18 hours, or at least about 24 hours, or at least about 48 hours, or at least about 72 hours, or at least about 96 hours. In certain embodiments the mixture can be agitated during the incubation period.

In certain embodiments the incubation is in a solution comprising ethyl acetate and acetic acid. In certain embodiments this solution comprises about 1% to about 25% acetic acid, or about 2% to about 20% acetic acid, or about 3% to about 15 % acetic acid, or about 4% to about 10 % acetic acid, or about 4% to about 8% acetic acid, or from about 4% to about 6% acetic acid, or about 5% acetic acid.

In certain embodiments the incubation is in a solution comprising ethyl lactate and lactic acid. In certain embodiments this solution comprises about 1% to about 25% lactic acid, or about 2% to about 20% lactic acid, or about 3% to about 15 % lactic acid, or about 4% to about 10 % lactic acid, or about 4% to about 8% lactic acid, or from about 4% to about 6% lactic acid, or about 5% lactic acid.

After incubation, the liquid phase of the mixture is collected and dried to provide a concentrated dry powder extract that displays the biological activity of the apoA-I mimetic peptide. In certain embodiments this collecting and drying can comprise drying the liquid phase to produce a dry residue and optionally, resuspending said residue in water (*e.g.* distilled water, de-ionized water, food-grade water) or a buffer to provide a resuspended mixture; and optionally drying the resuspended mixture to provide the dry powder extract.

In various embodiments the drying operations can be performed using any conventional drying procedure including, but not limited to lyophilization, drying at room temperature (*e.g.,* under a dry gas such as argon), drying at elevated temperature, drying at low pressure, and the like.

The resulting extract can optionally be compounded with any edible components for administration to a subject.

In various embodiments the resulting extract provides ApoA-I activity at least 5-fold greater per unit weight or at least 10 fold greater per unit weight, or at least 15 fold greater per unit weight or at least 20 fold greater per unit weight than the ApoA-I activity of the dry plant tissue starting material. Illustrative ApoA-I activities include, but are not limited to producing a decrease in plasma levels of lyophosphatidic acid (LPA), and/or producing a decrease SAA level, and/or that produces an increase in plasma paroxonase activity in a mouse model (*e.g.,* as described herein).

While the concentration protocol is illustrated with respect to a transgenic tomato plant expressing a heterologous 6F peptide, it is believe the same protocols can be effectively utilized with any of a number of other plants/plant tissues expressing any one or more peptides having ApoA-I activity.

Thus, in various embodiments the transgenic plant tissue comprises a tissue from a transgenic plant such as tomatoes, carrots, potatoes, apples, pears, plums, peaches, oranges, kiwis, papayas, pineapples, guava, lilikoi, starfruit, lychee, mango, grape, pomegranate, mustard greens, kale, chard, lettuce, soybean, rice, corn and other grains (*e.g.,* wheat, rice, barley, bulgur, faro, kamut, kañiwa, millet, oats, quinoa, rice, rye, sorghum, spelt, teff, triticale, and the like), berries such as strawberries, blueberries, blackberries, goji berries, and raspberries, banana, rice, turnip, maize, grape, fig, plum, potato, safflower seeds, nuts (*e.g.,* almond, walnut, pecan, peanut, cashew, macademia, hazelnut, *etc*.), legumes (*e.g.,* alfalfa, clover, peas, beans (including black beans), lentils, lupins, mesquite, carob, soybeans, and the like). In certain embodiments the plant is selected from the group consisting of tomato, rice, tobacco, turnip, maize, corn, soybean, grape, fig, plum, potato, carrot, pomegranate, mustard greens, chard, kale, lettuce, broccoli, and safflower seeds.

In certain embodiments the tissue of the transgenic plant comprise a fruit (*e.g.* a tomato). In certain embodiments the tissue of the transgenic plant comprises a seed, a leaf, a root, a tuber, a flower, and the like.

### ApoA-I mimetics and other peptides for expression in plants and concentration using the methods described herein.

Having demonstrated that the 6F peptide when expressed in a plant (*e.g.,* a tomato) shows significant biological activity when the plant or plant part is fed to a mammal without purification of the peptide away from the plant tissue, and substantially greater activity when concentrated using the methods described above, it is believed that a similar result can be obtained with any of a number of other therapeutic peptides comprising or consisting of domains that are therapeutic peptide sequences and these results can be obtained by expression of the peptide(s) in other plants, *e.g.,* as described herein and in PCT Publication No: WO 2013/148214 A1 and in Chattopadhyay et al. (2013) J. Lipid Res., 54: 995-10101, and Navab et al. (2013) J. Lipid Res., 54: 3403-3418.

In certain embodiments these peptides include, but are not limited to class A amphipathic helical peptides, class A amphipathic helical peptide mimetics of apoA-I having aromatic or aliphatic residues in the non-polar face, Apo-J (G* peptides), apoE peptides, and the like, and peptide mimetics, *e.g*., as described below.

### ApoA-I mimetic peptides.

In certain embodiments the peptides expressed in a transgenic plant comprise or consist of apoA-I mimetic peptides. In certain embodiments such peptides include, but are not limited to, class A amphipathic helical peptides, *e.g.* as described in U.S. Patent 6,664,230, and PCT Publications WO 02/15923 and WO 2004/034977. It was discovered that peptides comprising a class A amphipathic helix ("class A peptides"), in addition to being capable of mitigating one or more symptoms of atherosclerosis are also useful in the treatment of one or more of the other indications described herein.

Class A peptides are characterized by formation of an α-helix that produces a segregation of polar and non-polar residues thereby forming a polar and a nonpolar face with the positively charged residues residing at the polar-nonpolar interface and the negatively charged residues residing at the center of the polar face *(see, e.g.,* Anantharamaiah (1986) Meth. Enzymol, 128: 626-668). It is noted that the fourth exon of apo A-I, when folded into 3.667 residues/turn produces a class A amphipathic helical structure.

Significant biological activity has been demonstrated for various apoA-I mimetic peptides including, but not limited to the peptides designated 4F, retro (reverse 4F), 5F, 6F, and the like. Various class A peptides inhibited lesion development in atherosclerosis-susceptible mice. In addition, the peptides show varying, but significant degrees of efficacy in mitigating one or more symptoms of the various pathologies described herein. A number of such peptides described in PCT patent application Nos: PCT/US2001/026497 (WO 2002/015923), PCT/US2003/032442 (WO 2004/034977), PCT/US2008/085409, and in Bielicki et al. (2010) J. Lipid Res. 51: 1496-1503, Zheng et al. (2011) Biochemistry 50: 4068-4076, Di Bartolo et al. (2011) Lipids in Health and Disease 10: 224. In certain embodiments the peptides expressed in the transgenic plants comprise one or more domains that have an amino acid sequence shown in Table 1 or the reverse sequence.

**Table 1. Certain ApoA-I mimetic peptides that can be expressed in transgenic plants, e.g., as described herein. The table includes various class A and/or class Y peptide analogs. For each sequence listed in this table, the retro form of the sequence is also contemplated. Thus, for example where the 6F peptide sequence DWLKAFYDKFFEKFKEFF (SEQ ID NO:1) is shown, the retro amino acid sequence FFEKFKEFFKDYFAKLWD (SEQ ID NO:2) is also contemplated.**

| **Peptide Name** | **Amino Acid Sequence** | **SEQ ID NO.** |
|---|---|---|
| 18A | DWLKAFYDKVAEKLKEAF | 7 |
| 2F | DWLKAFYDKVAEKLKEAF | 7 |
| 3F | DWFKAFYDKVAEKLKEAF | 8 |
| 3F14 | DWLKAFYDKVAEKFKEAF | 9 |
| 4F | DWFKAFYDKVAEKFKEAF | 3 |
| 5F | DWLKAFYDKVFEKFKEFF | 10 |
| 6F | DWLKAFYDKFFEKFKEFF | 1 |
| 7F | DWFKAFYDKFFEKFKEFF | 11 |
| | DWLKAFYDKVAEKLKEFF | 12 |
| Rev18A | FAEKLKEAVKDYFAKLWD | 13 |
| Rev2F | FAEKLKEAVKDYFAKLWD | 13 |
| Ref3F | FAEKLKEAVKDYFAKFWD | 14 |
| Rev4F | FAEKFKEAVKDYFAKFWD | 4 |
| Rev5F | FFEKFKEFVKDYFAKLWD | 15 |
| Rev6F | FFEKFKEFFKDYFAKLWD | 2 |
| Rev7F | FFEKFKEFFKDYFAKFWD | 16 |
| | DWLKAFYDKVFEKFKEAF | 17 |
| | DWLKAFYDKVFEKLKEFF | 18 |
| | DWLKAFYDKVAEKFKEFF | 19 |
| | DWLKAFYDKVFEKFKEFF | 10 |
| | EWLKLFYEKVLEKFKEAF | 20 |
| | EWLKAFYDKVAEKFKEAF | 21 |
| | EWLKAFYDKVAEKLKEFF | 22 |
| | EWLKAFYDKVFEKFKEAF | 23 |
| | EWLKAFYDKVFEKLKEFF | 24 |
| | EWLKAFYDKVAEKFKEFF | 25 |
| | EWLKAFYDKVFEKFKEFF | 26 |
| | AFYDKVAEKLKEAF | 27 |
| | AFYDKVAEKFKEAF | 28 |
| | AFYDKVAEKFKEAF | 28 |
| | AFYDKFFEKFKEFF | 29 |
| | AFYDKFFEKFKEFF | 29 |
| | AFYDKVAEKFKEAF | 28 |
| | AFYDKVAEKLKEFF | 30 |
| | AFYDKVFEKFKEAF | 31 |
| | AFYDKVFEKLKEFF | 32 |
| | AFYDKVAEKFKEFF | 33 |
| | KAFYDKVFEKFKEF | 34 |
| | LFYEKVLEKFKEAF | 35 |
| | AFYDKVAEKFKEAF | 28 |
| | AFYDKVAEKLKEFF | 30 |
| | AFYDKVFEKFKEAF | 31 |
| | AFYDKVFEKLKEFF | 32 |
| | AFYDKVAEKFKEFF | 33 |
| | AFYDKVFEKFKEFF | 36 |
| | DWLKALYDKVAEKLKEAL | 37 |
| | DWFKAFYEKVAEKLKEFF | 38 |
| | DWFKAFYEKFFEKFKEFF | 39 |
| | EWLKALYEKVAEKLKEAL | 40 |
| | EWLKAFYEKVAEKLKEAF | 41 |
| | EWFKAFYEKVAEKLKEFF | 42 |
| | EWLKAFYEKVFEKFKEFF | 43 |
| | EWLKAFYEKFFEKFKEFF | 44 |
| | EWFKAFYEKFFEKFKEFF | 45 |
| | DFLKAWYDKVAEKLKEAW | 46 |
| | EFLKAWYEKVAEKLKEAW | 47 |
| | DFWKAWYDKVAEKLKEWW | 48 |
| | EFWKAWYEKVAEKLKEWW | 49 |
| | DKLKAFYDKVFEWAKEAF | 50 |
| | DKWKAVYDKFAEAFKEFL | 51 |
| | EKLKAFYEKVFEWAKEAF | 52 |
| | EKWKAVYEKFAEAFKEFL | 53 |
| | DWLKAFVDKFAEKFKEAY | 54 |
| | EKWKAVYEKFAEAFKEFL | 53 |
| | DWLKAFVYDKVFKLKEFF | 55 |
| | EWLKAFVYEKVFKLKEFF | 56 |
| | DWLRAFYDKVAEKLKEAF | 57 |
| | EWLRAFYEKVAEKLKEAF | 58 |
| | DWLKAFYDRVAEKLKEAF | 59 |
| | EWLKAFYERVAEKLKEAF | 60 |
| | DWLKAFYDKVAERLKEAF | 61 |
| | EWLKAFYEKVAERLKEAF | 62 |
| | DWLKAFYDKVAEKLREAF | 63 |
| | EWLKAFYEKVAEKLREAF | 64 |
| | DWLKAFYDRVAERLKEAF | 65 |
| | EWLKAFYERVAERLKEAF | 66 |
| | DWLRAFYDKVAEKLREAF | 67 |
| | EWLRAFYEKVAEKLREAF | 68 |
| | DWLRAFYDRVAEKLKEAF | 69 |
| | EWLRAFYERVAEKLKEAF | 70 |
| | DWLKAFYDKVAERLREAF | 71 |
| | EWLKAFYEKVAERLREAF | 72 |
| | DWLRAFYDKVAERLKEAF | 74 |
| | EWLRAFYEKVAERLKEAF | 74 |
| | DWLKAFYDKVAEKLKEAFPDWLKAFYD KVAEKLKEAF | 75 |
| | DWLKAFYDKVAEKLKEFFPDWLKAFYD KVAEKLKEFF | 76 |
| | DWFKAFYDKVAEKLKEAFPDWFKAFYD KVAEKLKEAF | 77 |
| | DKLKAFYDKVFEWAKEAFPDKLKAFYD KVFEWLKEAF | 78 |
| | DKWKAVYDKFAEAFKEFLPDKWKAVYD KFAEAFKEFL | 79 |
| | DWFKAFYDKVAEKFKEAFPDWFKAFYD KVAEKFKEAF | 80 |
| | DWLKAFVYDKVFKLKEFFPDWLKAFVY DKVFKLKEFF | 81 |
| | DWLKAFYDKFAEKFKEFFPDWLKAFYD KFAEKFKEFF | 82 |
| | EWFKAFYEKVAEKFKEAF | 83 |
| | DWFKAFYDKVAEKF | 84 |
| | FKAFYDKVAEKFKE | 85 |
| | FKAFYEKVAEKFKE | 86 |
| | FKAFYDKVAEKFKE | 85 |
| | FKAFYEKVAEKFKE | 86 |
| | DWFKAFYDKVAEKFKEAF | 3 |
| | EWFKAFYEKVAEKFKEAF | 83 |
| | AFYDKVAEKFKEAF | 28 |
| | DWFKAFYDKVAEKF | 3 |
| | DWLKAFYDKVFEKFKEFF | 10 |
| | EWLKAFYEKVFEKFKEFF | 43 |
| | AFYDKVFEKFKEFF | 36 |
| | AFYEKVFEKFKEFF | 87 |
| | DWLKAFYDKVFEKF | 88 |
| | EWLKAFYEKVFEKF | 89 |
| | LKAFYDKVFEKFKE | 90 |
| | LKAFYEKVFEKFKE | 91 |
| [Switch D-E]-1-4F | **E**WFKAFY**E**KVA**D**KFK**D**AF | 92 |
| [Switch D-E]-2-4F | **E**WFKAFYDKVADKFK**E**AF | 93 |
| [Switch D-E]-3-4F | DWFKAFY**E**KVA**D**KFKEAF | 94 |
| [Switch D-E]-4-4F | DWFKAFY**E**KVAEKFK**D**AF | 95 |
| 4F-2 | D**FW**KAFYDKVAEKFKEAF | 96 |
| [Switch D-E]-1-4F-2 | **E**FWKAFY**E**KVA**D**KFK**D**AF | 97 |
| [Switch D-E]-2-4F-2 | **E**FWKAFYDKVADKFK**E**AF | 98 |
| [Switch D-E]-3-4F-2 | DFWKAFY**E**KVA**D**KFKEAF | 99 |
| [Switch D-E]-4-4F-2 | DFWKAFY**E**KVAEKFK**D**AF | 100 |
| 4F-3 | DWFKA**YF**DKVAEKFKEAF | 101 |
| [Switch D-E]-1-4F-5 | **E**WFKAYF**E**KVADKFK**D**AF | 102 |
| [Switch D-E]-2-4F-5 | **E**WFKAYFDKVADKFK**E**AF | 103 |
| [Switch D-E]-3-4F-5 | DWFKAYF**E**KVA**D**KFKEAF | 104 |
| [Switch D-E]-4-4F-5 | DWFKAYF**E**KVAEKFK**D**AF | 105 |
| 4F-4 | DWFKAF**V**DK**Y**AEKFKEAF | 106 |
| [Switch D-E]-1-4F-4 | **E**WFKAFV**E**KYA**D**KFK**D**AF | 107 |
| [Switch D-E]-2-4F-4 | **E**WFKAFVDKYADKFK**E**AF | 108 |
| [Switch D-E]-3-4F-4 | DWFKAFV**E**KYA**D**KFKEAF | 109 |
| [Switch D-E]-4-4F | DWFKAFV**E**KYAEKFK**D**AF | 110 |
| 4-F-5 | DWFKAFYDK**AV**EKFKEAF | 111 |
| [Switch D-E]-1-4F-5 | **E**WFKAFY**E**KAV**D**KFK**D**AF | 112 |
| [Switch D-E]-2-4F-5 | **E**WFKAFYDKAVDKFK**E**AF | 113 |
| [Switch D-E]-3-4F-5 | DWFKAFY**E**KAV**D**KFKEAF | 114 |
| [Switch D-E]-4-4F-5 | DWFKAFY**E**KAVEKFK**D**AF | 115 |
| 4F-6 | DWFKAFYDKV**F**EK**A**KEAF | 116 |
| [Switch D-E]-1-4F-6 | **E**WFKAFY**E**KVF**D**KAK**D**AF | 117 |
| [Switch D-E]-2-4F-6 | **E**WFKAFYDKVFDKAK**E**AF | 118 |
| [Switch D-E]-3-4F-6 | DWFKAFY**E**KVF**D**KAKEAF | 119 |
| [Switch D-E]-4-4F-6 | DWFKAFY**E**KVFEKAK**D**AF | 120 |
| 4F-7 | DWFKAFYDKVAEK**A**KE**F**F | 121 |
| [Switch D-E]-1-4F-7 | **E**WFKAFY**E**KVA**D**KAK**D**FF | 122 |
| [Switch D-E]-2-4F-7 | **E**WFKAFYDKVADKAK**E**FF | 123 |
| [Switch D-E]-3-4F-7 | DWFKAFY**E**KVA**D**KAKEFF | 124 |
| [Switch D-E]-4-4F-7 | DWFKAFY**E**KVAEKAK**D**FF | 125 |
| 4F-8 | DWFKAFYDKVAEKFKE**FA** | 126 |
| [Switch D-E]-1-4F-8 | **E**WFKAFY**E**KVA**D**KFK**D**FA | 127 |
| [Switch D-E]-2-4F-8 | **E**WFKAFYDKVADKFK**E**FA | 128 |
| [Switch D-E]-3-4F-8 | DWFKAFY**E**KVA**D**KFKEFA | 129 |
| [Switch D-E]-4-4F-8 | DWFKAFY**E**KVAEKFK**D**FA | 130 |
| 4F-9 | D**A**FKAFYDKVAEKFKE**W**F | 131 |
| [Switch D-E]-1-4F-9 | **E**AFKAFY**E**KVA**D**KFK**D**WF | 132 |
| [Switch D-E]-2-4F-9 | **E**AFKAFYDKVADKFK**E**WF | 133 |
| [Switch D-E]-3-4F-9 | DAFKAFY**E**KVA**D**KFKEWF | 134 |
| [Switch D-E]-4-4F-9 | DAFKAFY**E**KVAEKFK**D**WF | 135 |
| 4F-10 | D**A**FKAFYDKV**W**EKFKEAF | 136 |
| [Switch D-E]-1-4F-10 | **E**AFKAFY**E**KVW**D**KFK**D**AF | 137 |
| [Switch D-E]-2-4F-10 | **E**AFKAFYDKVWDKFK**E**AF | 138 |
| [Switch D-E]-3-4F-10 | DAFKAFY**E**KVW**D**KFKEAF | 139 |
| [Switch D-E]-4-4F-10 | DAFKAFY**E**KVWEKFK**D**AF | 140 |
| 4F-11 | D**Y**FKAF**W**DKVAEKFKEAF | 141 |
| [Switch D-E]-1-4F-11 | **E**YFKAFW**E**KVA**D**KFK**D**AF | 142 |
| [Switch D-E]-2-4F-11 | **E**YFKAFWDKVADKFK**E**AF | 143 |
| [Switch D-E]-3-4F-11 | DYFKAFW**E**KVA**D**KFKEAF | 144 |
| [Switch D-E]-4-4F-11 | DYFKAFW**E**KVAEKFK**D**AF | 145 |
| 4F-12 | DW**A**KAFYDKVAEKFKE**F**F | 146 |
| [Switch D-E]-1-4F-12 | **E**WAKAFY**E**KVA**D**KFK**D**FF | 147 |
| [Switch D-E]-2-4F-12 | **E**WAKAFYDKVADKFKE**F**F | 148 |
| [Switch D-E]-3-4F-12 | DWAKAFY**E**KVA**D**KFKEFF | 149 |
| [Switch D-E]-4-4F-12 | DWAKAFY**E**KVAEKFK**D**FF | 150 |
| 4F-13 | DWFKA**A**YDKVAEKFKE**F**F | 151 |
| [Switch D-E]-1-4F-13 | **E**WFKAAY**E**KVA**D**KFK**D**FF | 152 |
| [Switch D-E]-2-4F-13 | **E**WFKAAYDKVADKFK**E**FF | 153 |
| [Switch D-E]-3-4F-13 | DWFKAAY**E**KVA**D**KFKEFF | 154 |
| [Switch D-E]-4-4F-13 | DWFKAAY**E**KVAEKFK**D**FF | 155 |
| 4F-14 | DWFK**A**FADKVAEKFKE**Y**F | 156 |
| [Switch D-E]-1-4F-14 | **E**WFKAFA**E**KVA**D**KFK**D**YF | 157 |
| [Switch D-E]-2-4F-14 | **E**WFKAFADKVADKFK**E**YF | 158 |
| [Switch D-E]-3-4F-14 | DWFKAFA**E**KVA**D**KFKEYF | 159 |
| [Switch D-E]-4-4F | DWFKAFA**E**KVAEKFK**D**YF | 160 |
| 4F-15 | DWFKAFYDK**A**AEKFKE**V**F | 161 |
| [Switch D-E]-1-4F-15 | **E**WFKAFY**E**KAA**D**KFK**D**VF | 162 |
| [Switch D-E]-2-4F-15 | **E**WFKAFYDKAADKFK**E**VF | 163 |
| [Switch D-E]-3-4F-15 | DWFKAFY**E**KAA**D**KFKEVF | 164 |
| [Switch D-E]-4-4F-15 | DWFKAFY**E**KAAEKFK**D**VF | 165 |
| 4F-16 | DW**Y**KAF**F**DKVAEKFKEAF | 166 |
| [Switch D-E]-1-4F-16 | **E**WYKAFF**E**KVA**D**KFK**D**AF | 167 |
| [Switch D-E]-2-4F-16 | **E**WYKAFFDKVADKFK**E**AF | 168 |
| [Switch D-E]-3-4F-16 | DWYKAFF**E**KVA**D**KFKEAF | 169 |
| [Switch D-E]-4-4F-16 | DWYKAFF**E**KVAEKFK**D**AF | 170 |
| 4F-17 | DW**V**KAFYDK**F**AEKFKEAF | 171 |
| [Switch D-E]-1-4F-17 | **E**WVKAFY**E**KFA**D**KFK**D**AF | 172 |
| [Switch D-E]-2-4F-17 | **E**WVKAFYDKFADKFK**E**AF | 173 |
| [Switch D-E]-3-4F-17 | DWVKAFY**E**KFA**D**KFKEAF | 174 |
| [Switch D-E]-4-4F-17 | DWVKAFY**E**KFAEKFK**D**AF | 175 |
| 4F-18 | DWFKAF**F**DKVAEK**Y**KEAF | 176 |
| [Switch D-E]-1-4F-18 | **E**WFKAFF**E**KVA**D**KYK**D**AF | 177 |
| [Switch D-E]-2-4F-18 | **E**WFKAFFDKVADKYK**E**AF | 178 |
| [Switch D-E]-3-4F-18 | DWFKAFF**E**KVA**D**KYKEAF | 179 |
| [Switch D-E]-3-4F-18 | DWFKAFF**E**KVA**D**KYKEAF | 180 |
| 4F-19 | DWFKAF**F**DKVAEKFKEA**Y** | 181 |
| [Switch D-E]-1-4F-19 | **E**WFKAFF**E**KVA**D**KFK**D**AY | 182 |
| [Switch D-E]-2-4F-19 | **E**WFKAFFDKVADKFK**E**AY | 183 |
| [Switch D-E]-3-4F-19 | DWFKAFF**E**KVA**D**KFKEAY | 184 |
| [Switch D-E]-4-4F-19 | DWFKAFF**E**KVAEKFK**D**AY | 185 |
| 4F-20 | DWFKAFYDKFAEK**F**KEA**V** | 186 |
| [Switch D-E]-1-4F-20 | **E**WFKAFY**E**KFA**D**KFK**D**AV | 187 |
| [Switch D-E]-2-4F-20 | **E**WFKAFYDKFADKFK**E**AV | 188 |
| [Switch D-E]-3-4F-20 | DWFKAFY**E**KFA**D**KFKEAV | 189 |
| [Switch D-E]-4-4F-20 | DWFKAFY**E**KFAEKFK**D**AV | 190 |
| 4F-21 | D**K**FKAFYDKVAEKF**W**EAF | 191 |
| [Switch D-E]-1-4F-21 | **E**KFKAFY**E**KVA**D**KFW**D**AF | 192 |
| [Switch D-E]-2-4F-21 | **E**KFKAFYDKVADKFW**E**AF | 193 |
| [Switch D-E]-3-4F-21 | DKFKAFY**E**KVA**D**KFWEAF | 194 |
| [Switch D-E]-4-4F-21 | DKFKAFY**E**KVAEKFW**D**AF | 195 |
| 4F-22 | D**KW**KAFYDKVAEKF**F**EAF | 196 |
| [Switch D-E]-1-4F-22 | **E**KWKAFY**E**KVA**D**KFF**D**AF | 197 |
| [Switch D-E]-2-4F-22 | **E**KWKAFYDKVADKFF**E**AF | 198 |
| [Switch D-E]-3-4F-22 | DKWKAFY**E**KVA**D**KFFEAF | 199 |
| [Switch D-E]-4-4F-22 | DKWKAFY**E**KVAEKFF**D**AF | 200 |
| 4F-23 | D**K**FKAFYDK**W**AE**V**FKEAF | 201 |
| [Switch D-E]-1-4F-23 | **E**KFKAFY**E**KWA**D**VFK**D**AF | 202 |
| [Switch D-E]-2-4F-23 | **E**KFKAFYDKWADVFK**E**AF | 203 |
| [Switch D-E]-3-4F-23 | DKFKAFY**E**KWA**D**VFKEAF | 204 |
| [Switch D-E]-4-4F-23 | DKFKAFY**E**KWAEVFK**D**AF | 205 |
| 4F-24 | D**K**FKAFYDKVAE**FW**KEAF | 206 |
| [Switch D-E]-1-4F-24 | **E**KFKAFY**E**KVA**D**FWK**D**AF | 207 |
| [Switch D-E]-2-4F-24 | **E**KFKAFYDKVADFWK**E**AF | 208 |
| [Switch D-E]-3-4F-24 | DKFKAFY**E**KVA**D**FWKEAF | 209 |
| [Switch D-E]-4-4F-24 | DKFKAFY**E**KVAEFWK**D**AF | 210 |
| Rev-4F | FAEKFKEAVKDYFAKFWD | 4 |
| [Switch D-E]-1-Rev-4F | FA**D**KFK**D**AVK**E**YFAKFW**E** | 211 |
| [Switch D-E]-2-Rev-4F | FA**D**KFKEAVKDYFAKFW**E** | 212 |
| [Switch D-E]-3-Rev-4F | FAEKFK**D**AVK**E**YFAKFWD | 213 |
| [Switch D-E]-4-Rev-4F | FAEKFK**D**AVKDYFAKFW**E** | 214 |
| Rev-4F-1 | F**W**EKFKEAVKDYFAKF**A**D | 215 |
| [Switch D-E]-1-Rev-4F-1 | FW**D**KFK**D**AVK**E**YFAKFA**E** | 216 |
| [Switch D-E]-2-Rev-4F-1 | FA**D**KFKEAVKDYFAKFW**E** | 212 |
| [Switch D-E]-3-Rev-4F-1 | FAEKFK**D**AVK**E**YFAKFWD | 213 |
| [Switch D-E]-4-Rev-4F-1 | FAEKFK**D**AVKDYFAKFW**E** | 214 |
| Rev-4F-2 | F**F**EKFKEAVKDYFAK**A**WD | 217 |
| [Switch D-E]-1-Rev-4F-2 | FF**D**KFK**D**AVK**E**YFAKAW**E** | 218 |
| [Switch D-E]-2-Rev-4F-2 | FF**D**KFKEAVKDYFAKAW**E** | 219 |
| [Switch D-E]-3-Rev-4F-2 | FFEKFK**D**AVK**E**YFAKAWD | 220 |
| [Switch D-E]-4-Rev-4F-2 | FFEKFK**D**AVKDYFAKAW**E** | 221 |
| Rev-4F-3 | FAEK**A**KE**F**VKDYFAKFWD | 222 |
| [Switch D-E]-1-Rev-4F-3 | FA**D**KAK**D**FVK**E**YFAKFW**E** | 223 |
| [Switch D-E]-2-Rev-4F-3 | FA**D**KAKEFVKDYFAKFW**E** | 224 |
| [Switch D-E]-3-Rev-4F-3 | FAEKAK**D**FVK**E**YFAKFWD | 225 |
| [Switch D-E]-4-Rev-4F-3 | FAEKAK**D**FVKDYFAKFW**E** | 226 |
| Rev-4F-4 | FAEKFKE**VA**KDYFAKFWD | 227 |
| [Switch D-E]-1-Rev-4F-4 | FA**D**KFK**D**VAK**E**YFAKFW**E** | 228 |
| [Switch D-E]-2-Rev-4F-4 | FA**D**KFKEVAKDYFAKFW**E** | 229 |
| [Switch D-E]-3-Rev-4F-4 | FAEKFK**D**VAK**E**YFAKFWD | 230 |
| [Switch D-E]-4-Rev-4F-4 | FAEKFK**D**VAKDYFAKFW**E** | 231 |
| Rev-4F-5 | FAEKFKEA**Y**KD**V**FAKFWD | 232 |
| [Switch D-E]-1-Rev-4F-5 | FA**D**KFK**D**AYK**E**VFAKFW**E** | 233 |
| [Switch D-E]-2-Rev-4F-5 | FA**D**KFKEAYKDVFAKFW**E** | 234 |
| [Switch D-E]-3-Rev-4F-5 | FAEKFK**D**AYK**E**VFAKFWD | 235 |
| [Switch D-E]-4-Rev-4F-5 | FAEKFK**D**AYKDVFAKFW**E** | 236 |
| Rev-4F-6 | FAEKFKEAVKD**FY**AKFWD | 237 |
| [Switch D-E]-1-Rev-4F-6 | FA**D**KFK**D**AVK**E**FYAKFW**E** | 238 |
| [Switch D-E]-2-Rev-4F-6 | FA**D**KFKEAVKDFYAKFW**E** | 239 |
| [Switch D-E]-3-Rev-4F-6 | FAEKFK**D**AVK**E**FYAKFWD | 240 |
| [Switch D-E]-4-Rev-4F-6 | FAEKFK**D**AVKDFYAKFW**E** | 241 |
| Rev-4F-7 | FAEKF**W**EAVKDYFAKF**K**D | 242 |
| [Switch D-E]-1-Rev-4F-7 | FA**D**KFW**D**AVK**E**YFAKFK**E** | 243 |
| [Switch D-E]-2-Rev-4F-7 | FA**D**KFWEAVKDYFAKFK**E** | 244 |
| [Switch D-E]-3-Rev-4F-7 | FAEKFW**D**AVK**E**YFAKFKD | 245 |
| [Switch D-E]-4-Rev-4F-7 | FAEKFW**D**AVKDYFAKFK**E** | 246 |
| Rev-4F-8 | **AF**EKFKEAVKDYFAKFWD | 247 |
| [Switch D-E]-1-Rev-4F-8 | AF**D**KFK**D**AVK**E**YFAKFW**E** | 248 |
| [Switch D-E]-2-Rev-4F-8 | AF**D**KFKEAVKDYFAKFW**E** | 249 |
| [Switch D-E]-3-Rev-4F-8 | AFEKFK**D**AVK**E**YFAKFWD | 250 |
| [Switch D-E]-4-Rev-4F-8 | AFEKFK**D**AVKDYFAKFW**E** | 251 |
| Rev-F-9 | **V**AEKFKEA**F**KDYFAKFWD | 252 |
| [Switch D-E]-1-Rev-4F-9 | VA**D**KFK**D**AFK**E**YFAKFW**E** | 253 |
| [Switch D-E]-2-Rev-4F-9 | VA**D**KFKEAFKDYFAKFW**E** | 254 |
| [Switch D-E]-3-Rev-4F-9 | VAEKFK**D**AFK**E**YFAKFWD | 255 |
| [Switch D-E]-4-Rev-4F-9 | VAEKFK**D**AFKDYFAKFW**E** | 256 |
| Rev-4F-10 | **Y**AEKFKEAVKD**F**FAKFWD | 257 |
| [Switch D-E]-1-Rev-4F-10 | YA**D**KFK**D**AVK**E**FFAKFW**E** | 258 |
| [Switch D-E]-2-Rev-4F-10 | YA**D**KFKEAVKDFFAKFW**E** | 259 |
| [Switch D-E]-3-Rev-4F-10 | YAEKFK**D**AVK**E**FFAKFWD | 260 |
| [Switch D-E]-4-Rev-4F-10 | YAEKFK**D**AVKDFFAKFW**E** | 261 |
| Rev-4F-11 | **A**AEKFKE**F**VKDYFAKFWD | 262 |
| [Switch D-E]-1-Rev-4F-11 | AA**D**KFK**D**FVK**E**YFAKFW**E** | 263 |
| [Switch D-E]-2-Rev-4F-11 | AA**D**KFKEFVKDYFAKFW**E** | 264 |
| [Switch D-E]-3-Rev-4F-11 | AAEKFK**D**FVK**E**YFAKFWD | 265 |
| Switch D-E]-4-Rev-4F-11 | AAEKFK**D**FVKDYFAKFW**E** | 266 |
| Rev-4F-12 | F**F**EK**A**KEAVKDYFAKFWD | 267 |
| [Switch D-E] - 1 -Rev-4F- 12 | FF**D**KAK**D**AVK**E**YFAKFW**E** | 268 |
| [Switch D-E]-2-Rev-4F-12 | FF**D**KAKEAVKDYFAKFW**E** | 269 |
| [Switch D-E]-3-Rev-4F-12 | FFEKAK**D**AVK**E**YFAKFWD | 270 |
| [Switch D-E]-4-Rev-4F-12 | FFEKAK**D**AVKDYFAKFW**E** | 271 |
| Rev-4F-13 | F**Y**EKFKEAVKD**A**FAKFWD | 272 |
| [Switch D-E]-1-Rev-4F-13 | FY**D**KFK**D**AVK**E**AFAKFW**E** | 273 |
| [Switch D-E]-2-Rev-4F-13 | FY**D**KFKEAVKDAFAKFW**E** | 274 |
| [Switch D-E]-3-Rev-4F-13 | FYEKFK**D**AVK**E**AFAKFWD | 275 |
| [Switch D-E]-4-Rev-4F-13 | FYEKFK**D**AVKDAFAKFW**E** | 276 |
| Rev-4F-14 | F**V**EKFKEA**A**KDYFAKFWD | 277 |
| [Switch D-E]-1-Rev-4F-14 | FV**D**KFK**D**AAK**E**YFAKFW**E** | 278 |
| [Switch D-E]-2-Rev-4F-14 | FV**D**KFKEAAKDYFAKFW**E** | 279 |
| [Switch D-E]-3-Rev-4F-14 | FVEKFK**D**AAK**E**YFAKFWD | 280 |
| [Switch D-E]-4-Rev-4F-14 | FVEKFK**D**AAKDYFAKFW**E** | 281 |
| Rev-4F-15 | FAEK**Y**KEAVKD**F**FAKFWD | 282 |
| [Switch D-E]-1-Rev-4F-15 | FA**D**KYK**D**AVK**E**FFAKFW**E** | 283 |
| [Switch D-E]-2-Rev-4F-15 | FA**D**KYKEAVKDFFAKFW**E** | 284 |
| [Switch D-E]-3-Rev-4F-15 | FAEKYK**D**AVK**E**FFAKFWD | 285 |
| [Switch D-E]-4-Rev-4F-15 | FAEKYK**D**AVKDFFAKFW**E** | 286 |
| Rev-4F-16 | FAEK**V**KEA**F**KDYFAKFWD | 287 |
| [Switch D-E]-1-Rev-4F-16 | FA**D**KVK**D**AFK**E**YFAKFW**E** | 288 |
| [Switch D-E]-2-Rev-4F-16 | FA**D**KVKEAFKDYFAKFW**E** | 289 |
| [Switch D-E]-3-Rev-4F-16 | FAEKVK**D**AFK**E**YFAKFWD | 290 |
| [Switch D-E]-4-Rev-4F-16 | FAEKVK**D**AFKDYFAKFW**E** | 291 |
| Rev-4F-17 | FAEKFKE**Y**VKD**A**FAKFWD | 292 |
| [Switch D-E]-1-Rev-4F-17 | FA**D**KFK**D**YVK**E**AFAKFW**E** | 293 |
| [Switch D-E]-2-Rev-4F-17 | FA**D**KFKEYVKDAFAKFW**E** | 294 |
| [Switch D-E]-3-Rev-4F-17 | FAEKFK**D**YVK**E**AFAKFWD | 295 |
| [Switch D-E]-4-Rev-4F-17 | FAEKFK**D**YVKDAFAKFW**E** | 296 |
| Rev-4F-18 | FAEKFKEA**F**KDY**V**AKFWD | 297 |
| [Switch D-E]-1-Rev-4F-18 | FA**D**KFK**D**AFK**E**YVAKFW**E** | 298 |
| [Switch D-E]-2-Rev-4F-18 | FA**D**KFKEAFKDYVAKFW**E** | 299 |
| [Switch D-E]-3-Rev-4F-18 | FAEKFK**D**AFK**E**YVAKFWD | 300 |
| [Switch D-E]-4-Rev-4F-18 | FAEKFK**D**AFKDYVAKFW**E** | 301 |
| Rev-4F-19 | FAEKFKEA**F**KDYFAK**V**WD | 302 |
| [Switch D-E]-1-Rev-4F-19 | FA**D**KFK**D**AFK**E**YFAKVW**E** | 303 |
| [Switch D-E]-2-Rev-4F-19 | FA**D**KFKEAFKDYFAKVW**E** | 304 |
| [Switch D-E]-3-Rev-4F-19 | FAEKFK**D**AFK**E**YFAKVWD | 305 |
| Switch D-E]-4-Rev-4F-19 | FAEKFK**D**AFKDYFAKVW**E** | 306 |
| Rev-4F-20 | FAEKFKEAVKD**F**FAK**Y**WD | 307 |
| [Switch D-E]-1-Rev-4F-20 | FA**D**KFK**D**AVK**E**FFAKYW**E** | 308 |
| [Switch D-E]-2-Rev-4F-20 | FA**D**KFKEAVKDFFAKYW**E** | 309 |
| [Switch D-E]-3-Rev-4F-20 | FAEKFK**D**AVK**E**FFAKYWD | 310 |
| [Switch D-E]-4-Rev-4F-20 | FAEKFK**D**AVKDFFAKYW**E** | 311 |
| Rev-4F-21 | **W**AEK**F**FEAVKDYFAKF**K**D | 312 |
| [Switch D-E]-1-Rev-4F-7 | WA**D**KFF**D**AVK**E**YFAKFK**E** | 313 |
| [Switch D-E]-2-Rev-4F-7 | WA**D**KFFEAVKDYFAKFK**E** | 314 |
| [Switch D-E]-3-Rev-4F-7 | WAEKFF**D**AVK**E**YFAKFKD | 315 |
| Switch D-E]-4-Rev-4F-7 | WAEKFF**D**AVKDYFAKFK**E** | 316 |
| Rev-4F-22 | FAEK**WF**EAVKDYFAKF**K**D | 317 |
| [Switch D-E]-1-Rev-4F-22 | FA**D**KWF**D**AVK**E**YFAKFK**E** | 318 |
| [Switch D-E]-2-Rev-4F-22 | FA**D**KWFEAVKDYFAKFK**E** | 319 |
| [Switch D-E]-3-Rev-4F-22 | FAEKWF**D**AVK**E**YFAKFKD | 320 |
| [Switch D-E]-4-Rev-4F-22 | FAEKWF**D**AVKDYFAKFK**E** | 321 |
| Rev-4F-23 | FAEKF**V**EA**W**KDYFAKF**K**D | 322 |
| [Switch D-E]-1-Rev-4F-23 | FA**D**KFV**D**AWK**E**YFAKFK**E** | 323 |
| [Switch D-E]-2-Rev-4F-23 | FA**D**KFVEAWKDYFAKFK**E** | 324 |
| [Switch D-E]-3-Rev-4F-23 | FAEKFV**D**AWK**E**YFAKFKD | 325 |
| [Switch D-E]-4-Rev-4F-23 | FAEKFV**D**AWKDYFAKFK**E** | 326 |
| Rev-4F-24 | F**Y**EKF**A**EAVKD**W**FAKF**K**D | 327 |
| [Switch D-E]-1-Rev-4F-24 | FY**D**KFA**D**AVK**E**WFAKFK**E** | 328 |
| [Switch D-E]-2-Rev-4F-24 | FY**D**KFAEAVKDWFAKFK**E** | 329 |
| [Switch D-E]-3-Rev-4F-24 | FYEKFA**D**AVK**E**WFAKFKD | 330 |
| [Switch D-E]-4-Rev-4F-24 | FYEKFA**D**AVKDWFAKFK**E** | 331 |
| [A-5>H]4F | DWFK**H**FYDKVAEKFKEAF | 332 |
| [A-5>H, D-E switched]4F | **E**WFK**H**FY**E**KVA**D**KFK**D**AF | 333 |
| [A-5>H, D-1>E]4F | **E**WFK**H**FYDKVAEKFKEAF | 334 |
| [A-5>H, D-8>E]4-F | DWFK**H**FY**E**KVAEKFKEAF | 335 |
| [A-5>H, E-12>D] 4F | DWFK**H**FYDKVA**D**KFKEAF | 336 |
| [A-5>H, E-16>D] 4F | DWFK**H**FYDKVAEKFK**D**AF | 337 |
| [F-3>H,A-5>F] -4F | DW**H**KF**F**YDKVAEKFKEAF | 338 |
| [F-3>H,A-5>F, D-E switched] -4F | **E**W**H**K**F**FY**E**KVA**D**KFK**D**AF | 339 |
| [F-3>H,A-5>F,D-1>E] -4F | **E**W**H**K**F**FYDKVAEKFKEAF | 340 |
| [F-3>H,A-5>F,D-8>E] -4F | DW**H**K**F**FY**E**KVAEKFKEAF | 341 |
| [F-3>H,A-5>F,E-12>D] -4F | DW**H**K**F**FYDKVA**D**KFKEAF | 342 |
| [F-3>H,A-5>F, E-16>D] -4F | DW**H**K**F**FYDKVAEKFK**D**AF | 343 |
| [A-5>F,F-6>H]4F | DWFK**FH**YDKVAEKFKEAF | 344 |
| [A-5>F,F-6>H,D-E switched]4F | **E**WFK**FH**Y**E**KVA**D**KFK**D**AF | 345 |
| [[A-5>F,F-6>H, D-1>E]4F | **E**WFK**FH**YDKVAEKFKEAF | 346 |
| [A-5>F,F-6>H, D-8>E]4F | DWFK**FH**Y**E**KVAEKFKEAF | 347 |
| [A-5>F,F-6>H, E-12>D]4F | DWFK**FH**Y**D**KVADKFKEAF | 348 |
| [A-5>F,F-6>H,E-16>D]4F | DWFK**FH**YDKVAEKFK**D**AF | 349 |
| [A-5>V, V-10>H]4F | DWFK**V**FYDK**H**AEKFKEAF | 350 |
| [A-5>V, V-10>H,D-E switched]4F | **E**WFK**V**FY**E**K**H**A**D**KFK**D**AF | 351 |
| [A-5>V, V-10>H,D-1>E]4F | **E**WFK**V**FYDK**H**AEKFKEAF | 352 |
| [A-5>V, V-10>H, D-8>E] 4F | DWFK**V**FY**E**K**H**AEKFKEAF | 353 |
| [A-5>V, V-10>H,E-12>D] 4F | DWFK**V**FYDK**H**A**D**KFKEAF | 354 |
| [A-5>V, V-10>H,E16>D] 4F | DWFK**V**FYDK**H**AEKFK**D**AF | 355 |
| [[A-17>H]4F | DWFKAFYDKVAEKFKE**H**F | 356 |
| [A-17>H, D-E switched] 4F | **E**WFKAFY**E**KVA**D**KFK**DH**F | 357 |
| [[A-17>H,D-1>E]4F | **E**WFKAFYDKVAEKFKE**H**F | 358 |
| [[A-17>H, D-8>E]4F | DWFKAFY**E**KVAEKFKE**H**F | 359 |
| [[A-17>H,E-12>D]4F | DWFKAFYDKVA**D**KFKE**H**F | 360 |
| [[A-17>H, E16>D]4F | DWFKAFYDKVAEKFK**DH**F | 361 |
| [A-17>F, F-18>H] 4F | DWFKAFYDKVAEKFKE**FH** | 362 |
| [A-17>F, F-18>H,D-E switched] 4F | **E**WFKAFY**E**KVA**D**KFK**DFH** | 363 |
| [A-17>F, F-18>H,D-1>E] -4F | **E**WFKAFYDKVAEKFKE**FH** | 364 |
| [A-17>F, F-18>H] 4F | DWFKAFYDKVAEKFKE**FH** | 365 |
| [A-17>F, F-18>H,D-8>E] -4F | DWFKAFY**E**KVAEKFKE**FH** | 366 |
| [A-17>F, F-18>H,E-12>D] 4F | DWFKAFYDKVAEKFKE**FH** | 367 |
| [A-17>F, F-18>H],E-16>D]-4F | DWFKAFYDKVAEKFK**DFH** | 368 |
| Rev-4F | FAEKFKEAVKDYFAKFWD | 4 |
| [A-2>H]Rev4F | F**H**EKFKEAVKDYFAKFWD | 369 |
| Rev-[A-2>H, D>E]-4F | F**H**EKFKEAVK**E**YFAKFW**E** | 370 |
| Rev-[A-2>H, E>D]4F | F**HD**KFK**D**AVKDYFAKFWD | 371 |
| [A-2>H, D-E switched] Rev-4F | F**HD**KFK**D**AVK**E**YFAKFW**E** | 372 |
| [A-2>H, E-3>D]Rev-4F | F**HD**KFKEAVKDYFAKFWD | 373 |
| [A-2>H, E-7>D]Rev-4F | F**H**EKFK**D**AVKDYFAKFWD | 374 |
| [A-2>H, D-11>E]Rev-4F | F**H**EKFKEAVK**E**YFAKFWD | 375 |
| [A-2>H, D-18>E]Rev-4F | F**H**EKFKEAVKDYFAKFW**E** | 376 |
| [F-1>H, A-2>F]Rev-4F | **HF**EKFKEAVKDYFAKFWD | 377 |
| [F-1>H, A-2>F,D-E switched]Rev-4F | **HFD**KFK**D**AVK**E**YFAKFW**E** | 378 |
| [F-1>H, A-2>F, D>E]Rev-4F | **HF**EKFKEAVK**E**YFAKFW**E** | 379 |
| [F-1>H, A-2>F,E-3>D]Rev-4F | **HFD**KFKEAVKDYFAKFWD | 380 |
| [F-1>H, A-2>F,E-7>D]Rev-4F | **HF**EKFK**D**AVKDYFAKFWD | 381 |
| [F-1>H, A-2>F,D-11>E]Rev-4F | **HF**EKFKEAVK**E**YFAKFWD | 382 |
| [F-1>H, A-2>F, D-18>E]Rev-4F | **HF**EKFKEAVKDYFAKFW**E** | 383 |
| [A-2>F, F-5>H] Rev D-4F | F**F**EK**H**KEAVKDYFAKFWD | 384 |
| [A-2>F, F-5>H,D-E switched] Rev D-4F | F**FD**K**H**K**D**AVK**E**YFAKFW**E** | 385 |
| [A-2>F, F-5>H, D>E] Rev D-4F | F**F**EK**H**KEAVK**E**YFAKFW**E** | 386 |
| [A-2>F, F-5>H,E>D] Rev D-4F | F**FD**K**H**K**D**AVK**D**YFAKFWD | 387 |
| [A-2>F, F-5>H,E-3>D] Rev D-4F | F**FD**K**H**KEAVKDYFAKFWD | 388 |
| [A-2>F, F-5>H,D-11>E] Rev D-4F | F**F**EK**H**KEAVK**E**YFAKFWD | 389 |
| [A-2>F, F-5>H,D-18>E] Rev D-4F | F**F**EK**H**KEAVKDYFAKFWE | 390 |
| [A-2>V, V-9>H] Rev D-4F | F**V**EKFKEA**H**KDYFAKFWD | 391 |
| [A-2>V, V-9>H,D-E switched] Rev D-4F | F**VD**KFK**D**A**H**K**E**YFAKFW**E** | 392 |
| [A-2>V, V-9>H,D>E] Rev D-4F | F**V**EKFKEA**H**K**E**YFAKFW**E** | 393 |
| [A-2>V, V-9>H,E>D] Rev D-4F | F**VD**KFK**D**A**H**KDYFAKFWD | 394 |
| [A-2>V, V-9>H,E-3>D] Rev D-4F | F**VD**KFKEA**H**KDYFAKFWD | 395 |
| [A-2>V, V-9>H,E-7>D] Rev D-4F | F**V**EKFK**D**A**H**KDYFAKFWD | 396 |
| [A-2>V, V-9>H,D-11>E] Rev D-4F | F**V**EKFKEA**H**K**E**YFAKFWD | 397 |
| [A-2>V, V-9>H,D-18>E] Rev D-4F | F**V**EKFKEA**H**KDYFAKFW**E** | 398 |
| [A-8>H]Rev-4F | FAEKFKE**H**VKDYFAKFWD | 399 |
| [A-8>H,D-E switched]Rev-4F | FA**D**KFK**DH**VK**E**YFAKFW**E** | 400 |
| [A-8>H,D>E]Rev-4F | FAEKFKE**H**VK**E**YFAKFW**E** | 401 |
| [A-8>H,E>D]Rev-4F | FA**D**KFK**DH**VKDYFAKFWD | 402 |
| [A-8>H,E-3>D]Rev-4F | FA**D**KFKE**H**VKDYFAKFWD | 403 |
| [A-8>H, E-7>D]Rev-4F | FAEKFK**DH**VKDYFAKFWD | 404 |
| [A-8>H, D-11>E]Rev-4F | FAEKFKE**H**VK**E**YFAKFWD | 405 |
| [A-8>H, D-18>E]Rev-4F | FAEKFKE**H**VKDYFAKFW**E** | 406 |
| [A-8>F,F-13>H]Rev-4F | FAEK**F**KEFVKDY**H**AKFWD | 407 |
| [A-8>F,F-13>H,D-E switched]Rev-4F | FA**D**KFK**DF**VK**E**Y**H**AKFW**E** | 408 |
| [A-8>F,F-13>H, E-3>D]Rev-4F | FA**D**KFKE**F**VKDY**H**AKFWD | 409 |
| [A-8>F,F-13>H, E-7>D]Rev-4F | FAEKFK**DF**VKDY**H**AKFWD | 410 |
| [A-8>F,F-13>H, E>D]Rev-4F | FA**D**KFK**DF**VKDY**H**AKFWD | 411 |
| [A-8>F,F-13>H,D>E]Rev-4F | FAEKFKE**F**VK**E**Y**H**AKFW**E** | 412 |
| [A-8>F,F-13>H,D-11>E]Rev-4F | FAEKFKE**F**VK**E**Y**H**AKFWD | 413 |
| [A-8>F,F-13>H, D-18>E]Rev-4F | FAEKFKE**F**VKDY**H**AKFW**E** | 414 |
| [A-8>F, F16>H]Rev.-4F | FAEKFKE**F**VKDYFAK**H**WD | 415 |
| [A-8>F, F16>H,D-E switched] Rev. -4F | FA**D**KFK**DF**VK**E**YFAK**H**W**E** | 416 |
| [A-8>F, F16>H,D>E]Rev.-4F | FAEKFKE**F**VK**E**YFAK**H**W**E** | 417 |
| [A-8>F, F16>H, E>D]Rev.-4F | FA**D**KFK**DF**VKDYFAK**H**WD | 418 |
| [A-8>F, F16>H,E-3>D]Rev.-4F | FA**D**KFKE**F**VKDYFAK**H**WD | 419 |
| [A-8>F, F16>H,E-7>D]Rev.-4F | FAEKFK**DF**VKDYFAK**H**WD | 420 |
| [A-8>F, F16>H,D-11>E]Rev.-4F | FAEKFKE**F**VK**E**YFAK**H**WD | 421 |
| [A-8>F, F16>H,D-18>E]Rev.-4F | FAEKFKE**F**VKDYFAK**H**W**E** | 422 |
| Rev-[D>E]-4F | FAEKFKEAVK**E**YFAKFW**E** | 423 |
| Rev-[E>D]4F | FA**D**KFK**D**AVKDYFAKFWD | 424 |
| Rev-R4-4F | FAE**R**FREAVKDYFAKFWD | 425 |
| Rev-R6-4F | FAEKF**R**EAVKDYFAKFWD | 426 |
| Rev-R10-4F | FAEKFKEAV**R**DYFAKFWD | 427 |
| Rev-R14 -4F | FAEKFKEAVKDYFA**R**FWD | 428 |
| Rev-[D>E]-4F | FAEKFKEAVK**E**YFAKFW**E** | 429 |
| Rev-[E>D]4F | FA**D**KFK**D**AVKDYFAKFWD | 430 |
| Rev-R4-4F | FAE**R**FREAVKDYFAKFWD | 431 |
| Rev-R6-4F | FAEKF**R**EAVKDYFAKFWD | 432 |
| Rev-R10-4F | FAEKFKEAV**R**DYFAKFWD | 433 |
| Rev-R14 -4F | FAEKFKEAVKDYFA**R**FWD | 428 |
| Rev-[D>E]-4F | FAEKFKEAVK**E**YFAKFW**E** | 434 |
| Rev-[E>D]4F | FA**D**KFK**D**AVKDYFAKFWD | 435 |
| Rev-R4-4F | FAE**R**FREAVKDYFAKFWD | 436 |
| Rev-R6-4F | FAEKF**R**EAVKDYFAKFWD | 437 |
| Rev-R10-4F | FAEKFKEAV**R**DYFAKFWD | 438 |
| Rev-R14 -4F | FAEKFKEAVKDYFA**R**FWD | 428 |
| Rev-R4-4F | FAE**R**FREAVKDYFAKFWD | 439 |
| Rev-R6-4F | FAEKF**R**EAVKDYFAKFWD | 440 |
| Rev-R10-4F | FAEKFKEAV**R**DYFAKFWD | 441 |
| Rev-R14 -4F | FAEKFKEAVKDYFA**R**FWD | 428 |
| Rev-[D>E]-4F | FAEKFKEAVK**E**YFAKFW**E** | 442 |
| Rev-[E>D]4F | FA**D**KFK**D**AVKDYFAKFWD | 443 |
| Rev-R4-4F | FAE**R**FREAVKDYFAKFWD | 444 |
| Rev-R6-4F | FAEKF**R**EAVKDYFAKFWD | 445 |
| Rev-R10-4F | FAEKFKEAV**R**DYFAKFWD | 446 |
| Rev-R14 -4F | FAEKFKEAVKDYFA**R**FWD | 428 |
| Rev3F-2 | LFEKFAEAFKDYVAKWKD | 447 |
| RevR4-3F-2 | LFERFAEAFKDYVAKWKD | 448 |
| RevR10-3F2 | LFEKFAEAFRDYVAKWKD | 449 |
| RevR15-3F-2 | LFEKFAEAFKDYVARWKD | 450 |
| Rev R17-3F-2 | LFEKFAEAFKDYVAKWRD | 451 |
| Rev[D>E]3F2 | LFEKFAEAFKEYVAKWKE | 452 |
| Rev[E>D]3F-2 | LFDKFADAFKDYVAKWKD | 453 |
| Rev-[E3>D]-3F-2 | LFDKFAEAFKDYVAKWKD | 454 |
| Rev-[E7>D]-3F-2 | LFEKFADAFKDYVAKWKD | 455 |
| Rev[D11>E]3F-2 | LFEKFAEAFKEYVAKWKD | 456 |
| Rev-[D18>E]3F-2 | LFEKFAEAFKDYVAKWKE | 457 |
| Rev3F-1 | FAEKAWEFVKDYFAKLKD | 458 |
| RevR4-3F-1 | FAERAWEFVKDYFAKLKD | 459 |
| RevR10-3F-1 | FAEKAWEFVKDYFAKLKD | 460 |
| RevR15-3F-1 | FAEKAWEFVKDYFAKLKD | 461 |
| RevR17-3F-1 | FAEKAWEFVKDYFAKLRD | 462 |
| Rev[D>E]3F-1 | FAEKAWEFVKEYFAKLKE | 463 |
| Rev[E>D}3F-1 | FADKAWDFVKDYFAKLKD | 464 |
| Rev[E3>D]-3F -1 | FADKAWEFVKDYFAKLKD | 465 |
| Rev[E7>D]3F -1 | FAEKAWDFVKDYFAKLKD | 466 |
| Rev-[D11>E] 3F-1 | FAEKAWEFVKEYFAKLKD | 467 |
| Rev-[D18>E]3F-1 | FAEKAWEFVKDYFAKLK**E** | 468 |
| Rev-5F | FFEKFKEFVKDYFAKLWD | 15 |
| Rev-[D>E]5F | FFEKFKEFVK**E**YFAKLW**E** | 469 |
| Rev-[E>D]5F | FF**D**KFK**D**FVKDYFAKLWD | 470 |
| Rev-R4-5F | FFE**R**FKEFVKDYFAKLWD | 471 |
| Rev-R6-5F | FFEKF**R**EFVKDYFAKLWD | 472 |
| Rev-R10-5F | FFEKFKEFV**R**DYFAKLWD | 473 |
| Rev-R15-5F | FFEKFKEFVKDYFA**R**LWD | 474 |
| Rev-[E3>D]-5F | FF**D**KFKEFVKDYFAKLWD | 475 |
| Rev-[E7>D]5F | FFEKFK**D**FVKDYFAKLWD | 476 |
| Rev-[D11>E]-5F | FFEKFKEFVK**E**YFAKLWD | 477 |
| Rev-[D18>E]-5F | FFEKFKEFVKDYFAKLW**E** | 478 |
| Rev-5F-2 | F**L**EKFKEFVKDYFAK**F**WD | 479 |
| Rev-[D>E]-5F-2 | FLEKFKEFVK**E**YFAKFW**E** | 480 |
| Rev-[E>D]-5F-2 | FL**D**KFK**E**FVKDYFAKFWD | 481 |
| Rev-[E3>D]-5F-2 | FL**D**KFKEFVKDYFAKFWD | 482 |
| Rev-[E7>D]-5F-2 | FLEKFK**D**FVKDYFAKFWD | 483 |
| Rev-[D11>E]-5F-2 | FLEKFKEFVK**E**YFAKFWD | 484 |
| Rev-[D18>E]-5F-2 | FLEKFKEFVKDYFAKFW**E** | 485 |
| Rev-R4-5F-2 | FLE**R**FKEFVKDYFAKFWD | 486 |
| Rev-R6-5F-2 | FLEKF**R**EFVKDYFAKFWD | 487 |
| RevR10-5F-2 | FLEKFKEFV**R**DYFAKFWD | 488 |
| Rev-R16-5F-2 | FLEKFKEFVKDYFA**R**FWD | 489 |
| Rev-6F | F**F**EK**F**KE**FF**KDYFAKLWD | 2 |
| Rev-[D>E]-6F | FFEKFKEFFK**E**YFAKLW**E** | 490 |
| Rev-[E>D]-6F | FF**D**KFK**D**FFKDYFAKLWD | 491 |
| Rev-R4-6F | FFE**R**FKEFFKDYFAKLWD | 492 |
| Rev-R6-6F | FFEKF**R**EFFKDYFAKLWD | 493 |
| Rev-R10-6F | FFE**K**FKEFF**R**DYFAKLWD | 494 |
| Rev-R14-6F | FFERFKEFFKDYFA**R**LWD | 495 |
| Rev-[E3>D]-6F | FF**D**KFKEFFKDYFAKLWD | 496 |
| Rev-[E7>D]-6F | FFEKFK**D**FFKDYFAKLWD | 497 |
| Rev-[D11>E]-6F | FFEKFKEFFK**E**YFAKLWD | 498 |
| Rev-[D18>E]-6F | FFEKFKEFFKDYFAKLW**E** | 499 |
| Rev-4F | FAEKFKEAVKDYFAKFWD | 4 |
| Rev-[D>E]-4F | FAEKFKEAVK**E**YFAKFW**E** | 500 |
| Rev-[E>D]4F | FA**D**KFK**D**AVKDYFAKFWD | 501 |
| Rev-R4-4F | FAE**R**FREAVKDYFAKFWD | 502 |
| Rev-R6-4F | FAEKF**R**EAVKDYFAKFWD | 503 |
| Rev-R10-4F | FAEKFKEAV**R**DYFAKFWD | 504 |
| Rev-R14 -4F | FAEKFKEAVKDYFA**R**FWD | 428 |
| 4F-2 | DKWKAVYDKFAEAFKEFF | 505 |
| [D>E]-4F-2 | EKWKAVYEKFAEAFKEFF | 506 |
| [E>D]-4F-2 | DKWKAVYDKFA**D**AFK**D**FF | 507 |
| R2-4F-2 | D**R**WKAVYDKFAEAFKEFF | 508 |
| R4-4F-2 | DKW**R**AVYDKFAEAFKEFF | 509 |
| R9-4F-2 | DKWKAVYD**R**FAEAFKEFF | 510 |
| R14-4F-2 | DKWKAVYDKFAEAF**R**EFF | 511 |
| Rev4F-2 | FFEKFAEAFKDYVAKWKD | 512 |
| Rev-[D>E]-4F-2 | FFEKFAEAFK**E**YVAKWK**E** | 513 |
| Rev-[E>D]-3F-2 | FF**D**KFA**D**AFKDYVAKWKD | 514 |
| Rev-R4-4F-2 | FFE**R**FAEAFKDYVAKWKD | 515 |
| Rev-R10-4F-2 | FFERFAEAF**R**DYVAKWKD | 516 |
| Rev-R15-4F-2 | FFEKFAEAFKDYVA**R**WKD | 517 |
| Rev-R17-4F-2 | FFE**R**FAEAFKDYVAKW**R**D | 518 |
| Rev-[E3>D]-4F-2 | FF**D**KFAEAFKDYVAKWKD | 519 |
| Rev-[E7>D]-4F-2 | FFEKFA**D**AFKDYVAKWKD | 520 |
| Rev-[D11>E]-4F-2 | FFERFAEAFK**E**YVAKWKD | 521 |
| Rev-[D18>E]-4F-2 | FFERFAEAFKDYVAKWK**E** | 522 |
| Rev-7F | FFEKFKEFFKDYFAKFWD | 16 |
| Rev-[E>D]-7F | FF**D**KFK**D**FFKDYFAKFWD | 523 |
| Rev-[D>E]-7F | FFEKFKEFFK**E**YFAKFW**E** | 524 |
| Rev-R4-7F | FFE**R**FKEFFKDYFAKFWD | 525 |
| Rev-R6-7F | FFEKF**R**EFFKDYFAKFWD | 526 |
| Rev-R10-7F | FFEKFKEFF**R**DYFAKFWD | 527 |
| Rev-R14-7F | FFEKFKEFFKDYFA**R**FWD | 528 |
| Rev-[E3>D]-7F | FF**D**KFKEFFKDYFAKFWD | 529 |
| Rev-[E7>D]7F | FFEKFK**D**FFKDYFAKFWD | 530 |
| Rev-[D11>E]-7F | FFEKFKEFFK**E**YFAKFWD | 531 |
| Rev-[D18>E]-7F | FFEKFKEFFKDYFAKFWE | 532 |
| | EVRAKLEEQAQQIRLQAEAFQARLKSW FEPLVE | 533 |
| | EVRAKLEEQAQQIRLQAEAFQARLKSW FE | 534 |
| | EVRSKLEEWFAAFREFAEEFLARLKS | 535 |
| | PVLDLFRELLNELLEALKQKLK | 536 |
| | DWLKAFYDKVAEKLKEAF- P- DWAKAAYDKAAEKAKEAA | 537 |
| | EELKEKLEELKEKLEEKL-P- EELKEKLEELKEKLEEKL | 538 |
| | EELKAKLEELKAKLEEKL-P- EELKAKLEELKAKLEEKL | 539 |
| | EKLKALLEKLLAKLKELL P- EKLKALLEKLLAKLKELL | 540 |
| | EWLKELLEKLLEKLKELL-P- EWLKELLEKLLEKLKELL | 541 |
| | EKFKELLEKFLEKFKELL-P- EKFKELLEKFLEKFKELL | 542 |
| | EKLKELLEKLLELLKKLL-P- EKLKELLEKLLELLKKLL | 543 |
| | EKLKELLEKLKAKLEELL-P- EKLKELLEKLKAKLEELL | 544 |
| | EKLKELLEKLLAKLKELL-P- EKLKELLEKLLAKLKELL | 545 |
| | EKFKELLEKLLEKLKELL-P- EKFKELLEKLLEKLKELL | 546 |
| | EKLKAKLEELKAKLEELL-P- EKLKAKLEELKAKLEELL | 547 |
| | EELKELLKELLKKLEKLL-P- ELKELLKELLKKLEKLL | 548 |
| | EELKKLLEELLKKLKELL-P- EELKKLLEELLKKLKELL | 549 |
| | EKLKELLEKLLEKLKELL-A- EKLKELLEKLLEKLKELL | 550 |
| | EKLKELLEKLLEKLKELL-AA- EKLKELLEKLLEKLKELL | 551 |
| | EKLKAKLEELKAKLEELL-P- EKAKAALEEAKAKAEELA | 552 |
| | EKLKAKLEELKAKLEELL-P- EHAKAALEEAKCKAEELA | 553 |
| | DHLKAFYDKVACKLKEAF-P- DWAKAAYDKAAEKAKEAA | 554 |
| | DWLKAFYDKVAEKLKEAF-P- DHAKAAYDKAACKAKEAA | 555 |
| | DWLKAFYDKVACKLKEAF-P- DWAKAAYNKAAEKAKEAA | 556 |
| | DHLKAFYDKVAEKLKEAF-P- DWAKAAYDKAAEKAKEAA | 557 |
| | VLESFKVSFLSALEEYTKKLNTQ | 558 |
| (3F^{cπ}) | DKWKAVYDKFAEAFKEFL | 51 |
| (3F^{Iπ}) | DKLKAFYDKVFEWAKEAF | 50 |

### Apo-J (G* peptides).

It was also discovered that peptides that mimic the amphipathic helical domains of apoJ are also capable of mitigating one or more symptoms of atherosclerosis and/or other pathologies described herein. Apolipoprotein J possesses a wide nonpolar face termed globular protein-like, or G* amphipathic helical domains. The class G amphipathic helix is found in globular proteins, and thus, the name class G. This class of amphipathic helix is characterized by a random distribution of positively charged and negatively charged residues on the polar face with a narrow nonpolar face. Because of the narrow nonpolar face this class does not readily associate with phospholipids. The G* of amphipathic helix possesses similar, but not identical, characteristics to the G amphipathic helix. Similar to the class G amphipathic helix, the G* class peptides possesses a random distribution of positively and negatively charged residues on the polar face. However, in contrast to the class G amphipathic helix which has a narrow nonpolar face, this class has a wide nonpolar face that allows this class to readily bind phospholipid and the class is termed G* to differentiate it from the G class of amphipathic helix.

A number of suitable G* amphipathic peptides are described U.S. Patent Nos: 6,930,085, and 7,638,494, and in PCT Publication No: PCT/US03/09988 (WO 2003/086326). In certain embodiments the G* (apoJ) peptides expressed in the transgenic plants comprise one or more domains that have an amino acid sequence shown in Table 2 or the reverse sequence.

**Table 2. Certain peptides related to G* amphipathic helical domains of apo J that can be expressed in transgenic plants, e.g., as described herein. For each sequence listed in this table, the retro form of the sequence is also contemplated. Thus, for example where the sequence DQYYLRVTTVA (SEQ ID NO:559) is shown, the amino acid sequence AVTTVRLYYQD (SEQ ID NO:601) is also contemplated.**

| **Amino Acid Sequence** | **SEQ ID NO** |
|---|---|
| DQYYLRVTTVA | 559 |
| ECKPCLKQTCMKFYARVCR | 560 |
| FSRASSIIDELFQD | 561 |
| IQNAVNGVKQIKTLIEKTNEE | 562 |
| LLEQLNEQFNWVSRLANL | 5 |
| LLEQLNEQFNWVSRLANLTEGE | 563 |
| LLEQLNEQFNWVSRLANLTQGE | 564 |
| LVGRQLEEFL | 6 |
| MNGDRIDSLLEN | 565 |
| NELQEMSNQGSKYVNKEIQNAVNGV | 566 |
| PCLKQTCMKFYARVCR | 567 |
| PFLEMIHEAQQAMDI | 568 |
| PGVCNETMMALWEECK | 569 |
| PKFMETVAEKALQEYRKKHRE | 570 |
| PSGVTEVWKLFDS | 571 |
| PSQAKLRRELDESLQVAERLTRKYNELLKSYQ | 572 |
| PTEFIREGDDD | 573 |
| QQTHMLDVMQD | 574 |
| RKTLLSNLEEAKKKKEDALNETRESETKLKEL | 575 |
| RMKDQCDKCREILSV | 576 |

### ApoE mimetic peptides

ApoE mimetic peptides have also been demonstrated to have activities similar to those described above for ApoA-I mimetic peptides, particularly with respect to neurological and/or ocular dysfunction *(see, e.g.,* Handattu et al. (2010) J. Lipid Res. 51: 3491-3499; Laskowitz et al. (2001) Experimental Neurology 167: 74-85; Minami et al. (2010) Molecular Neurodegeneration, 5:16; Bhattacharjee et al. (2008) Invest Ophthalmol Vis Sci. 49: 4263-4268; Li et al. 92010) J. Pharmacol. and Experimental Therapeutics 334: 106-115; Klein and Yakel (2004) Neurosci., 127: 563-567; Laskowitz et al. (2007) J. of Neurotrauma 24: 1093-1107; Christensen et al. (2011) J. Immunol., 186: 2535-2542; Croy et al. 92004) Biochemistry 43: 7328-7335). In certain embodiments the peptides expressed in the transgenic plants comprise one or more domains that have an apoE amino acid sequence or a dual ApoE/ApoA-I sequence shown in Table 3 or the reverse sequence.

**Table 3. Certain ApoE peptides that can be expressed in transgenic plants, e.g., as described herein. For each sequence listed in this table, the retro form of the sequence is also contemplated. Thus, for example where the sequence GIKKFLGSIWKFIKAFVG (SEQ ID NO:578) is shown, the amino acid sequence GVFAKIFKWISGLFKKIG (SEQ ID NO:577) is also contemplated.**

| **Amino Acid Sequence** | **SEQ ID NO** |
|---|---|
| **ApoE peptides:** | |
| GIKKFLGSIWKFIKAFVG | 578 |
| GFKKFLGSWAKIYKAFVG | 579 |
| GFRRFLGSWARIYRAFVG | 580 |
| TEELRVRLASHLRKLRKRLL | 581 |
| TEELRVRLASHLRKLRK | 582 |
| LRVRLASHLRKLRKRLL | 583 |
| RLASHLRKLRKRLL | 584 |
| SHLRKLRKRLL | 585 |
| LRKLRKRLL | 586 |
| LRKLRKRLLLRKLRKRLL | 587 |
| LRKLRKRLLLRKLRKRLLLRKLRKRLL | 588 |
| RQIKIWFQNRRMKWKKCLRVRLASHLRKLRKRLL | 589 |
| LRVRLASHLRKLRKRLL | 590 |
| EELRVRLASHLRKLRKRLLRDADDLQKRLAVYEEQAQQIRLQAEAFQARL KSWFEPLVEDM | 591 |
| CEELRVRLASHLRKLRKRLLRDADDLQKRLAVY | 592 |
| LRKLRKRLLRDADDLLRKLRKRLLRDADDL | 593 |
| TEELRVRLASHLRKLRKRLL | 594 |
| TEELRVRLASHLEKLRKRLL | 595 |
| TEELRVRLASHLRELRKRLL | 596 |
| LREKKLRVSALRTHRLELRL | 597 |

| **Dual ApoE and ApoA-I mimetic peptides:** | |
|---|---|
| LRKLRKRLLRDWLKAFYDKVAEKLKEAF | 598 |
| LRRLRRRLLRDWLKAFYDKVAEKLKEAF | 599 |
| RRRRRRRRRRDWLKAFYDKVAEKLKEAF | 600 |

It has been demonstrated that in certain embodiments, linking the receptor binding domain of apolipoprotein E (apoE) to a class A amphipathic helix can enhance internalization and degradation of LDL by fibroblasts and can lower plasma cholesterol and restore endothelial function (*see, e.g.,* Datta et al. (2000) Biochemistry 39: 213-220; Gupta et al. (2005) Circulation 111: 3112-3118).

Accordingly in certain embodiments, any of the peptides described herein, when expressed in a transgenic plant, can be expressed as a peptide also comprising an apoE receptor binding domain (*see, e.g.,* SEQ ID NOs:598-600 for illustrative examples).

In various embodiments, peptides comprising an oxpholipin domain such as Arg-Glu-Dpa-Thr-Gly-Leu-Ala-Trp-Glu-Trp-Trp-Arg-Thr-Val (SEQ ID NO:602), where Dpa (3,3'-diphenyl alanine) is substituted with Trp, Phe, or Ala are also contemplated. Oxpholipin peptides are described by Ruchala et al. (2010) PLoS ONE 5(4): e10181) and in PCT Publication No: PCT/US2010/046534 (WO/2011/031460), and where such peptides incorporate 3,3'-diphenylalanine, this residue is substituted with Trp, Phe, or Ala.

In addition to the sequences listed in Tables 1, 2, and 3 amino acid sequences comprising 1 conservative substitution, 2 conservative substitutions, 3 conservative substitutions, 4 conservative substitutions, 5 conservative substitutions, 6 conservative substitutions, 7 conservative substitutions, 8 conservative substitutions, 9 conservative substitutions, or 10 conservative substitutions are contemplated.

The foregoing peptides are intended to be illustrative and not limiting. In view of the surprising discovery that ApoA-I mimetic peptides and other related peptides can be expressed in a transgenic plant and can be effective when plant parts are administered to a mammal, one of skill in the art will recognized that numerous other such peptides can also be expressed in such plants and fed to a mammal to afford a similar utility.

### Construction and propagation of transgenic plants.

The construction and propagation of transgenic plants expressing ApoA-I peptides or mimetics thereof and suitable for concentration of ApoA-I activity as described herein is detailed in PCT Publication No: WO 2013/148214 A1 and in Chattopadhyay et al. (2013) J. Lipid Res., 54: 995-10101, and Navab et al. (2013) J. Lipid Res., 54: 3403-3418. The methods typically involve constructing a vector *(e.g.,* a plasmid vector) or a DNA fragment by operably linking a DNA sequence encoding the peptide(s) of interest (e.g., peptides comprising ApoA-I, and/or G*, and/or ApoE domain(s)) to a plant-functional promoter capable of directing the expression of the peptide in the plant and then transforming a plant cell with the plasmid vector or DNA fragment. Where preferred, the method may be extended to produce transgenic plants from the transformed cells by including a step of regenerating a transgenic plant from the transgenic plant cell. The resulting plants provide tissues, extracts of which having ApoA-I activity can be produced using the methods described herein.

Illustrative, but non-limiting methods of producing such transgenic plants are also described below.

### Nucleic acids and vectors expressing the peptide(s) of interest.

Typically, the codon usage of the nucleic acid that is to express the desired amino acid sequence(s) is selected to reflect the optimal codon usage in that plant. Methods of optimizing codon usage for expression of a nucleic acid in a particular host organism are known to those of skill in the art, and numerous software tools are available for such optimization. For example, codon tables are available from the Codon Usage Database, maintained by the Department of Plant Gene Research in Kazusa, Japan (*see, e.g.,* www.kazusa.or.jp/codon/).

In certain embodiments the codon optimized nucleic acid sequence is incorporated into an expression vector (*e.g.,* a plasmid). Typically the nucleic acid sequence is operably linked (put under control of) a promoter capable of directing expression of the nucleic acid sequence in the host plant.

### Promoters

Promoters that are known or found to cause transcription of a foreign gene in plant cells are well known to those of skill in the art. Such promoters include, for example, promoters of viral origin and promoters of plant origin. The promoters can be constitutive or inducible, and in various embodiments, are tissue-specific promoters. In various embodiments any of these promoters are contemplated for the expression of a peptide described herein in a plant/plant tissue.

The most common promoters used for constitutive overexpression in plants are derived from plant virus sources, such as the cauliflower mosaic (CaMV) 35S promoter (Odell et al. (1985) Nature, 313: 810-812). This promoter, like similar virally derived promoters used in plant systems, is harvested from double-stranded DNA viral genomes, which use host nuclear RNA polymerase and do not appear to depend on any trans-acting viral gene products. The CaMV 35S promoter delivers high expression in virtually all regions of the transgenic plant, is readily obtainable in research and academic settings, and available in plant transformation vector cassettes that allow for easy subcloning of the transgene of interest. The CaMV 35S promoter can drive high levels of transgene expression in both dicots and monocots (Battraw and Hall (1990) Plant Mol. Biol. 15: 527-538; Benfey et al. (1990) EMBO J. 9: 1677-1684). In various embodiments the full-sized 35S promoter (-941 to +9 bp) (Odell et al. (1985) Nature, 313: 810-812) or various fragments such as a 2343 bp fragment can be used. Other viral promoters are also well known to those of skill in the art. These include, but are not limited to the cassava vein mosaic virus (CsVMV) promoter *(see, e.g.,* Verdaguer et al. (1996) Plant Mol. Biol. 31: 1129-1139; Verdaguer et al. (1998) Plant Mol. Biol. 37: 1055-1067; Li et al. (2001) Plant Sci. 160: 877-887), Australian banana streak virus (BSV) promoters *(see, e.g.,* Schenk et al. (2001) Plant Mol. Biol. 47: 399-412), mirabilis mosaic virus (MMV) promoter *(see, e.g.,* Dey and Maiti (1999) Plant Mol. Biol. 40: 771-782), the figwort mosaic virus (FMV) promoter *(see, e.g.,* Sanger et al. (1990) Plant Mol. Biol. 14: 433-443;; Maiti et al. (1997) Transgenic Res. 6: 143-156) and the like.

Endogenous plant promoters are also used regularly to drive high constitutive levels of transgene expression (Gupta et al. (2001) Plant Biotechnol. 18: 275-282; Dhankher et al. (2002) Nature Biotechnol. 20: 1-6). A number of these strong constitutive promoters are derived from actin and ubiquitin genes. For example, the Act2 promoter was developed from the actin gene family in Arabidopsis (An et al. (1996) Plant J. 10: 107-121). The rice actin 1 gene promoter has also been developed for use in cereal systems (McElroy *et al.* (1991; Zhang et al. (1991) Plant Cell 3: 1155-1165) and drives expression in virtually all tissues except xylem when transformed back into rice. Ubiquitin promoters, for example the maize ubiquitin 1 promoter (pUbi) has provided high expression in of heterologous genes in maize protoplasts. The maize Ubi1 promoter: GUS fusion has been used in rice (Cornejo et al. (1993) Plant Mol. Biol. 23: 567-581). The Ubi.U4 gene promoter has also been shown to drive high expression activity (Garbarino et al. (1995) Plant Physiol. 109: 1371-1378).

A number of tissue-specific (*e.g.,* specific to fruit, seed/grain, tubers/root storage systems, florets/flowers, Leaves/green tissue, anthers/pollen, and the like) are known. Illustrative, but non-limiting fruit-specific promoters include, for example promoters from the 1-aminocyclopropane-1-carboxylate (ACC) oxidase gene, the E8 gene, and polygalacturonase (PG) genes have been characterized in apple (Atkinson et al. (1998) Plant Mol. Biol. 38: 449-460) and tomato (Montgomery et al. (1993) Plant Cell 5: 1049-1062; Nicholass et al. (1995) Plant Mol. Biol. 28: 423-435; Deikman and Fischer (1988) EMBO J. 7: 3315-3320). The promoter of the tomato E8 gene has been used successfully in a number of instances to target transgene expression to fruit. The promoter of the tomato polygalacturonase gene (PG gene product accumulates during ripening and is associated with fruit softening) has been used to drive expression of heterologous genes (Fraser et al. (2002) Eur. J. Biochem. 270: 1365-1380). In tomato, a single gene encodes PG, and analysis of a 1.4 kb promoter fragment shows that it also directs ripening-specific expression (Montgomery et al. (1993) Plant Cell 5: 1049-1062). Phytoene desaturase (Pds) is the second dedicated enzyme in carotenoid biosynthesis and is also encoded by a single gene in tomato (Giuliano et al. (1993) Plant Cell 5: 379-387). Because carotenoids accumulate in the chloroplasts and chromoplasts, the tomato Pds promoter (2.0 kb from start of translation) drives high levels of expression in organs and developing tissues where chromoplasts are found (fruits, petals, anthers) (Corona et al. (1996) Plant J. 9: 505-512).

Seed-specific transgene expression has been used for a number of genetic engineering applications. Illustrative seed specific promoters include, but are not limited to the promoters of various seed storage proteins. Other seed specific promoters include for example, those from the soybean β-conglycinin (Chen et al. (1989) Dev. Genet. 10: 112-122; Chamberland et al. (1992) Plant Mol. Biol. 19: 937-949; Lessard et al. (1993) Plant Mol. Biol. 5: 873-885), the sunflower helianthinin genes (Nunberg et al. (1994) Plant Cell 6: 473-486), and the like. One of the best-characterized and most commonly used seed-specific promoters is the French bean β-phaseolin gene *(see, e.g.,* Bustos et al. (1989) Plant Cell 1: 839-853; van der Geest and Hall (1997) Plant J. 6: 413-423). Another useful seed specific promoter is the cotton α-globulin promoter (Sunilkumar et al. (2002) Transgenic Res. 11: 347-359) and has been characterized in cotton, Arabidopsis, and tobacco. In monocots, several promoters of storage proteins include, but are not limited to the endosperm-specific hordein promoters in barley (Forde et al. (1985) Nucleic Acids Res. 13: 7327-7339), glutenin promoters from wheat (Lamacchia et al. (2001) J. Exp. Bot. 52: 243-250), the zein promoters in maize (Marzabal et al. (1998) Plant J. 16: 41-52), and the granule-bound starch synthase 1 (*gbss1*) gene in wheat (Kluth et al. (2002) Plant Mol. Biol. 49: 669-682).

Tubers/root storage specific promoters include, but are not limited to the potato class I patatin family members, B33 and PAT 21 (Jefferson *et al.* (1990; Liu *et al.* (1991), the potato granule-bound starch synthase (GBSS) promoter , sweet potato, sporamin and β-amylase promoters (Maeo et al. (2001) Plant Mol. Biol. 46: 627-637), *e.g.,* the gSPO-A1 promoter (Ohta et al. (1991) Mol. Gen. Genet. 225: 369-378).

Promoters specific to legume - rhizobium-associated root nodules include promoters of genes expressed early in nodule organogenesis (ENOD genes) (see, *e.g.,* Lauridsen et al. (1993) Plant J. 3: 484-492; Vijn et al. (1995) Plant Mol. Biol. 28: 1103-1110; Fang and Hirsch (1998) Plant Physiol. 116: 53-68; Hohnjec et al. (2000) Mol. Gen. Genet. 264: 241-250), late nodulin promoters (see, *e.g.,* Sandal et al. (1987) Nucleic Acids Res. 15: 1507-1519; Stougaard et al. (1987) EMBO J. 6: 3565-3569), leghemoglobin promoters, the *Sesbania rostrata* leghemoglobin *glb3* promoter (*see, e.g.,* Szabados et al. (1990) Plant Cell 2: 973-986; Szczyglowski et al. (1996) Plant Mol. Biol. 31: 931-935), and the like.

Root specific promoters are described, for example, by Yamamoto et al. (1991) Plant Cell 3: 371-382. Non-plant root-specific promoters include the promoters of the rooting loci (rol) genes found in the Ri (root-inducing) plasmid of *A. rhizogenes* (*e.g.,* the rolD promoter), Domain A of the CaMV 35S promoter (Benfey and Chua (1989) Plant Cell 2: 849-856), the TobRB7 promoter from tobacco (Yamamoto et al. (1991) Plant Cell 3: 371-382), and the like.

Promoters specific to leaves/green tissues include, but are not limited to, promoters from the rbcS multigene family encoding the small subunit of ribulose-1,5-bisphosphate carboxylase such as the pea rbcS-3A promoter the alfalfa rbcS promoter the Rubisco promoter, promoters from the chlorophyll a/b-binding (Cab) protein genes *(e.g.,* CAB2 promoter) (Piechulla et al. (1998) Plant Mol. Biol. 38: 655-662), the alfalfa 1532 bp RAc promoter, and the like.

Illustrative, but non-limiting examples of tissue specific promoters are shown in Table 4.

**Table 4 shows illustrative, but non-limiting examples of tissue specific promoters.**

| Tissue | Illustrative Promoters |
|---|---|
| Fruit specific | Apple ACC oxidase |
| | Tomato polygalactouronidase |
| | Tomato *E*8 |
| | Tomato *PDS* |
| Green tissue specific | Pea *rbcs-3A* |
| | Arabidopsis *CAB2* |
| | Alfalfa *RAc* |
| Nodule specific | *Vicia faba* VfEnod12 |
| | Bean NVP30 |
| | *S. rostrata* leghemoglobin |
| Root specific | *A. rhizogenes rolD* |
| | Domain A, CaMV 35S |
| | Tobacco TobRB7 |
| Tuber/storage organ specific | Potato patatin B33 |
| | Potato patatin PAT21 |
| | Potato GBSS |
| Seed specific | Bean beta-phaseolin |
| | Cotton alpha-globulin |
| | Wheat *gbssl* |
| | Zma10 Kz or Zmag12 (maize zein gene) |
| | Zmag12 (maize glutelin gene) |
| Seed coat specific | Pea GsGNS2 |
| Floral specific | Chrysanthemum *UEP1* |
| | Bean *CHS15* |
| | Petunia EPSPS |
| Pollen specific | Maize ZMC5 |
| | Tomato *lat*52 |
| Pistil specific | Pear *PsTL1* |
| | Potato *SK2* |

In certain embodiments, the peptide(s) described are expressed under the control of the CaMV promoter. As used herein, the phrase "CaMV 35S" promoter includes variations of CaMV 35S promoter, e.g. promoters derived by means of ligations with operator regions, random or controlled mutagenesis, *etc.*). In certain embodiments, the peptide(s) described herein are expressed under the control of the E8 promoter. In certain embodiments, the peptide(s) described herein are expressed under the control of the hybrid tomato E4/E8 plant promoter (see, *e.g.,* U.S. Patent 6,118,049).

### Vectors

As indicated above, the nucleic acid encoding the peptide(s) described herein is placed in a vector (*e.g.,* a plasmid vector) under control of the desired promoter. In certain embodiments the vector (*e.g.*, plasmid vector) can further encode one or more selectable markers (*e.g.,* an antibiotic resistance marker such as the *npt II* gene for kanamycin resistance) and markers that confer selection by hygromycin, streptomycin, spectinomycin, or phosphinotricin. Illustrative selectable markers for use in plants include, but are not limited to neomycin phosphotransferase, hygromycin phosphotransferase, dihydrofolate reductase, chloramphenicol acetyl transferase, gentamycin acetyl transferase, nopaline synthase, octopine synthase, p-galactosidase, p-glucuronidase, streptomycin phosphotransferase, bleomycin resistance, firefly luciferase, bacterial luciferase, threonine dehydratase, metallothionein i1, epsp synthase, phosphinothricin acetyl transferase, acetolactate synthase, bromoxynil nitrilase, and the like.

In certain embodiments the vector can encode a signal peptide (*e.g., ALPAH-*Al1*-Phaseolus vulgaris*)*.* Sequences that can be provided include, for example, a leader sequence (*e.g.*, to allow secretion or vacuolar targeting), and translation termination signals.

More generally a number of vectors for plant cell transformation and heterologous gene expression are known to those of skill in the art. For example, the structures of a wide array of plasmids that have proven effective in (a) plant transformation and expression of heterologous genes including constructs that confer resistance to kanamycin, hygromycin, streptomycin, spectinomycin and phosphinotricin, or that confer β-glucuronidase (GUS) gene expression are described by Jones et al.(1992) Transgenic Res., 1: 285-297. Binary vector constructs that carry polylinkers of the pUC and Bluescript types, plasmids that permit the expression of any heterologous reading frame from either nopaline synthase (nos) or octopine synthase (ocs) promoters, as well as the cauliflower mosaic virus 35S promoter, using either the nopaline synthase or octopine synthase 3' polyadenylation sequences, are also presented in this reference. These constructs permit a choice of orientation of the resulting transgene of interest, relative to the orientation of the selection marker gene. Most of the plasmids described by Jones *et al. (supra.)* are publicly/commercially available.

Illustrative and non-limiting examples of vectors include the pRL200 vector that has been used to stably transform lettuce (*see, e.g.,* Kanamoto et al. (2006) Transgenic Res., 15: 205-217), the *pCAMBI1381-GUS* plasmid has been used to target specific tissues in tomatoes (*see, e.g.,* Lim et al.(2012) Molecules and Cells 34: 53-59), the pSBS4642 vector, the chloroplast transformation vector pLD, and the like.

Means of constructing the heterologous "gene" and incorporating it into a plasmid are well known to those of skill in the art. For example the heterologous "gene" can be chemically synthesized using a DNA synthesizer. Commercial services can also provide nucleic acid sequences synthesized to order. The construct can then be cloned into the vector using, for example, PCR cloning procedures. Methods of making the nucleic acid constructs described herein are well known to those of skill in the art, and specific methods are illustrated in the examples. Cloning and transformation methods, DNA vectors and the use of regulatory sequences are well known to the skilled artisan and may for instance be found in Current Protocols in Molecular Biology, F. M. Ausubel et al, Wiley Interscience, 2004.

In certain embodiments the marker genes (e.g., selectable markers) are removed from the transgenic plant. Methods of removing selectable markers are well known to those of skill in the art. In one illustrative, but non-limiting approach the marker genes are eliminated using MAT vector systems. MAT (Multi-Auto-Transformation) vectors are designed to use the oncogenes (ipt, iaaM/H, rol) of Agrobacterium, which control the endogenous levels of plant hormones and the cell responses to plant growth regulators, to differentiate transgenic cells, and to select marker-free transgenic plants. The oncogenes are combined with the site-specific recombination system (R/RS). At transformation, the oncogenes regenerate transgenic plants and then are removed by the R/RS system to generate marker-free transgenic plants. Protocols for the choice of a promoter for the oncogenes and the recombinase (R) gene, the state of plant materials and the tissue culture conditions are described, for example, by Ebinuman et al. (2005) Meth. Mol. Biol., 286: 237-254.

### Host Plant Selection

A wide variety of plant species have been genetically transformed with foreign DNA, using several different techniques to insert genes (*see, e.g.,* Wu (1989) Pp. 35-15 In: Plant Biotechnology, Kung, S. and Arntzen, eds., Butterworth Publishers, Boston, Mass; Deak et al. (1986) Plant Cell Rep. 5, 97-100; McCormick et al. (1986) Plant Cell Rep., 5: 81-84; Shahin and Simpson (1986) Hort. Sci. 21: 1199-1201; Umbeck et al. (1987) Bio/Technology 5: 263-266; Christon et al. (1990) Trends Biotechnol. 8: 145-151; Datta et al. (1990) Bio/Technology 8: 736-740; Hinchee et al. (1988) Bio/Technology 6: 915-922; Raineri et al. (1990) Bio/Technology, 8: 33-38; Fromm et al. (1990) Bio/Technology 8: 833-839; and the like). Since many edible plants used by humans for food or as components of animal feed are dicotyledenous plants, in certain embodiments, it is preferred to employ dicotyledons for expression of the peptide(s) described herein, although monocotyledon transformation is also applicable especially in the production of certain grains useful for animal feed.

In certain embodiments the host plant selected for genetic transformation has edible tissue in which the peptide(s) of interest can be expressed. Thus, in various embodiments, the peptide(s) can be expressed in a part of the plant, such as the fruit, leaves, stems, seeds, or roots, which may be consumed by a human or an animal for which the peptide(s) are intended.

Various other considerations can inform selection of the host plant. It is sometimes preferred that the edible tissue of the host plant not require heating prior to consumption since the heating may reduce the effectiveness of apolipoprotein or mimetic for animal or human use. Also, it is sometimes preferred that the host plant express the peptide(s) in the form of a drinkable liquid.

In certain embodiments plants that are suitable for expression of the peptide(s) described herein include any dicotyledon or monocotyledon that is edible in part or in whole by a human or an animal. Illustrative plants include, for example, tomatoes, carrots, potatoes, apples, pears, plums, peaches, oranges, kiwis, papayas, pineapples, guava, lilikoi, starfruit, lychee, mango, grape, pomegranate, mustard greens, kale, chard, lettuce, soybean, rice, corn and other grains (*e.g*., wheat, rice, barley, bulgur, faro, kamut, kaniwa, millet, oats, quinoa, rice, rye, sorghum, spelt, teff, triticale, and the like), berries such as strawberries, blueberries, blackberries, goji berries, and raspberries, banana, rice, turnip, maize, grape, fig, plum, potato, safflower seeds, nuts (*e.g.*, almond, walnut, pecan, peanut, cashew, macademia, hazelnut, *etc.*), legumes (*e.g.,* alfalfa, clover, peas, beans (including black beans), lentils, lupins, mesquite, carob, soybeans, and the like), and the like. In certain embodiments expression in plants such as tobacco and the like, is also contemplated.

### Methods of Gene Transfer into Plants

Any of a number of transformation protocols can be used to transform the plant cells and plants described herein. While certain preferred embodiments described below utilize particular transformation protocols, it will be understood by those of skill in the art that any transformation method may be utilized within the definitions and scope of the invention.

There are a number of methods for introducing foreign genes into both monocotyledenous and dicotyledenous plants (*see, e.g.,* Potrykus (1991) Annu. Rev. Plant Physiol, Plant Mol. Biol. 42: 205-225; Shimamoto et al. (1989) Nature 338: 274-27, and the like. Methods for stable integration of exogenous DNA into plant genomic DNA include for example agrobacterium-mediated gene transfer, direct DNA uptake including methods for direct uptake DNA into protoplasts, DNA uptake induced by brief electric shock of plant cells, DNA injection into plant cells or tissues by particle bombardment, or by the use of micropipette systems, or by the direct incubation of DNA with germinating pollen; and the use of plant virus as gene vectors.

Plant transformation and regeneration in dicotyledons by Agrobacterium tumefaciens (A. tumefaciens) is well documented. The application of the Agrobacterium tumefaciens system with, for example, the leaf disc transformation method (*see, e.g.,* Horsch et al. (1988) Pp. 1-9 In: Plant Molecular Biology Manual AS, Kluwer Academic Publishers, Dordrecht) permits efficient gene transfer, selection and regeneration.

Monocotyledons have also been found to be capable of genetic transformation by *Agrobacterium tumefaciens* as well as by other methods such as direct DNA uptake mediated by PEG (polyethylene glycol), or electroporation. Successful transfer of foreign genes into corn *(see, e.g.,* Rhodes et al. (1989) Science 240: 204-207) and rice *(see, e.g.,* Toriyama et al. (1988) Bio/Technology 6: 1072-1074; Zhang and Wu (1988) Theor. Appl. Genet. 76: 835-840), tomato *(see, e.g.,* Frary and Earl (1996) Plant Cell Rept. 15: 235-240), as well as wheat and sorghum protoplasts, and numerous other species has been demonstrated.

The Agrobacterium system includes the use of plasmid vectors that contain defined DNA segments that integrate into the plant genomic DNA. Methods of inoculation of the plant tissue vary depending upon the plant species and the Agrobacterium delivery system. One illustrative approach is the leaf disc procedure which can be performed with any tissue explant that provides a good source for initiation of whole plant differentiation. The Agrobacterium system is especially viable in the creation of transgenic dicotyledenous plants.

As indicated above there are various methods of direct DNA transfer into plant cells. In electroporation, the protoplasts are briefly exposed to a strong electric field. In microinjection, the DNA is mechanically injected directly into the cells using very small micropipettes. In microparticle bombardment, the DNA is adsorbed on microprojectiles such as magnesium sulfate crystals or tungsten particles, and the microprojectiles are physically accelerated into cells or plant tissues.

Another method of vector transfer is the transmission of genetic material using modified plant viruses. DNA of interest is integrated into DNA viruses, and these viruses are used to infect plants at wound sites.

One method of transfection utilizing *Agrobacterium tumafaciens* is illustrated herein in the Examples. Using these teachings, numerous other plants can be similarly transformed. Those skilled in the art should recognize that there are multiple choices of Agrobacterium strains and plasmid construction strategies that can be used to optimize genetic transformation of plants. They will also recognize that *A. tumefaciens* may not be the only Agrobacterium strain used. Other Agrobacterium strains such as A. *rhizogenes* might be more suitable in some applications.

Methods of inoculation of the plant tissue vary depending upon the plant species and the Agrobacterium delivery system. A very convenient approach is the leaf disc procedure which can be performed with any tissue explant that provides a good source for initiation of whole plant differentiation. The addition of nurse tissue may be desirable under certain conditions. Other procedures such as the *in vitro* transformation of regenerating protoplasts with *A. tumefaciens* may be followed to obtain transformed plant cells as well.

It is noted that heterologous genes have been expressed in a wide variety of plants, particular edible plants. Thus, for example, a minimal peach chlorophyll *a*/*b-*binding protein gene (*Lhcb2*Pp*/) promoter (Cab19) and an enhanced *mas35S* CaMV promoter has been used to express heterologous genes in tomatoes (*see, e.g.,* Bassett et al. (2007) BMC Biotechnology 7: 47). A 35S::PtFT1 promoter (35S CaMV promoter) has been used successfully in plums (*see, e.g.,* Srinivasan *PLoS ONE* 7(7):e40715) and in apples (*see, e.g.,* Trankener et al. (2010) Planta 232: 1309-1324). Suc2 promoter sequence of the *A. Thaliana SUC2* gene (sucrose-H + symporter) has also been used (*Id.*). Another promoter used in apples was the Pgst1 promoter from potato (*see, e.g.,* Malnoy et al. (2006) Transgenic Res., 15: 83-93). The 35S CaMV promoter has been used in apples for many years (*see, e.g.,* Gleave (1992) Plant Mol Biol. 20: 1203-1207). Other promoters that are derivatives of the 35S CaMV promoter have been used in apples such as the potato proteinase inhibitor II (*Pin2*) promoter *(see, e.g.,* Ko et al. (2002) J. Amer. Soc. Hort. Sci. 127: 515-519). Butelli *et al.* used a binary vector (pDEL.ROS) containing both *Delila* and *Rosea1* cDNAs from snapdragon under the control of the *E8* promoter from tomato to produce tomatoes enriched in anthocyanins (*see e.g.,* Butelli et al. (2008) Nature Biotechnology 26: 1301-1308). Kesanakurti et al. (2012) Physiologia Plantarum 146: 136-148) used the E8 promoter to produce tomato plants to transgenically produce tomato anionic peroxidase (*tap1*)*.* Yang et al. (2012) Transgenic Res. 21: 1043-1056) demonstrated that the *Gentiana lutea* zeaxanthin epoxidase (*GlZEP*) promoter was highly expressed in transgenic tomato plants.

In view of the foregoing, one of skill will recognize that using the teachings and examples provided herein, any of peptides (*e.g*., apoA-I mimetic peptides) described herein can be expressed in an effective amount in a plant tissue with at most routine experimentation.

The resulting plant tissue that contains one or more peptides having the desired ApoA-I activity can then be concentrated suite the method described herein to produce a biologically activive (ApoA-I active) plant extract.

### Method of administering transgenic plants extracts for therapeutic and/or prophylactic use.

In various embodiments mMethods for the prophylaxis and/or treatment of various pathologies, especially pathologies characterized by an inflammatory response (*see, e.g.,* Table 5) are disclosed. In certain embodiments the methods involve administering to a mammal in need thereof (*e.g.,* a human, a non-human mammal) an extract of a at least a portion of a transgenic plant as described herein. In certain embodiments the extract is simply orally administered to the mammal. In certain embodiments the extract is administered in combination with a food, and/or a protein powder, and/or a nutritional supplement, and/or a "power bar", and/or a "defined diet".

In various embodiments the extracts described herein are used in the prophylaxis and/or treatment of pathologies that include, but are not limited to atherosclerosis, arthritis, cancer, diabetes, hepatic fibrosis, macular degeneration, kidney disease, obesity, osteoporosis, scleroderma, systemic lupus erythematosus, transplant vasculopathy, and vascular dementia.

In certain embodiments the pathology is atherosclerosis and the administration is for the treatment of disease or is a prophylactic administration. In certain embodiments, the prophylactic administration is to a subject (*e.g.,* a human or non-human mammal) showing one or more risk factors for atherosclerosis (*e.g*., obesity, family history, elevated cholesterol, hypertension, diabetes, metabolic syndrome, low levels of HDL-cholesterol, elevated levels of triglycerides, or levels of high sensitivity CRP that are in the upper half of normal or are frankly elevated, and the like).

In certain embodiments the pathology is a cancer and the administration is as a therapeutic method in its own right and/or to augment therapeutic methods and/or to reduce adverse side effects to therapeutic methods (*e.g.,* chemotherapy, radiotherapy, *etc.*). Various cancers for which the administration is believed to be suitable include, but are not limited to ovarian cancer, colon cancer, myeloma or multiple myeloma, breast cancer, bone cancer, cervical cancer, brain cancer, lung cancer, skin cancer including malignant melanoma, and prostate cancer.

In certain embodiments the administration of the extract is to prevent the onset, slow the onset and/or slow the progression of Alzheimer's disease and/or other dementia.

### Administration of transgenic plant extract.

In certain embodiments the mammal is administered the transgenic plant extract comprising or consisting of one or more apolipoprotein domains (*e.g*., 6F domains, 4F domains, *etc.*)*.* In certain embodiments the mammal is simply fed the extract as a powder, or in a solution or suspension, or formulated into a gel or pill. In certain embodiments the transgenic plant extract is combined with other dietary components (*e.g.,* as a food ingredient) for administration to the subject.

### Pills capsules and lozenges.

In certain embodiments the transgenic plant extracts having ApoA-I activity can be formulated for direct administration to a subject. Such formulations include, for example a simple powder that can be directly administered or combined with, *e.g.,* a drink for administration. Suitable unit dosage forms, include, but are not limited to powders, tablets, pills, capsules, lozenges, bars, and the like.

Such compositions can be manufactured by means of conventional mixing, dissolving, granulating, dragee-making, levigating, emulsifying, encapsulating, entrapping or lyophilizing processes.

For oral administration, the formulations can involve combining the transgenic plant extract(s) with carriers well known in the art. Such carriers enable the compounds of the invention to be formulated as tablets, pills, dragees, capsules, liquids, gels, syrups, slurries, suspensions and the like, for oral ingestion by a patient to be treated. For oral solid formulations such as, for example, powders, capsules and tablets, suitable excipients include fillers such as sugars, such as lactose, sucrose, mannitol and sorbitol; cellulose preparations such as maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP); granulating agents; and binding agents. If desired, disintegrating agents may be added, such as the cross-linked polyvinylpyrrolidone, agar, or alginic acid or a salt thereof such as sodium alginate. If desired, solid dosage forms may be sugar-coated or enteric-coated using standard techniques.

For oral liquid preparations such as, for example, suspensions, elixirs and solutions, suitable carriers, excipients or diluents include water, glycols, oils, alcohols, *etc.* Additionally, flavoring agents, preservatives, coloring agents and the like can be added. For buccal administration, the compositions may take the form of tablets, lozenges, *etc.* formulated in conventional manner.

### Protein powder

In certain embodiments the mammal is administered a "protein powder comprising a transgenic plant extract as described herein. In certain embodiments the protein powder further comprise an additional protein. Illustrative proteins include, but are not limited to whey protein (e.g., whey concentrate, whey isolate, and whey hydrolysate), casein protein (or milk protein), soy protein, egg-white protein, hemp seed protein, rice protein, pea protein, and the like.

Methods of isolating/producing protein powder are well known to those of skill in the art. Typical methods involve a crude isolation step (*e.g.,* filtering processes to separate lactose from milk in the preparation of whey protein) followed by a concentration step, *e.g.,* an ion exchange purification to purify the protein without denaturing it. In certain embodiments the transgenic plant extract(s) described herein are simply added to a commercially available protein powder.

### Food or food ingredient comprising a plant or plant part.

In certain embodiments the mammal is administered a food or a food ingredient that is eaten before or after or in combination with the transgenic plant extract(s) described herein. In certain embodiments, the food is a food product that naturally comprises all or a part of the same type of plant from which the transgenic plant extract is derived. Thus, where the extract is a transgenic tomato extract, the food may also comprise a tomato, tomato paste or sauce, and the like.

### Nutritional supplement.

In certain embodiments the transgenic plant extract(s) described herein are provided as a component of a nutritional supplement (*e.g.*, a vitamin supplement, a protein supplement, *etc.*)*.* Illustrative vitamin supplements include, for example, vitamin A supplements, vitamin B supplements, vitamin D supplements, vitamin C supplements, fatty acid supplements (*e.g*., omega 3 fatty acids), mineral supplements such as calcium, zinc, and iron, and various combinations thereof.

In certain embodiments the transgenic plant extract(s) described herein are provided as a component of a multivitamin formulation or combined in a multi-component package with other vitamin/FA/mineral supplements. In certain embodiments where the extract is used in such a supplement the extract, *e.g*., provided as a fine powder is then incorporated into a multivitamin, or tableted or encapsulated by itself. In certain embodiments the vitamin supplement comprises vitamin A, and/or vitamin B1, and/or B2, and/or B6 and/or B12, and/or vitamin C, and/or vitamin E, and/or a fatty acid.

### Defined diet/meal replacement product.

In certain embodiments the transgenic plant extract(s) described herein are provided as a component of a "defined diet" and/or meal replacement products (MRPs). A defined diet is a diet, optionally pre-packaged, that is intended to meet all the dietary requirements of a particular subject. For example, for humans a defined diet can be a predetermined diet designed to facilitate a particular dietary goal (*e.g.*, weight reduction, reduction of allergens, lactose, weight gain, protein elevation, *etc.).* In the case of non-human mammals *(e.g.,* canines, felines, porcines, equines, bovines, *etc.)* the "defined diet" can be provided in the form an animal food product. The animal food product can be designed to meet particular dietary goals, *e.g.,* as described above for a human.

In certain embodiments, the animal food product can be provided as the component of a treatment regimen *(e.g.,* for a farm animal, pet, *etc.)* afflicted with, or at risk for, a particular pathology, *e.g.,* cancer, atherosclerosis, kidney disease, *etc.*

Meal replacement products are a form of defined diet, either pre-packaged powdered drink mixes or edible bars designed to replace prepared meals. MRPs are generally high in protein, low in fat, have a low to moderate amount of carbohydrates, and contain a wide array of vitamins and minerals. The majority of MRPs use whey protein, casein (often listed as calcium caseinate or micellar casein), soy protein, and/or egg albumin as protein sources. Carbohydrates are typically derived from maltodextrin, oat fiber, brown rice, and/or wheat flour. Some MRPs also contain flax oil powder as a source of essential fatty acids. MRPs can also contain other ingredients. These can include, but are not limited to creatine monohydrate, glutamine peptides, L-glutamine, calcium alpha-ketoglutarate, additional amino acids, lactoferrin, conjugated linoleic acid, and medium chain triglycerides.

In certain embodiments the "defined diet" comprises one or more food items. Each food item may be individually prepackaged. In addition, one or more of the food items may be nutritionally enhanced by fortification of vitamins and minerals and/or by incorporation of transgenic plant extract(s) described herein,

The individual food items may be prepared by processing, *e.g*., mixing, precooking, cooking, freezing, dehydrating or freeze-drying, such that the meal may be maintained in a frozen or dry condition for an extended period. Additionally, an individual food item may be packaged in such a way that, before consumption, the food item must be mixed by hand or blender, cooked by placing the food component on a stove top, in an oven or microwave, or prepared by adding cool, hot or boiling water or by submerging the food item into boiling water. One or more of the food items may be shelf-stable. Preferably, a food item has a sufficiently long storage or shelf-life such that defined diet may be stored in advance of consumption. In certain embodiments a storage or shelf-life under retail conditions in a range of about six to twelve months is desirable.

In certain embodiments individual food items may be in the form of solids, semi-solids or liquids and may include, but are not limited to, soup products, protein supplements, grain foods, starch foods, fruit or vegetables foods, nutritional drinks and beverages.

In certain embodiments the transgenic plant extract(s) described herein are simply combined with/incorporated into the defined diet and/or meal replacement product (MRP). For example, in certain embodiments, the extract powder is added to one or more of the food components comprising the defined diet or MRP.

### Power bars.

In certain embodiments the transgenic plant extract(s) described herein are provided as a component of a "power bar"/energy bar. Energy bars are supplemental bars that typically contain cereals and/or dried fruit(s), and/or other high energy foods and/or fiber targeted at people that require quick energy or that are on certain weight loss regimens, but do not have time for a meal. They are different from energy drinks, which contain caffeine, whereas bars provide food energy.

Numerous power bar formulations are known to those of skill in the art. In certain embodiments the peptide comprising or consisting of one or more apolipoprotein domains is incorporated into the power bar as a protein (amino acid) component. In certain embodiments the transgenic plant or at least a portion thereof is provided as a component of the power bar. In various embodiments the plant can be provided as all or a portion of a fruit and/or fiber component of the power bar formulation.

### Animal uses.

As indicated above, in various embodiments, the transgenic plant extract(s) described herein are is provided as a component of an animal diet. The diet can be provided to simply maintain a healthy animal or in certain embodiments, the diet is optimized to facilitate a prophylactic or therapeutic effect.

Illustrative animal diets include, but are not limited to diets for juvenile animals, diets for normal adult animals, diets for old animals, weight loss diets, dental health diets, thyroid health diets, gastrointestinal health diets, hypoallergenic diets, kidney health diets, bladder health diets, aging diets, and the like. In certain embodiments the diet is a diet optimized for treatment of an animal with kidney disease and/or with cancer. In certain embodiments the diet is designed for administration to an animal receiving chemotherapy and/or radiotherapy.

In certain embodiments the transgenic plant extract(s) described herein are simply added to the diet as an additional food (*e.g.,* amino acid) source. The extract can be incorporated into a wet animal food or a dried (*e.g.,* pellet) animal food. In certain embodiments the extract can provides a fiber component of the diet.

### Therapeutic/Prophylactic applications of transgenic plant extracts comprising apoproteins.

It has been demonstrated that the peptides described herein (*e.g.,* the peptides listed in Tables 1, 2, and/or 3) are therapeutically and/or prophylactically effective in a number of indications characterized by an inflammatory response. Such indications include, for example atherosclerosis as described for example, in U.S. Patent Nos: 6,664,230, 6,933,279, 7,144,862, 7,166,578, 7,199,102 and PCT Publication Nos: PCT/US2001/026497 (WO 2002/015923), and PCT/US2008/085409.

Accordingly, it is believed that transgenic plant extracts as described herein comprising the peptides or portions thereof are similarly effective in such indications. Thus, in certain embodiments, methods for the treatment or prophylaxis of a pathology characterized by an inflammatory response are disclosed where the method comprises administering to a mammal in need thereof an effective amount of a transgenic plant extract comprising one or more peptides from Tables 1, 2, and/or 3, and/or a food comprising a transgenic plant extract as described herein and a food capable of being ingested for its nutritional value, and /or a protein powder wherein at least a portion of the protein powder comprises a transgenic plant extract as described herein.

In certain embodiments the transgenic plant extract comprises a peptide having an amino acid sequence selected from the group consisting of DWLKAFYDKFFEKFKEFF (6F, SEQ ID NO:1), FFEKFKEFFKDYFAKLWD (rev6F, SEQ ID NO:2), DWFKAFYDKVAEKFKEAF (4F, SEQ ID NO:3), FAEKFKEAVKDYFAKFWD (rev4F, SEQ ID NO:4), LLEQLNEQFNWVSRLANL (SEQ ID NO:5), and LVGRQLEEFL (SEQ ID NO:6).

An illustrative, but non-limiting list of indications/conditions for which the peptides described herein have been shown to be effective and/or are believed to be effective is shown in Table 5.

**Table 5. Illustrative conditions in which the peptides described herein (e.g., 4F, 6F, etc.) have been shown to be or are believed to be effective. It is believed the transgenic plant extracts described herein will be similarly effective.**

| atherosclerosis/symptoms/consequences thereof |
|---|
| plaque formation |
| lesion formation |
| myocardial infarction |
| stroke |
| congestive heart failure |

| vascular function: |
|---|
| arteriole function |
| arteriolar disease |
| associated with aging |
| associated with Alzheimer's disease |
| associated with chronic kidney disease |
| associated with hypertension |
| associated with multi-infarct dementia |
| associated with subarachnoid hemorrhage |
| peripheral vascular disease |

| pulmonary disease: |
|---|
| chronic obstructive pulmonary disease (COPD), |
| emphysema |
| asthma |
| idiopathic pulmonary fibrosis |
| pulmonary fibrosis |
| adult respiratory distress syndrome |
| osteoporosis |
| Paget's disease |
| coronary calcification |
| autoimmune: |
| rheumatoid arthritis |
| polyarteritis nodosa |
| polymyalgia rheumatica |
| lupus erythematosus |
| multiple sclerosis |
| Wegener's granulomatosis |
| central nervous system vasculitis (CNSV) |
| Sjögren's syndrome |
| Scleroderma |
| polymyositis |
| AIDS inflammatory response |

| infections: |
|---|
| bacterial |
| fungal |
| viral |
| parasitic |
| influenza (including avian flu) |
| viral pneumonia |
| endotoxic shock syndrome |
| sepsis |
| sepsis syndrome |
| (clinical syndrome where it appears that the patient is septic but no organisms are recovered from the blood) |

| trauma/wound: |
|---|
| organ transplant |
| transplant atherosclerosis |
| transplant rejection |
| corneal ulcer |
| chronic/non-healing wound |
| ulcerative colitis |
| reperfusion injury (prevent and/or treat) |
| ischemic reperfusion injury (prevent and/or treat) |
| spinal cord injuries (mitigating effects) |

| cancers |
|---|
| myeloma/multiple myeloma |
| ovarian cancer |
| breast cancer |
| colon cancer |
| bone cancer |
| cervical cancer |
| prostate cancer |
| osteoarthritis |
| inflammatory bowel disease |
| allergic rhinitis |
| cachexia |
| diabetes |
| Alzheimer's disease |
| implanted prosthesis |
| biofilm formation |
| Crohns' disease |
| renal failure (acute renal failure, chronic renal failure) |
| sickle cell disease, sickle cell crisis |
| amelioration of adriamycin toxicity |
| amelioration of anthracylin toxicity |
| to improve insulin sensitivity |
| to treat the metabolic syndrome |
| to increase adiponectin |
| to reduce abdominal fat |

| dermatitis, acute and chronic |
|---|
| eczema |
| psoriasis |
| contact dermatitis |
| scleroderma |

| diabetes and related conditions |
|---|
| Type I Diabetes |
| Type II Diabetes |
| Juvenile Onset Diabetes |
| Prevention of the onset of diabetes |
| Diabetic Nephropathy |
| Diabetic Neuropathy |
| Diabetic Retinopathy |
| erectile dysfunction |
| macular degeneration |
| multiple sclerosis |
| nephropathy |
| neuropathy |
| Parkinson's Disease |
| peripheral vascular disease |
| meningitis |

| Specific biological activities: |
|---|
| increase Heme Oxygenase 1 |
| increase extracellular superoxide dismutase |
| prevent endothelial sloughing |
| prevent the association of myeloperoxidase with ApoA-I |
| prevent the nitrosylation of tyrosine in ApoA-I |
| render HDL anti-inflammatory |
| improve vasoreactivity |
| increase the formation of pre-beta HDL |
| promote reverse cholesterol transport |
| promote reverse cholesterol transport from macrophages |
| synergize the action of statins |

It is noted that the conditions listed in Table 5 are intended to be illustrative and not limiting.

### Methods of preventing or reducing the uptake of dietary pro-inflammatory micro-lipid components.

Without being bound by a particular theory, it is believed that a major action of the transgenic plant extracts described herein is the reduction in small intestine levels of lysophosphatidic acid (LPA). This could be mediated by several mechanisms. It was previously postulated that the formation of LPA occurs by two pathways (Aoki et al. (2008) Biochimica et Biophysica Acta, 1781: 513-518)). The first involves the action of PLA₁ or PLA₂ on a phospholipid such as phosphatidylcholine to produce a lysophospholipid (*i.e.* the removal of a fatty acid from position one or two from the phospholipid). The next step is the action by a Phospholipase D such as autotaxin to remove the choline and yield the lysophosphatidic acid. The second pathway involves phosphatidic acid which is either formed from the action of phospholipase D on a phospholipid such as phosphatidylcholine generating phosphatidic acid or the action of diacyl glycerol kinase (DGK) on diacyglycerol (DAG) which results in the formation of phosphatidic acid. These processes can occur in the enterocyte or in the lumen of the small intestine or prior to ingestion of food.

Another possible mechanism for regulating LPA levels involves three enzymes known as lysophosphatidic acid phosphatase 1, 2 or 3 (LPP1, LPP2, LPP3). These phosphatases rapidly remove the phosphate from LPA and hence contribute to regulation of LPA levels.

To explore the mechanism of action, a microarray experiment to determine gene expression levels in the small intestine from mice fed the Western Diet or a chow diet and given or not given D-4F in their drinking water was performed. It was previously shown that D-4F administration reduces LPA levels similar to transgenic 6F tomatoes. In this experiment, none of the enzymes involved in the formation of LPA changed their expression significantly in the microarray. While this experiment does not completely rule out the effect of the apolipoprotein mimetics on these enzymes, without being bound to a particular theory, it is believed the transgenic plants described herein alter/reduce LPA by another mechanism.

In particular it is believed that the mechanism of action of the transgenic 6F tomato extracts described herein is that they block, or at least partially inhibit, the uptake of precursors into the enterocyte such as phosphatidic acid (PA) or they block, or at least partially inhibit, the uptake into the enterocyte of pre-formed LPA which is contained in the diet. Such a mechanism is consistent with the known action of the apoA-I mimetic peptides. In this regard, it is noted that the binding affinity of L-4F for LPA approaches the binding affinity of avidin for biotin, which is the highest binding affinity known.

Without being bound to a particular theory, it is believed that lipid components of the diet can be divided into two classes: macro-lipid components of the diet and micro-lipid components of the diet. The former in a Western diet would include phospholipids such as phosphatidylcholine and sterols such as cholesterol. These are present in milligram amounts per gram of diet. Even lysophosphatidylcholine is likely to be present in milligram quantities after phosphatidylcholine is acted upon in the Duodenum by PLA₂. The micro-lipid components are present in microgram amounts per gram of diet. As shown in previous studies the amount of intact 6F peptide found in the small intestine about two hours after the mice consumed the Western diet with transgenic 6F tomatoes was 15.6 ± 7.4 µg 6F per 200 mg small intestine. It is believed to be unlikely that this amount of peptide could bind and prevent the uptake of significant amounts of phospholipids or lysophosphatidylcholine or sterol. However, this amount of peptide could bind and prevent the uptake of microgram quantities of phosphatidic acid (PA) such as is present in foods (*see, e.g.,* Tanaka et al. ((2012) Agric. Food Chem., 60: 4156-4161) or preformed LPA which is present in hen egg yolk at 44.23nmol/g tissue or in hen egg white (8.81 nmol/g tissue) (Nakane et al. (2001) Lipids, 36: 413-419).

Accordingly, in view of the foregoing, methods of preventing or reducing the uptake of dietary pro-inflammatory micro-lipid components (e.g., lysophosphatidic acid, phosphatidic acid, and the like) are contemplated. In certain embodiments the method comprises administering to the mammal an effective amount of a transgenic plant extract having ApoA-I activity as described and/or claimed herein; and/or a food or food ingredient as described and/or claimed herein; and/or a protein powder as described and/or claimed herein; and/or a nutritional supplement as described and/or claimed herein.

We have observed that feeding a Western diet (WD) to LDLR null mice increased small intestine levels of unsaturated (but not saturated) lysophosphatidic acid (LPA). Adding unsaturated LPA to low-fat mouse chow resulted in levels of unsaturated LPA in the small intestine and plasma similar to that achieved on WD; mimicked WD in causing dyslipidemia and systemic inflammation; and produced similar levels of aortic atherosclerosis. Adding transgenic tomatoes expressing the apoA-I mimetic peptide 6F (Tg6F) to mouse chow supplemented with unsaturated LPA or adding Tg6F to WD decreased unsaturated LPA levels in the small intestine ameliorating dyslipidemia and aortic atherosclerosis.

Adding unsaturated (but not saturated) lysophophatidylcholine (LysoPC) to low-fat mouse chow produced dyslipidemia similar to adding unsaturated LPA or feeding WD. A specific oral inhibitor of autotaxin reduced LysoPC-mediated dyslipidemia. We hypothesize that LysoPC (produced by the action of Group 1B phospholipase A₂ on dietary phosphatidylcholine) is acted upon by autotaxin in the small intestine to produce LPA. Based on preliminary data, we hypothesize that intestinally-derived unsaturated LPA decreases inositol-requiring enzyme 1β (*Ire1β*) mRNA and protein levels and increases microsomal triglyceride transfer protein (*Mtp*) mRNA levels and activity in the small intestine, which results in increased secretion of lipoproteins from the small intestine.

WD caused the induction of enzymes (*Lpcat3; Scd1*) in the small intestine that promote the formation of unsaturated phospholipids. It is believed that intestine-specific knockdown of *Lpcat3* will lead to increased levels of unsaturated LPA in the small intestine in response to WD, and intestine-specific knockdown of *Scd1* will lead to decreased unsaturated LPA levels in the small intestine. Preliminary data demonstrate that the inflammatory response to intestinally-derived LPA is genetically determined.

In a preliminary experiment to identify the genetic factors controlling the response to intestinally-derived LPA using the Hybrid Mouse Diversity Panel data indicated that Tg6F prevents the WD-mediated increase in *Mtp* mRNA levels in the small intestine. Accordingly, it is believed that one mechanism by which Tg6F decreases WD-mediated dyslipidemia is by increasing *Ire1β* mRNA and protein levels and decreasing *Mtp* mRNA and activity in the small intestine leading to decreased secretion of lipoproteins from the small intestine. Based on other preliminary data showing that i) unsaturated LPA induces *Cd36* expression in the small intestine similar to WD; ii) Tg6F inhibits the WD-mediated increase in *Cd36* expression in the small intestine; and iii) enterocytes from the small intestine of mice on WD supplemented with Tg6F absorb less cholesterol, we hypothesize that Tg6F decreases cholesterol absorption by interacting with enterocyte CD36 in the proximal small intestine.

CD36 is recognized as a lipid and fatty acid receptor and plays an important role in the metabolic syndrome and associated cardiac events (*see, e.g.,* Geloen et al. (2012) Plos One, DOI: 10.1371/journal.pone.0037633). In view of the effect of Tg6F on CD36 it is believed that administration of the extract described herein is also of use in the treatment and/or prophylaxis of metabolic syndrome and/or associated cardiac events.

### EXAMPLES

The following examples are offered to illustrate, but not to limit the claimed invention.

### Example 1

### Concentration of 6F Peptide Activity in Tomato Plant Extract

Transgenic tomatoes were constructed with the vector pBI121 containing the *GUS* gene (empty vector control tomatoes; EV) or transgenic tomatoes were constructed with the vector pBI121 in which the *GUS* gene was replaced with a sequence designed to express the apoA-I mimetic peptide 6F (D-W-L-K-A-F-Y-D-K-F-F-E-K-F-K-E-F-F, Seq ID NO:1) (Tg6F) and grown, freeze-dried and ground into powder as previously described (Chattopadhyay et al. (2013) J. Lipid Res., 54: 995-10101). Ten grams of freeze-dried tomato powder from EV and ten grams of freeze-dried tomato powder from Tg6F tomatoes were thoroughly mixed an incubated for 24 hours in 250 mL of ethyl acetate (HPLC grade from Fisher Scientific, catalogue number E195-4) containing 5% glacial acetic acid (HPLC grade from Fisher Scientific, catalogue number (A35-500). The liquid phase was collected and dried under argon gas. The remaining solids were dissolved in 20 mL of distilled water and then lyophilized. The final extracts were weighed and stored at -20°C until use. The weight of the final extract from EV was 300 mg and the weight of the final Tg6F extract was 310 mg. The extracts were taken from the freezer and all of the EV extract and all of the T6F extract was added to 500 grams of powdered Western diet (WD) (Teklad, Harlan, catalogue number TD88137) in an industrial mixer and was thoroughly mixed for 30 minutes as previously described (1,2). The diets were then packaged into 16 gram portions in aluminum foil and kept at -20°C until use. Female LDL receptor null mice age 6 - 7 months were housed 4 per cage and each cage was provided with either 16 grams WD or 16 grams WD + EV or 16 grams WD + Tg6F each night. After two weeks, the mice were fasted overnight and blood was collected for determination of plasma cholesterol (Fig. 1) and triglycerides (Fig. 2), which were determined as previously described (Chattopadhyay et al. (2013) J. Lipid Res., 54: 995-10101; Navab et al. (2013) J. Lipid Res., 54: 3403-3418).

The extracts that were added to WD from EV and Tg6F were taken from 10 grams of starting freeze-dried ground tomato powder. The results shown in Figures 1 and 2 above are at least equal to what we previously published for feeding the mice WD with 2.2% freeze-dried ground Tg6F tomato powder (*Id.*)*.* However, in the experiments shown in Figures 1 and 2 the extracts only accounted for 0.06% and 0.062% of the diets by weight for EV and Tg6F, respectively. The amount of freeze-dried tomato powder needed by weight was reduced by about 97% by the concentration process. Thus, instead of having to eat three cups of freeze-dried Tg6F tomato powder three times daily to achieve a total dose of 450 mg of 6F peptide per day, a person would only need to eat ∼two tablespoons of the powdered extract three times daily to achieve the same dose. This will greatly enhance the chance of compliance for patients.

### Example 2

### Transgenic Plaant Extracts for the Treatment of Cancer.

BALB/c mice were maintained on normal mouse chow and were injected via tail venin with 2 x 10⁴ CT26 cells in 100 µL of PBS. The mice were fed chow or chow supplemented with freeze-dried powder of an extract of transgenic tomatoes expression a control marker protein (EV) or expressing the apoA-I mimetic peptide 6F (Tg6F) at 0.06% of diet by weight.

After 5 weeks, as shown in Fig. 3A, the number of metastatic tumors in the lungs was determined and as shown in Fig. 3B the plasma level of LPA 20:4 was determined. Three of the 12 mice receiving Chow alone died and 2 of 12 mice receiving Chow + EV died. None of the 12 mice receiving Chow + Tg6F died. The number of tumors in mice that died was determined, but plasma LPA levels were not. Plasma levels of LPA 20:4 correlated with the number of tumors in the lungs. p = 0.0159.

### Example 3

### Efficacy of Tomato Concentrates in Mouse Models of Dyslipidemia and Cancer

We have demonstrated that adding freeze-dried tomato powder from transgenic plants expressing the apolipoprotein A-I mimetic peptide 6F at 2.2% by weight to a Western diet (WD) ameliorated dyslipidemia and atherosclerosis in mice. It was determined that the same dose in a human would require three cups of tomato powder three times daily. To reduce the volume, we sought a method to concentrate 6F. Remarkably, extracting the transgenic freeze-dried tomato overnight in ethyl acetate with 5% acetic acid resulted in a 37-fold reduction in the amount of transgenic tomato needed for biologic activity. In a mouse model of dyslipidemia, adding 0.06% by weight of the tomato concentrate expressing the 6F peptide (Tg6F) to a Western diet significantly reduced plasma total cholesterol and triglycerides (p < 0.0065). In a mouse model of colon cancer metastatic to the lungs, adding 0.06% of Tg6F, but not a control tomato concentrate (EV), to standard mouse chow reduced tumor-associated neutrophils by 94 ± 1.1% (p = 0.0052), and reduced tumor burden by two-thirds (p = 0.0371). Adding 0.06% of either EV or Tg6F by weight to standard mouse chow significantly reduced tumor burden in a mouse model of ovarian cancer; however, Tg6F was significantly more effective (35% reduction for EV versus 53% reduction for Tg6F; p = 0.0069). Providing the same dose of tomato concentrate to humans would require only about two tablespoons three times daily making this a practical approach for testing oral apoA-I mimetic therapy in the treatment of dyslipidemia and cancer.

### Introduction to Example 3

Recent studies suggest that apolipoprotein (apo) A-I and apoA-I mimetic peptides may have a role in the treatment of a number of malignancies. HDL-associated proteins apoA-I, transthyretin and transferrin have been identified as biomarkers of ovarian cancer (Kozak et al. 2003, 2005; Nossov et al. 2008, 2009). In a mouse model of ovarian cancer in which the mice have a normal immune system, it was found that transgenic expression of human apoA-1 significantly decreased tumor burden and extended survival of the mice (Su et al. 2010). Subsequently, Hazen and colleagues (Zamanian-Daryoush et al. 2013) reported that apoA-I potently suppressed tumor growth and metastasis in multiple animal tumor models through both innate and adaptive immune processes.

Su et al. (2010) demonstrated that the apoA-I mimetic peptides 4F and 5F administered either orally or by injection decreased tumor growth, and decreased levels of a known tumor growth promoter, lysophosphatidic acid (LPA). Mechanisms deduced to play a role in the efficacy of apoA-I mimetic peptides included decreased tumor angiogenesis (Gao et al. 2011).

In other studies, it was found that the 4F peptide upregulated the antioxidant enzyme manganese superoxide dismutase (MnSOD) and inhibited proliferation and the ability to form tumors of epithelial ovarian cancer cells (Ganapathy et al. 2012).

The risk for endometrial (Cust et al. 2007) and colon cancer (van Duijnhoven et al. 2011) is inversely correlated with HDL-cholesterol levels. Adding the 4F peptide to mouse chow decreased tumor burden, tumor angiogenesis, and plasma LPA levels in a mouse model of colon cancer (Su et al. 2012). In a mouse model of familial adenomatous polyposis, administering 4F in mouse chow also reduced the number and size of tumors in the intestinal tract and decreased LPA levels in C57BL/6J-Apc^{Min/+} mice (Su et al. 2012).

Hypoxia-inducible factor-1α (HIF-1α) was induced in vitro by LPA in human ovarian cancer cell lines, and was decreased by addition of the 4F peptide (Gao et al. 2012). Moreover, administration of the 4F peptide markedly decreased the expression of HIF-1α in mouse ovarian tumor tissues (Gao et al. 2012).

Inflammation (Mantovani et al. 2008) and tumor-associated macrophages play an important role in the progression and metastasis of cancers (Zamanian-Daryoush et al. 2013; Qian et al. 2010). LPA has been reported to increase the expression of scavenger receptor A (SR-A) on macrophages (Chang et al. 2008). SR-A expression on macrophages has been shown to be necessary and sufficient to promote tumor invasiveness (Neyen et al. 2013a). The 4F peptide was reported to be a potent inhibitor of SR-A (Neyen et al. 2009); administration of the 4F peptide inhibited tumor invasiveness (Neyen et al. 2013a, 2013b). Thus, there is evidence in animal models that apoA-I and apoA-I mimetic peptides may be potential therapeutic agents for the amelioration of cancer.

We recently reported a novel means of administering apoA-I mimetic peptides in mouse models of atherosclerosis (Chattopadhyay et al. 2013; Navab et al. 2013, 2015). We showed that the apoA-I mimetic peptide 6F could be expressed in transgenic tomato plants. When the tomatoes were freeze-dried and fed to LDLR null mice in a Western diet (WD), they ameliorated dyslipidemia and atherosclerosis (Chattopadhyay et al. 2013).

The transgenic tomatoes expressing the 6F peptide (Tg6F) also ameliorated dyslipidemia and atherosclerosis induced by adding unsaturated lysophosphatidic acid (LPA) to standard mouse chow (Navab et al. 2015). In the mouse studies (Chattopadhyay et al. 2013; Navab et al. 2013, 2015) the freeze-dried, ground tomato powder was added to mouse diets at 2.2% by weight. Laboratory mice eat a single diet making it easy to mix in the freeze-dried tomato powder. In contrast, human diets are much more complicated, and it would be a challenge to use freeze-dried tomato powder as a dietary supplement because of the amount of powder required to achieve the same doses of the peptide as were achieved in mice; 3 cups of powder three times per day would be required. It was felt that this volume would be impractical for widespread use. Therefore, we sought a simple and economical method to concentrate the 6F peptide from freeze-dried tomatoes in order to decrease the volume required to achieve therapeutic doses. We now report that concentrates of transgenic tomatoes expressing the 6F peptide can easily be prepared, such that the required doses could be administered to humans using no more than two tablespoons of concentrate three times daily. We also present evidence that these concentrates are effective in mouse models of dyslipidemia, and in mouse models of cancer.

### Materials and Methods

### Chemical Reagents

Ethanol (catalog # BP2818-100), ethyl acetate (HPLC grade; catalog # E195-4), and glacial acetic acid (HPLC grade; catalog # A35-500) were purchased from Fisher Scientific.

### Tumor Cells

The ID8 cell line (a mouse ovarian epithelial papillary serous adenocarcinoma cell line) was a generous gift from K. F. Roby (Center for Reproductive Sciences, University of Kansas Medical Center, Kansas City, KS). The CT26 cell line derived from N-nitroso-N-methyl urethane-induced mouse colon carcinoma of BALB/c origin was purchased from the American Type Culture Collection (ATCC).

Mouse apoA-I ELISA kit was from USCN Life Science, Inc. Wuhan, China (catalogue # SEA519Mu). All other materials were from sources described previously (Chattopadhyay et al. 2013; Navab et al. 2013, 2015) or as specifically stated below.

### Mice

Female wild-type C57BL/6J and BALB/c mice were purchased from Jackson Laboratory and maintained on standard mouse chow (Ralston Purina). LDLR null mice on a C57BL/6J background were purchased from Jackson Laboratory and bred in the Division of Laboratory and Animal Medicine at UCLA. All LDLR null mice were female. All mice were maintained on standard mouse chow (Ralston Purina). The LDLR null mice were switched to a Western diet (WD) (Teklad, Harlan, catalog # TD88137) for the period stated in the figure legend. The ages of the mice are stated in the figure legends. All mouse experiments were approved by the Animal Research Committee at UCLA.

### Preparation of Tomato Concentrates

Transgenic tomato plants expressing the marker protein β-glucuronidase (EV), or expressing the 6F peptide (Tg6F) were constructed and grown at the Donald Danforth Plant Science Center in St. Louis, MO as previously described (Chattopadhyay et al. 2013). The tomatoes were rendered free of seeds, rapidly frozen, and shipped frozen by overnight courier to UCLA where they were freeze-dried (VirTis lyophilizer, Gardner, NY) to obtain dried tomato fruit tissue (pulp plus skin), which was stored at -80°C as previously described (Chattopadhyay et al. 2013).

The freeze-dried EV and Tg6F tomato fruit tissue was thoroughly mixed in either aqueous ethanol (ethanol/water, 60/40; v/v), or ethyl acetate containing 5% glacial acetic acid at a ratio of 1/25, w/v (gm/mL), and extracted for 24 hours at room temperature. After 24 hours the liquid phase was clearly separated from the solid phase in both the ethanol/water and ethyl acetate/acetic acid extractions. The liquid phase was collected and the ethanol/water extract was dried under vacuum; the ethyl acetate/acetic acid extract was dried under a stream of argon. The remaining solids were re-dissolved in distilled water to 10% of the original volume and then lyophilized. The final concentrates were weighed and stored at -20°C until use.

### Analysis of Tomato Concentrates

100 or 200 µg of tomato concentrate protein, or 5 - 20 µg of authentic 6F peptide produced by solid phase synthesis as previously described (Chattopadhyay et al. 2013) was added to lanes of 18% SDS PAGE gels that were run overnight. The gels were fixed in water/methanol /acetic acid (50/50/7, v/v/v, 30 min. at room temperature), stained overnight with Sypro Ruby (s12000 kit, Life Technologies, NY; Invitrogen), and de-stained with water/methanol/acetic acid (9/10/7, v/v/v, 30 min. at room temperature). Densitometry images of the de-stained gels were recorded using image J software as previously described (Chattopadhyay et al. 2013). To confirm the identity of 6F, bands of interest were manually excised and treated with trypsin (10 µL, 100 ng/mL 50 mM ammonium bicarbonate, Trypsin Gold, V5280, Promega) overnight, at 37°C. The treated gel slices were eluted with water/acetonitrile/TFA (1mL, 50/50/0.1, v/v/v) and the eluates were dried in a vacuum centrifuge and re-dissolved in 10 µL of water/acetonitrile/TFA and injected onto a reverse phase LC column (Agilent PLRP-S, 5 micron particle size, 300 Å pore diameter, 150 x 2 mm) equilibrated in 95% Solvent A (water/formic acid, 100/0.1, v/v) and 5% Solvent B (acetonitrile/formic acid, 100/0.1, v/v) and eluted at constant flow (40°C, 200 µL/min) with an increasing proportion of Solvent B (%B/min: 0/5, 5/5, 45/100, 50/5, 60/5). The effluent from the column was directed to an electrospray ion source connected to a quadrupole ion trap mass spectrometer (Thermo LCQ Deca XP Plus) scanning in the positive ion mode (m/z 50-2000, 3 microscans were averaged, 50 millisecond maximum inject time). Data were processed in Thermo Xcalibre™ software.

### Preparation of Diets

The tomato concentrates were taken from the freezer and added to standard mouse chow or to WD in an industrial mixer and thoroughly mixed for 30 min. as previously described (Chattopadhyay et al. 2013; Navab et al. 2013, 2015) to yield a final diet containing 0.015%, 0.03% or 0.06% by weight of each tomato concentrate. In some experiments, the starting material (i.e. the freeze-dried transgenic tomatoes from which the concentrates were made) was added to standard mouse chow at 2.2%, or 1.1% or 0.55% by weight as described previously (Chattopadhyay et al. 2013; Navab et al. 2013, 2015) and used as controls. The diets were packaged into 16 gram portions in aluminum foil and kept at -80°C until use. Addition of 0.06% by weight provided the mice with a daily dose of ∼120 mg/kg/day per mouse of tomato concentrate, which provided ∼7 mg/kg/day per mouse of the 6F peptide. In the cancer studies administration of the tomato concentrates began the day after the cancer cells were injected.

### Metastatic Colon Cancer Studies

Female BALB/c mice 6 weeks of age were administered 2 x 10⁴ CT26 cells in 100 µL of PBS via tail vein injection as described previously (Su et al. 2012). After injection the mice were maintained on either standard mouse chow or standard mouse chow containing 0.06% by weight of EV, or 0.06% by weight of Tg6F. After 4 weeks the mice were subjected to a terminal bleed, and after sacrifice the lungs were harvested, weighed and fixed with Bouin's solution (Sigma), and the number of tumor nodules on the surface of the lungs was determined as previously described (Su et al. 2012). For determination of tumor-associated neutrophils, sections were prepared of randomly selected tumors from the lungs of three mice in each treatment group. The slides were placed in xylene to remove paraffin and then processed through a series of ethanol rinses. After a wash in tap water, the slides were incubated in 3% hydrogen peroxide/methanol solution for 10 min. After a wash in distilled water, the slides were incubated for 2 minutes in EDTA solution pH 8.0 (Invitrogen, catalog number 005501) at 95°C using a pressure cooker. The slides were brought to room temperature, rinsed in phosphate buffered saline containing Tween-20 (PBST), and then incubated at room temperature for 1 hour with anti-Ly6G antibody (BD Pharmingen, catalog number 551459) at a dilution of 1:1500. The slides were rinsed with PBST and then incubated with polyclonal rabbit anti-rat immunoglobulins/HRP (Dako, catalog number P0450) at a dilution of 1:200 at room temperature for 30 min. The slides were rinsed with PBST, and incubated with Dako EnVision+ System-HRP labeled polymer anti-rabbit antibody (Dako, catalog number K4003) at room temperature for 30 min. After a rinse with PBST, the slides were incubated with 3,3'-Diaminobenzidine (DAB) for visualization. Subsequently, the slides were washed in tap water, counterstained with Harris' Hematoxylin, dehydrated in ethanol, and mounted with media. Controls consisted of sections exposed to secondary-only antibodies. Photomicrographs were captured using an Olympus BX51 microscope and the application Q Capture 7.0 (Q Imaging Inc.). Randomly selected fields were quantified for each sample and the ratio of the stain signal to the tumor surface area was determined using Image Pro Plus 7 (Media Cybernetics).

### Ovarian Cancer Studies

Female C57BL/6J mice 9 weeks of age were given an intraperitoneal injection of 8 x 10⁶ ID8 cells in a total volume of 0.8 mL of Dulbecco's Modified Eagle Medium (DMEM without supplements). Following injection, the mice were maintained on standard mouse chow or standard mouse chow containing 0.06% by weight of EV, or 0.06% by weight of Tg6F. After 12 weeks the mice were subjected to a terminal bleed, and after sacrifice the number of tumor nodules on the peritoneal surfaces and on the surface of abdominal organs was determined as previously described (Su et al. 2010).

### Plasma assays

Plasma total cholesterol, triglycerides, HDL-cholesterol, paraoxonase-1 (PON) activity, serum amyloid A (SAA), and apoA-I were assayed using kits and procedures outlined by the kit manufacturers as previously described (Chattopadhyay et al. 2013; Navab et al. 2013, 2015). Species of lysophosphatidic acid (LPA) were determined by LC-MS/MS/MRM as previously described (Chattopadhyay et al. 2013; Navab et al. 2013, 2015), and Fast Performance Liquid Chromatography (FPLC) was performed as previously described (Chattopadhyay et al. 2013; Navab et al. 2013).

### Statistical Methods

Statistical analyses were performed by ANOVA and by unpaired two-tailed t- test, or by linear regression using GraphPad Prism version 5.03 (GraphPad Software, San Diego, CA). Statistical significance was considered achieved if p<0.05.

### Results

### Ethyl acetate with 5% acetic acid (but not 60% ethanol) efficiently concentrates the 6F peptide produced in transgenic tomatoes

Twenty-three micrograms of 6F were found in 100 µg of tomato concentrate protein prepared with ethyl acetate with 5% acetic acid (Fig. 4A). In contrast, concentrates prepared with 60% ethanol did not contain detectable amounts of 6F (Fig. 4B). The presence of the 6F peptide was confirmed in the Tg6F concentrate (but not in the EV concentrate) by reverse phase LC and mass spectrometry (Fig. 4C). Based on these results, all subsequent experiments utilized tomato concentrates prepared with ethyl acetate with 5% acetic acid.

### Dose response of tomato concentrates

To determine the dose response of the tomato concentrates, we used a mouse model of dyslipidemia as shown in Figs. 5A-5H. The data in these figures demonstrate that the tomato concentrates were at least as effective in improving plasma lipids as was the starting material (i.e. the freeze-dried transgenic tomatoes from which the concentrates were made). Based on these results we used a dose of 0.06% by weight of the tomato concentrates in all subsequent experiments.

### Administration of tomato concentrate containing the 6F peptide significantly reduced metastasis of colon cancer cells to the lungs

The weight of the lungs after intravenous administration of the colon cancer cells was significantly less after administration of Tg6F compared to mice not receiving any tomato concentrate, or compared to mice receiving EV (Fig. 6A). The number of tumor nodules on the surface of the lungs was less after administration of EV, but this did not reach statistical significance (Fig. 6B). In contrast, there was a significant and remarkable two-thirds reduction in the number of tumor nodules on the surface of the lungs after administration of Tg6F (Fig. 6B). Consistent with our previous report on the efficacy of the 4F peptide in this model (Su et al. 2012), the plasma levels of LPA 20:4 were significantly reduced with administration of Tg6F, but plasma LPA 20:4 levels were not decreased with administration of EV (Fig. 6C). The plasma levels of other LPA species including LPA 18:2, LPA 18:1 and LPA 18:0 were not significantly reduced (data not shown).

As early as 1995 evidence was accumulating that granulocytes could promote tumor growth (Pekarek et al. 1995). Tumor growth in the lungs was later shown to be promoted by neutrophil elastase (Houghton et al. 2010). Therefore, we determined the content of tumor-associated neutrophils in this mouse model of colon cancer cells metastasizing to the lungs. A representative photomicrograph of the tumor-associated neutrophils in each of the treatment groups is shown in Fig. 7A and the data are shown in Fig. 7B. Remarkably, addition of 0.06% by weight of Tg6F to the standard mouse chow reduced tumor associated neutrophils by 94 ± 1.1% (p = 0.0052).

### Administration of tomato concentrate containing the 6F peptide was significantly more effective than the control tomato concentrate in reducing ovarian cancer cell tumor burden

In a mouse model of ovarian cancer in which mouse ovarian cancer cells were injected into the peritoneum of female mice with normal immune function, the total number of tumor nodules in the abdomen was significantly reduced by approximately 35% in mice receiving EV (p = 0.0013) compared to mice receiving standard mouse chow alone (Fig. 8A). In the mice receiving Tg6F, the total number of tumor nodules in the abdomen was reduced by approximately 53% (p <0.0001) compared to mice receiving standard mouse chow alone (Fig. 8A). The difference between the total number of tumor nodules in the abdomen of mice receiving EV compared to those receiving Tg6F was highly significant (p = 0.0069) (Fig. 8A).

The number of tumor nodules on the surface of the peritoneal cavity significantly decreased by approximately 35% in mice receiving EV (p = 0.0021) compared to standard mouse chow alone (Fig. 8B). The decrease in the mice receiving Tg6F was approximately 55% (p <0.0001) compared to standard mouse chow alone (Fig. 8B). The difference in the number of tumor nodules on the surface of the peritoneal cavity in mice receiving EV compared to mice receiving Tg6F was highly significant (p = 0.0048) (Fig. 8B).

The number of tumor nodules on the surface of the diaphragm was significantly reduced by approximately 35% in mice receiving EV (p = 0.0127) compared to standard mouse chow alone (Fig. 8C). The number of tumor nodules on the surface of the diaphragm was significantly reduced in mice receiving Tg6F by approximately 45% (p = 0.0016) compared to standard mouse chow alone (Fig. 8C). The difference between the mice receiving EV and Tg6F was not significant (Fig. 8C).

The number of tumor nodules on the surface of the intestine was not significantly changed in mice receiving EV compared to standard mouse chow alone, but there was a significant decrease in the mice receiving Tg6F (p = 0.0134) compared to mice receiving standard mouse chow alone (Fig. 8D). The difference between the number of tumor nodules on the surface of the intestine in mice receiving EV compared to the mice receiving Tg6F was highly significant (p = 0.0035) (Fig. 8D).

The number of tumor nodules in the abdomen that measured >1 mm in size was not significantly decreased by EV compared to mice receiving standard mouse chow alone, but there was a significant 52% decrease in the number of tumor nodules in the abdomen measuring greater than 1 mm in size in the mice receiving Tg6F (p = 0.0267) compared to mice receiving standard mouse chow alone (Fig. 8E). The difference between the mice receiving EV compared to the mice receiving Tg6F was also significant (p = 0.0267) (Fig. 8E).

In contrast to the case in the metastatic colon cancer model (Fig. 6C), and in contrast to our previous studies with the 4F peptide (Su et al. 2010, 2012) there was no significant change in the levels of plasma LPA species (data not shown). Similarly, there was no significant difference in total plasma cholesterol levels in mice receiving EV compared to Tg6F (Fig. 9A), and plasma total cholesterol levels did not correlate with the total number of tumor nodules (Fig. 9B). However, plasma triglyceride levels were significantly reduced in mice receiving either tomato concentrate compared to mice receiving standard mouse chow alone, and there was no significant difference in plasma triglyceride levels in mice receiving EV compared to Tg6F (Fig. 9C). The total number of tumor nodules was significantly and positively correlated with plasma triglyceride levels (r² = 0.1375; p = 0.0022) (Fig. 9D). Plasma HDL-cholesterol levels were significantly higher in mice receiving either tomato concentrate compared to mice receiving standard mouse chow alone, and there was no significant difference in the HDL-cholesterol levels in mice receiving EV compared to Tg6F (Fig. 9E). HDL-cholesterol levels were not significantly correlated with the total number of tumor nodules (Fig. 9F). Plasma apoA-I levels were significantly higher in mice receiving either tomato concentrate compared to mice receiving chow alone, and there was no significant difference in the apoA-I levels in mice receiving EV compared to Tg6F (Fig. 9G). Consistent with our previous data (Su et al. 2010) and that of Hazen and colleagues (Zamanian-Daryoush et al. 2013), plasma apoA-I levels were inversely and significantly correlated with the total number of tumor nodules (r² = 0.1096; p = 0.0062) (Fig. 9H). Plasma serum amyloid A (SAA) levels were significantly lower in mice receiving either tomato concentrate compared to mice receiving chow alone, and there was no significant difference in SAA levels in mice receiving EV compared to Tg6F (Fig. 9I). Plasma SAA levels were weakly but significantly correlated with the total number of tumor nodules (r² = 0.06950; p = 0.0325) (Fig. 9J).

### Discussion

The results reported here establish a novel way to concentrate apoA-I mimetic peptides produced in transgenic plants. Tomatoes are known to be rich in antioxidant polyphenols (Minoggio et al. 2003). Both ethanol and ethyl acetate concentrate polyphenols, with ethanol being superior at high concentration (Gadkari et al. 2014), however, the 6F peptide was found in abundance in ethyl acetate, but not in aqueous ethanol concentrates (Fig.1). The ability to concentrate the 6F peptide in ethyl acetate was enhanced by the addition of small amounts of acetic acid and the maximum effect was seen at 5% acetic acid (data not shown). The extraction efficiency of this solvent is likely due to the high content of phenylalanine (6 of the 18 amino acid residues are phenylalanine) and 8 out of 10 hydrophobic residues are aromatic in 6F. The extraction efficiency resulted in 23 µg of 6F peptide being found in the band from lanes in which 100 µg of Tg6 protein were loaded on to the gels (Fig. 1A). The explanation as to why seven of nine predicted 6F fragments (//web.expasy.org/peptide_mass/; maximum 1 missed cleavage) were detected in the trypsin digests from Tg6F, but not from the 6F standard where six of the predicted fragments in the trypsin digests were detected, likely was due to the fact that 23 µg of 6F were subjected to trypsin treatment followed by reverse phase LC and mass spectrometry in the case of Tg6F, while in the case of the 6F standard we chose to analyze the 5 µg band (Figs. 1A and 1C).

The concentration process resulted in a 37-fold reduction in the weight of transgenic tomato powder required for biological activity (Fig. 5). Perhaps more importantly, the volume of transgenic freeze-dried tomato powder required to deliver doses to humans similar to those used in the mouse studies would be reduced from 3.0 cups of freeze-dried tomato powder three times daily to two tablespoons three times daily. This dramatic reduction in the volume required makes this approach much more likely to be acceptable as a treatment modality.

We previously reported that the 6F peptide content of the freeze-dried transgenic tomatoes expressing 6F accounted for 0.6% to 1.0% of the weight of the freeze-dried tomato powder (Chattopadhyay et al. 2013). Thus, 1 mg of the freeze-dried tomato powder would contain 0.006 to 0.01 mg of 6F peptide. The data in Fig. 4 indicate that the 6F peptide in the tomato concentrate from the transgenic tomatoes expressing the 6F peptide constituted 23% of the weight of the proteins in the concentrate. The protein content of the tomato concentrates was ∼25% (data not shown). Thus, 1 mg of the tomato concentrate would contain 0.0575 mg of 6F. This represents approximately a 6 - 10-fold concentration of the 6F peptide compared to the starting freeze-dried tomato powder. However, there was a 37-fold reduction in the weight of the Tg6F concentrate required to achieve the same biologic activity as was achieved with the starting Tg6F freeze-dried tomato powder (Figs. 5E-5H). The explanation for the disproportionate gain in biologic activity might be due to the concomitant concentration of tomato anti-oxidants and/or a change in the conformation of the 6F peptide in the concentrate that favors interaction with receptors compared to the freeze-dried starting material.

It is interesting that ingestion of a tea rich in catechins for 12 weeks led to a significant reduction in body fat that was correlated with a change in the concentrations of malondialdehyde-modified LDL in humans (Nagao et al. 2005). Both ethanol and ethyl acetate efficiently concentrate catechins (Gadkari et al. 2014). Ethyl acetate is naturally found in some fruits and vegetables, and is used commercially to decaffeinate coffee and tea (Ramalakshmi et al. 1999) suggesting that its use to prepare tomato concentrates is likely to be safe for humans.

It has been reported that dietary lycopene and tomato extract supplementations inhibited nonalcoholic steatohepatitis-promoted hepatocarcinogenesis in rodents by a process that appears to involve reduced oxidative stress (Wang et al. 2009). Ethyl acetate extracts of tea that were rich in polyphenols and antioxidants were shown to reduce LDL and triglyceride levels in rats and induced human hepatoma cell growth arrest by inducing p53 expression and upregulating p21 expression (Way et al. 2009). Thus, it seems likely that some of the benefits seen in our studies are due to naturally-occurring tomato antioxidants that have been extracted into the concentrates.

The reduction in LPA 20:4 levels in the metastatic colon cancer model by Tg6F, but not by EV (Fig. 6C) is consistent with LPA playing a role in this model. However, the mechanism of action of Tg6F in reducing tumor burden in the ovarian cancer model must be more complicated since Tg6F did not reduce LPA levels. These results are in contrast to the case for 4F (Su et al. 2010, 2012). Despite the lack of superiority compared to EV in altering plasma lipids, apoA-I and SAA levels in the mouse model of ovarian cancer, Tg6F was clearly superior in reducing tumor burden in the mouse model of ovarian cancer compared to EV (Figs. 8A-8E). Thus, the mechanism(s) of action of Tg6F in reducing tumor burden in the mouse model of ovarian cancer remains unknown at this time.

In considering mechanisms which account for the superiority of Tg6F compared to EV, it should be remembered that 2 hours after feeding Tg6F to mice we found intact 6F peptide in the lumen of the small intestine, but not in the plasma (Chattopadhyay et al. 2013). Subsequently, another group using a completely different apoA-I mimetic peptide (Zhao et al. 2014) found that oral administration of their apoA-I mimetic peptide was as effective as when the same dose was administered by injection. However, when the peptide was administered by injection high plasma peptide levels were found, and when administered orally, the peptide was not detected in the plasma (Zhao et al. 2014). Thus, our work and that of Zhao et al. (2014) is consistent with a primary mechanism of action for these apoA-I mimetic peptides in the intestine; not in the plasma.

What mechanisms might be involved? In the case of the mouse model of metastatic colon cancer, and in the mouse model of ovarian cancer, the treatments began the day after the tumor cells were injected, therefore it seems unlikely that they prevented tumor uptake. Without being bound to a particular theory, it is believed that the most likely mechanism of action for Tg6F in reducing tumor burden is by modulating the immune cells that interact with the tumor as shown in Figs. 7A-7C.

There are at least two receptors known to be important in the small intestine that have been shown to interact with apoA-I mimetic peptides resulting in modulation of the biologic activity of these receptors; CD36 (Baranov et al. 2008) and class B scavenger receptor types I and II (Leelahavanichkul et al. 2012). CD36 is known to be important for fatty acid and cholesterol uptake by the proximal, but not the distal small intestine (Nassir et al. 2007). CD36 is also known to be important in modulating the interaction of gut bacteria with the small intestine (Barnaova et al. 2008). Thus, it is possible that Tg6F interacts with CD36 in the proximal small intestine resulting in an alteration in the immune cells in the lamina propria of the intestinal villi. The intestine contains the largest number of immune cells in the body (Mowat and Agace 2014). It has been proposed that there may be different types of neutrophils similar to the case for pro-tumorigenic and anti-tumorigenic macrophages (Fridlender and Albelda 2012). Without being bound to a particular theory, the striking results seen in Figs. 7A-7B with regard to tumor-associated neutrophils may be due to a change in myeloid differentiation induced by interaction of Tg6F with CD36 in the proximal small intestine.

Based on the data presented here and our previously published data, regardless of the mechanisms involved, the use of Tg6F for cancer prevention or treatment appears promising. The oral route of administration is convenient, the preparation of a tomato concentrate using the simple methods described here is likely to be economical, and a tomato concentrate containing an 18 amino acid residue that appears to be degraded in the process of digestion (i.e. intact peptide was found in the small intestine 2 hours after feeding, but the peptide was never detected in plasma) (Chattopadhyay et al. 2013) is likely to be safe.

In summary, these studies i) provide a simple and economical means of dramatically concentrating an apoA-I mimetic peptide produced in transgenic tomatoes that makes testing oral apoA-I therapy in humans a feasible strategy; and ii) they provide further evidence that oral apoA-I mimetic peptides are a novel means of reducing tumor burden in mouse models, but by mechanisms that are likely to be more complicated than previously thought.

### References for Example 3

Baranova et al. (2008). Role of CD36 in bacterial recognition, phagocytosis, and pathogen-induced JNK-mediated signaling. J Immunol. 181: 7147-7156.
Chang et al. (2008) Lysophosphatidic acid-induced oxidized low-density lipoprotein uptake is class A scavenger receptor-dependent in macrophages, Prostaglandins Other Lipid Mediat 87: 20-25.
Chattopadhyay et al. (2013) A novel approach to oral apoA-I mimetic therapy, J. Lipid Res 54: 995-1010.
Cust et al. (2007) Metabolic syndrome, plasma lipid, lipoprotein and glucose levels, and endometrial cancer risk in the European Prospective Investigation into Cancer and Nutrition (EPIC). Endocr. Relat. Cancer 14: 755-767.
Fridlender and Albelda (2012) Tumor-associated neutrophils: friend or foe? Carcinogenesis. 33: 949-955.
Gadkari PV, Kadimi US, and Balaraman M. (2014) Catechin concentrates of garden tea leaves (Camellia sinensis L.): extraction/isolation and evaluation of chemical composition. J Sci Food Agric 94: 2921-2928.
Ganapathy et al. (2012) D-4F, an apoA-I mimetic peptide, inhibits proliferation and tumorigenicity of epithelial ovarian cancer cells by upregulating the antioxidant enzyme MnSOD. Int J Cancer 130: 1071-1081.
Gao et al. (2011) L-5F, an apolipoprotein A-I mimetic, inhibits tumor angiogenesis by suppressing VEGF/basic FGF signaling pathways. Integr Biol 3: 479-489.
Gao et al. (2012) Apolipoprotein A-I mimetic peptides inhibit expression and activity of Hypoxia-inducible factor-1α in human ovarian cancer cell lines and a mouse ovarian cancer model. J. Pharmacol. Exp. Ther 342: 255-262.
Houghton et al. (2010). Neutrophil elastase-mediated degradation of IRS-1 accelerates lung tumor growth. Nat Med. 16: 219-223.
Kozak et al. (2003) Identification of biomarkers for ovarian cancer using strong anion-exchange ProteinChips: potential use in diagnosis and prognosis. Proc Natl Acad Sci. USA 100: 12343-12348.
Kozak et al. (2005) Characterization of serum biomarkers for detection of early stage ovarian cancer. Proteomics 5: 4589-4596.
Leelahavanichkul et al. (2012). Class B scavenger receptor types I and II and CD36 targeting improves sepsis survival and acute outcomes in mice. J Immunol. 188: 2749-2758.
Mantovani et al. (2008) Cancer-related inflammation, Nature 454: 436-444.
Minoggio et al. (2003) Polyphenol pattern and antioxidant activity of different tomato lines and cultivars. Ann Nutr Metab 47: 64-69.
Mowat and Agace (2014). Regional specialization within the intestinal immune system. Nat Rev Immunol. 14: 667-685.
Nagao et al. (2005) Ingestion of a tea rich in catechins leads to a reduction in body fat and malondialdehyde-modified LDL in men. Am J Clin Nutr 81: 122-129.
Nassir et al. (2007). CD36 is important for fatty acid and cholesterol uptake by the proximal but not the distal intestine. J Biol Chem. 282: 19493-19501.
Navab et al. (2013) Transgenic 6F tomatoes act on the small intestine to prevent systemic inflammation and dyslipidemia caused by Western diet and intestinally derived lysophosphatidic acid, J. Lipid Res 54: 3403-3418.
Navab et al. (2015) Source and role of intestinally-derived lysophosphatidic acid in dyslipidemia and atherosclerosis. J. Lipid Res 56: [Epub ahead of print].
Neyen et al. (2009) Macrophage scavenger receptor A mediates adhesion to apolipoproteins A-I and E, Biochemistry 48: 11858-11871.
Neyen et al. (2013a) Macrophage scavenger receptor A promotes tumor progression in murine models of ovarian and pancreatic cancer, J. Immunol 190: 3798-3805.
Neyen et al. (2013b) An apolipoprotein A-I mimetic targets scavenger receptor A on tumor-associated macrophages. A prospective treatment? Oncoimmunology 2: e24461.
Nossov et al. (2008) The early detection of ovarian cancer from traditional methods to proteomics. Can we really do better than serum CA-125? Am J. Obstet Gynecol. 199: 215-223.
Nossov et al. (2009) Validation of serum biomarkers for detection of early-stage ovarian cancer. Am J. Obstet Gynecol 200: 639.e1-639.e5.
Pekarek et al. (1995). Inhibition of tumor growth by elimination of granulocytes. J Exp Med. 181: 435-440.
Qian and Pollard (2010) Macrophage diversity enhances tumor progression and metastasis, Cell 141: 39-51.
Ramalakshmi and Raghavan (1999) Caffeine in coffee: Its removal. Why and how? Critical Reviews in Food Science and Nutrition. 39: 441-456.
Su et al. (2010) Apolipoprotein A-I (apoA-I) and apoA-I mimetic peptides inhibit tumor development in a mouse model of ovarian cancer. PNAS 107: 19997-20002.
Su et al. (2012) HDL mimetics inhibit tumor development in both induced and spontaneous mouse models of colon cancer, Mol. Cancer Ther 11: 1311-1319.
van Duijnhoven et al. (2011) Blood lipid and lipoprotein concentrations and colorectal cancer risk in the European Prospective Investigation into Cancer and Nutrition. Gut 60: 1094-1102.
Wang et al. (2009) Dietary lycopene and tomato extract supplementations inhibit nonalcoholic steatohepatitis-promoted hepatocarcinogenesis in rats. Int J Cancer. 126: 1788-1796.
Way et al. (2009) Pu-erh tea attenuates hyperlipogenesis and induces hepatoma cells growth arrest through activating AMP-activated protein kinase (AMPK) in human HepG2 cells. J Agric Food Chem 57: 5257-5264.
Zhao et al. (2014) In vivo efficacy of HDL-like nanolipid particles containing multivalent peptide mimetics of apolipoprotein A-I. J Lipid Res. 55: 2053-2063.
Zamanian-Daryoush et al. (2013) The cardioprotective protein apolipoprotein A1 promotes potent anti-tumorigenic effects. J Biol Chem 288: 21237-21252.

## Claims

1. A method of concentrating transgenic apoA-I mimetic peptides expressed in a transgenic tomato plant, said method comprising:
providing a tissue of said transgenic plant wherein said tissue contains a heterologous ApoA-I mimetic peptide expressed by said plant, and wherein said tissue is provided as a substantially dry powder;
mixing said powder with a solution comprising ethyl acetate and acetic acid to form an extraction mixture;
collecting the liquid phase of said mixture and drying said liquid phase to provide a concentrated dry powder extract that displays the biological activity of said apoA-I mimetic.

2. The method of claim 1, wherein said solution comprises about 1% to about 25% acetic acid, or about 2% to about 20% acetic acid, or about 3% to about 15 % acetic acid, or about 4% to about 10 % acetic acid, or about 4% to about 8% acetic acid, or about 4% to about 6% acetic acid.

3. The method according to claim 1 or claim 2, wherein said mixing comprises mixing said powder with a solution comprising ethyl acetate and acetic acid, optionally wherein said solution comprises about 5% acetic acid.

4. The method according to any one of claims 1-3, wherein said providing tissue comprises providing said tissue as a freeze-dried powder.

5. The method according to any one of claims 1-4, wherein said mixing said powder with a solution of ethyl acetate and acetic acid comprises incubating said extraction mixture for at least 15 minutes, or at least 1/2 hour, or at least 1 hour, or at least 2 hours, or at least 3 hours, or at least 4 hours, or at least 5 hours, or at least 6 hours, or at least 7 hours, or at least 12 hours, or at least 18 hours, or at least 24 hours, or at least 48 hours, or at least 72 hours, or at least 96 hours.

6. The method according to any one of claims 1-5, wherein said collecting the liquid phase and drying said liquid phase comprises drying the liquid phase to produce said dry powder extract.

7. The method according to any one of claims 1-5, wherein said collecting the liquid phase and drying said liquid phase comprises:
drying the liquid phase to produce a dry residue;
resuspending said residue in water to provide a resuspended mixture; and
drying the resuspended mixture to provide said dry powder extract.

8. The method of claim 7, wherein said drying the resuspended mixture comprises lyophilizing said mixture to provide said dry powder extract.

9. The method according to any one of claims 1-8, wherein said heterologous ApoA-I mimetic peptide expressed by said plant comprises the amino acid sequence DWLKAFYDKFFEKFKEFF (6F) (SEQ ID NO:1), or FFEKFKEFFKDYFAKLWD (rev6F) (SEQ ID NO:2).

10. The method according to any one of claims 1-9, wherein said dry powder extract is effective to decrease plasma levels of lyophosphatidic acid (LPA) in a mammal, and/or to decrease SAA levels in said mammal, and/or to increase plasma paraoxonase activity in said mammal when fed to said mammal alone or as a component of a food or diet.

11. The method of claim 10, wherein the amount of dry powder extract that produces the decrease in plasma levels of lyophosphatidic acid (LPA), and/or that decreases SAA level in said mouse model, and/or that increases plasma paroxonase activity, is less than 1/10, by weight, the amount of freeze dried tissue of said transgenic plant necessary to produce the same effect.

## Patentansprüche

1. Verfahren zum Konzentrieren in einer transgenen Tomatenpflanze exprimierter transgener apoA-I-mimetischer Peptide, das Folgendes umfasst:
Bereitstellen eines Gewebes der transgenen Tomatenpflanze, wobei das Gewebe ein durch die Pflanze exprimiertes heterologes ApoA-I-mimetisches Peptid enthält und wobei das Gewebe in Form eines weitgehend trockenen Pulvers bereitgestellt wird;
Mischen des Pulvers mit einer Lösung, die Essigsäureethylester und Essigsäure umfasst, unter Bildung eines Extraktionsgemischs;
Sammeln der Flüssigphase des Gemischs und Trocknen der Flüssigphase, so dass ein konzentrierter Trockenpulverextrakt bereitgestellt wird, der die biologische Aktivität des apoA-I-Mimetikums zeigt.

2. Verfahren nach Anspruch 1, wobei die Lösung etwa 1% bis etwa 25% Essigsäure oder etwa 2% bis etwa 20% Essigsäure oder etwa 3% bis etwa 15% Essigsäure oder etwa 4% bis etwa 10% Essigsäure oder etwa 4% bis etwa 8% Essigsäure oder etwa 4% bis etwa 6% Essigsäure umfasst.

3. Verfahren gemäß Anspruch 1 oder Anspruch 2, wobei das Mischen Mischen des Pulvers mit einer Essigsäureethylester und Essigsäure umfassenden Lösung umfasst, gegebenenfalls wobei die Lösung etwa 5% Essigsäure umfasst.

4. Verfahren gemäß einem der Ansprüche 1-3, wobei das Bereitstellen von Gewebe Bereitstellen des Gewebes in Form eines gefriergetrocketen Pulvers umfasst.

5. Verfahren gemäß einem der Ansprüche 1-4, wobei das Mischen des Pulvers mit einer Lösung von Essigsäureethylester und Essigsäure wenigstens 15 Minuten oder wenigstens 1/2 Stunde oder wenigstens 1 Stunde oder wenigstens 2 Stunden oder wenigstens 3 Stunden oder wenigstens 4 Stunden oder wenigstens 5 Stunden oder wenigstens 6 Stunden oder wenigstens 7 Stunden oder wenigstens 12 Stunden oder wenigstens 18 Stunden oder wenigstens 24 Stunden oder wenigstens 48 Stunden oder wenigstens 72 Stunden oder wenigstens 96 Stunden Inkubieren des Extraktionsgemischs umfasst.

6. Verfahren gemäß einem der Ansprüche 1-5, wobei das Sammeln der Flüssigphase und Trocknen der Flüssigphase Trocknen der Flüssigphase unter Erhalt des Trockenpulverextrakts umfasst.

7. Verfahren gemäß einem der Ansprüche 1-5, wobei das Sammeln der Flüssigphase und Trocknen der Flüssigphase Folgendes umfasst:
Trocknen der Flüssigphase unter Erhalt eines trockenen Rückstands;
Resuspendieren des Rückstands in Wasser, so dass ein resuspendiertes Gemisch bereitgestellt wird; und
Trocknen des resuspendierten Gemischs, so dass der Trockenpulverextrakt bereitgestellt wird.

8. Verfahren nach Anspruch 7, wobei das Trocknen des resuspendierten Gemischs Lyophilisieren des Gemisches umfasst, so dass der Trockenpulverextrakt bereitgestellt wird.

9. Verfahren gemäß einem der Ansprüche 1-8, wobei das durch die Pflanze exprimierte heterologe ApoA-I-mimetische Peptid die Aminosäuresequenz DWLKAFYDKFFEKFKEFF (6F) (SEQ ID NO:1) oder FFEKFKEFFKDYFAKLWD(rev6F) (SEQ ID NO:2) umfasst.

10. Verfahren gemäß einem der Ansprüche 1-9, wobei der Trockenpulverextrakt eine Abnahme der Plasmaspiegel von Lysophosphatidsäure (LPA) bei einem Säuger und/oder eine Abnahme der SAA-Spiegel bei dem Säuger und/oder eine Zunahme von Plasma-Paraoxonase-Aktivität bei dem Säuger bewirkt, wenn er dem Säuger allein oder als Bestandteil eines Lebensmittels bzw. einer Nahrung zugeführt wird.

11. Verfahren nach Anspruch 10, wobei die Menge an Trockenpulverextrakt, die zur Abnahme der Plasmaspiegel von Lysophosphatidsäure (LPA) führt und/oder den SAA-Spiegel im Mausmodell senkt und/oder Plasma-Paraoxonase-Aktivität erhöht, nach Gewicht weniger als 1/10 der Menge an gefriergetrocknetem Gewebe der transgenen Pflanze, die zur Erzeugung der gleichen Wirkung benötigt wird, beträgt.

## Revendications

1. Méthode de concentration de peptides mimétiques d'apoA-I transgéniques exprimés dans une plante de tomate transgénique, ladite méthode comprenant :
la fourniture d'un tissu de ladite plante transgénique, où ledit tissu contient un peptide mimétique d'apoA-I hétérologue exprimé par ladite plante, et où ledit tissu est fourni sous forme d'une poudre sensiblement sèche ;
le mélange de ladite poudre avec une solution comprenant de l'acétate d'éthyle et de l'acide acétique afin de former un mélange d'extraction ;
la collecte de la phase liquide dudit mélange et le séchage de ladite phase liquide afin de fournir un extrait concentré de poudre sèche qui présente l'activité biologique dudit mimétique d'apoA-I.

2. Méthode selon la revendication 1, dans laquelle ladite solution comprend d'environ 1% à environ 25% d'acide acétique, ou d'environ 2% à environ 20% d'acide acétique, ou d'environ 3% à environ 15% d'acide acétique, ou d'environ 4% à environ 10% d'acide acétique, ou d'environ 4% à environ 8% d'acide acétique, ou d'environ 4% à environ 6% d'acide acétique.

3. Méthode selon la revendication 1 ou la revendication 2, dans laquelle ledit mélange comprend le mélange de ladite poudre avec une solution comprenant de l'acétate d'éthyle et de l'acide acétique, éventuellement où ladite solution comprend environ 5% d'acide acétique.

4. Méthode selon l'une quelconque des revendications 1-3, dans laquelle ladite fourniture de tissu comprend la fourniture dudit tissu sous forme d'une poudre lyophilisée.

5. Méthode selon l'une quelconque des revendications 1-4, dans laquelle ledit mélange de ladite poudre avec une solution d'acétate d'éthyle et d'acide acétique comprend l'incubation dudit mélange d'extraction pendant au moins 15 minutes, ou au moins ½ heure, ou au moins 1 heure, ou au moins 2 heures, ou au moins 3 heures, ou au moins 4 heures, ou au moins 5 heures, ou au moins 6 heures, ou au moins 7 heures, ou au moins 12 heures, ou au moins 18 heures, ou au moins 24 heures, ou au moins 48 heures, ou au moins 72 heures, ou au moins 96 heures.

6. Méthode selon l'une quelconque des revendications 1-5, dans laquelle ladite collecte de la phase liquide et ledit séchage de ladite phase liquide comprend le séchage de la phase liquide afin de produire ledit extrait de poudre sèche.

7. Méthode selon l'une quelconque des revendications 1-5, dans laquelle ladite collecte de la phase liquide et ledit séchage de ladite phase liquide comprend :
le séchage de la phase liquide afin de produire un résidu sec ;
la remise en suspension dudit résidu dans de l'eau afin de fournir un mélange remis en suspension ; et
le séchage du mélange remis en suspension afin de fournir ledit extrait de poudre sèche.

8. Méthode selon la revendication 7, dans laquelle ledit séchage du mélange remis en suspension comprend la lyophilisation dudit mélange afin de fournir ledit extrait de poudre sèche.

9. Méthode selon l'une quelconque des revendications 1-8, dans laquelle ledit peptide mimétique d'apoA-I hétérologue exprimé par ladite plante comprend la séquence d'acides aminés DWLKAFYDKFFEKFKEFF (6F) (SEQ ID n° 1) ou FFEKFKEFFKDYFAKLWD (rev6F) (SEQ ID n° 2).

10. Méthode selon l'une quelconque des revendications 1-9, dans laquelle ledit extrait de poudre sèche est efficace pour diminuer les taux plasmatiques d'acide lyophosphatidique (LPA) chez un mammifère, et/ou pour diminuer les taux de SAA chez ledit mammifère, et/ou pour augmenter l'activité de paraoxonase plasmatique chez ledit mammifère, lors de sa distribution alimentaire audit mammifère seul ou comme composant d'un aliment ou d'un régime alimentaire.

11. Méthode selon la revendication 10, dans laquelle la quantité de l'extrait de poudre sèche qui produit la diminution des taux plasmatiques d'acide lyophosphatidique (LPA), et/ou qui diminue les taux de SAA chez ledit modèle murin, et/ou qui augmente l'activité de paraoxonase plasmatique, est inférieure à 1/10, en poids, de la quantité de tissu lyophilisé de ladite plante transgénique nécessaire afin de produire le même effet.
